# EUROPEAN PATENT APPLICATION

(11) **EP 4 640 693 A1**
(43) Date of publication of application: **29.10.2025**
(21) Application number: 23907081.6
(22) Date of filing: 20.12.2023
(51) Int. Cl.: C07H 15/18, C07H 1/00, C07H 19/067, C07H 21/04

(54) **METHOD FOR PRODUCING PRODUCTION INTERMEDIATE, PRODUCTION INTERMEDIATE THEREOF, AND METHOD FOR PRODUCING CROSSLINKED ARTIFICIAL NUCLEIC ACID INTERMEDIATE BY USING SAME**

(30) Priority: 21.12.2022 JP 2022204199
(71) Applicant: Mitsubishi Tanabe Pharma Corporation, Osaka-shi Osaka 541-8505 (JP)
(72) Inventor: MATSUMOTO, Koji, Osaka-shi, Osaka 541-8505 (JP); KANAZAWA, Kohei, Osaka-shi, Osaka 541-8505 (JP); MATSUDA, Hiromasa, Osaka-shi, Osaka 541-8505 (JP)
(74) Representative: Hoffmann Eitle
(86) International application number: PCT/JP2023/045658
(87) International publication number: WO 2024/135721

(57) **Abstract**

The present invention is intended to provide a method for producing a production intermediate that is advantageous and safe for industrial production, a production intermediate thereof, and a method for producing a crosslinked artificial nucleic acid intermediate using them.

The present invention includes a method for producing a compound represented by Formula (IV): 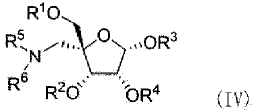 [wherein the symbols are as defined in the specification]
from a compound represented by Formula (I): 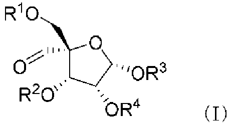 [wherein the symbols are as defined in the specification].

## Description

### [Technical Field]

The present invention relates to a method for producing a production intermediate, the production intermediate, and a method for producing a crosslinked artificial nucleic acid intermediate using them.

### [Technical Background]

As treatment methods for diseases using nucleic acid medicines, there are an antisense method, an antigene method, a method using an aptamer, a method using siRNA, and the like. Among these, the antisense method is a technique in which an oligonucleotide (antisense strand) complementary to disease-related mRNA or disease-related non-translated RNA is introduced from outside, forming a double strand, thereby regulating a function of disease-related RNA to treat or prevent a disease.

As materials for such nucleic acid medicines, various artificial nucleic acids have been developed, and those exhibiting excellent in vitro and/or in vivo pharmacological activity, such as ALNA[Ms] (where ALNA refers to 2'-amino LNA (Locked Nucleic Acid)), ALNA[mU], ALNA[ipU], ALNA[oxz], ALNA[Trz], and guanidine-bridged artificial nucleic acid (GuNA), have been identified (Patent Document 1). These ALNAs and GuNA have respectively been reported to be produced from key intermediates of crosslinked artificial nucleic acids shown below: 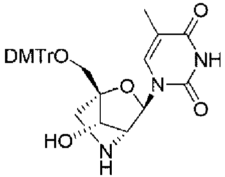 (see, for example, Patent Document 1 and Patent Document 2).

Methods for producing the crosslinked artificial nucleic acid intermediates are described in Patent Document 1 and Non-Patent Documents 1 and 2.

### [Related Art]

### [Patent Documents]

[Patent Document 1] International Publication No. 2020/100826
[Patent Document 2] International Publication No. 2017/047816

### [Non-Patent Documents]

[Non-Patent Document 1] J. Org. Chem., 2012, 77, 23, 10718-10728
[Non-Patent Document 2] Org. Biomol. Chem., 2003 Feb 21; 1 (4): 655-63

### [Summary of the Invention]

Regarding the methods for producing the crosslinked artificial nucleic acid intermediates described in Patent Document 1, Patent Document 2, and Non-Patent Document 1, there was room for improvement because, in a step of introducing a nitrogen atom into a crosslinked structure between the 4' and 2' ribosyl ring atoms, a metal azide, which poses risks such as explosiveness and/or toxic gass generation, is used as a reagent, and furthermore, an azide compound with explosive potential is involved as a production intermediate. Further, regarding the method for producing the crosslinked artificial nucleic acid intermediate described in Non-Patent Document 2, although it is a production method that does not involve an azide compound, it has been reported that yield of the step of introducing a nitrogen atom contained in a crosslinked structure between the 4' and 2' ribosyl ring atoms is significantly low. Under such circumstances, an industrially superior method for producing crosslinked artificial nucleic acid intermediates such as ALNA[Ms], ALNA[mU], ALNA[ipU], ALNA[oxz], ALNA[Trz], and GuNA is desired.

The conventional methods for producing crosslinked artificial nucleic acid intermediates use a metal azide, which poses risks as described above, and furthermore, involves an azide compound, which poses risks as described above, as a production intermediate, thus leaving room for improvement in terms of safety for workers during production. Further, a production method that does not involve an azide compound suffers from low yield and is disadvantageous for large-scale production, and thus has problems as an industrial production method.

Therefore, the present invention is intended to provide, among other things, a method for producing a production intermediate that is advantageous and safe for industrial production, a production intermediate thereof, and a method for producing a crosslinked artificial nucleic acid intermediate using them, which solve the above-described problems.

The present inventors and others conducted various studies to carry out each production step safely, with good reproducibility, and in high yield, and as a result, discovered an industrially advantageous production method capable of producing a production intermediate useful for safely manufacturing a crosslinked artificial nucleic acid intermediate in high yield, without using the above-described reagents or production intermediates that pose risks, by performing a reductive amination reaction. Further, they have discovered an industrially advantageous production method capable of producing a desired crosslinked artificial nucleic acid intermediate in high yield and high quality by using a step of crystallizing a type of production intermediate. Further, they have identified several novel production intermediates useful in these production methods.

That is, the present invention includes the following several embodiments.

A first embodiment of the present invention is a method for producing a compound represented by the following Formula (IV).
[1] A method for producing a compound represented by Formula (IV): 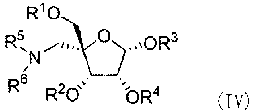 [wherein
   R¹ represents a hydroxyl protecting group;
   R² represents a hydroxyl protecting group;
   R³ and R⁴, which may be the same or different, each independently represent a hydroxyl protecting group, or together form a cyclic protecting group for a diol; and
   one of R⁵ and R⁶ represents a hydrogen atom or an amino protecting group, and the other represents an amino protecting group]
   from a compound represented by Formula (I): 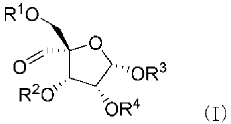 [wherein
   R¹ represents a hydroxyl protecting group;
   R² represents a hydroxyl protecting group; and
   R³ and R⁴, which may be the same or different, each independently represent a hydroxyl
   protecting group, or together form a cyclic protecting group for a diol],
   the production method comprising the following Steps 1 to 3:
      Step 1: a step of obtaining a compound represented by Formula (II): 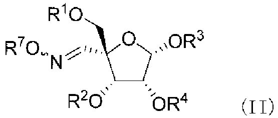 [wherein
         R¹ represents a hydroxyl protecting group;
         R² represents a hydroxyl protecting group;
         R³ and R⁴, which may be the same or different, each independently represent a hydroxyl protecting group, or together form a cyclic protecting group for a diol; and
         R⁷ represents a hydrogen atom or an alkyl group],
         by reacting the compound represented by Formula (I): 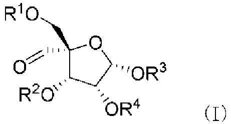 [wherein the symbols have the same meaning as described above] with a compound represented by Formula (V):

            R⁷O-NH₂ (V)

            [wherein R⁷ represents a hydrogen atom or an alkyl group] or a salt thereof;
      Step 2: a step of obtaining a compound represented by Formula (III): 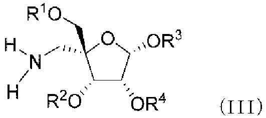 [wherein
         R¹ represents a hydroxyl protecting group;
         R² represents a hydroxyl protecting group; and
         R³ and R⁴, which may be the same or different, each independently represent a hydroxyl protecting group, or together form a cyclic protecting group for a diol],
         by subjecting a compound represented by Formula (II) to a reduction reaction with a reducing agent; and
      Step 3: a step of subjecting the compound represented by Formula (III) to a reaction for protecting an amino group.
[2] The production method according to the above [1], wherein the reducing agent used in Step 2 is sodium bis(2-methoxyethoxy)aluminum hydride (Red-Al).
[3] The production method according to the above [1] or [2], wherein the compound represented by Formula (I): 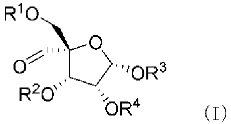 [wherein
   R¹ represents a hydroxyl protecting group;
   R² represents a hydroxyl protecting group; and
   R³ and R⁴, which may be the same or different, each independently represent a hydroxyl protecting group, or together form a cyclic protecting group for a diol]
   is produced by a process including the following Steps 4 to 8:
      Step 4: a step of obtaining a compound represented by Formula (VII): 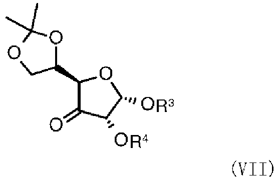 [wherein R³ and R⁴, which may be the same or different, each independently represent a hydroxyl protecting group, or together form a cyclic protecting group for a diol],
         by subjecting a compound represented by Formula (VI): 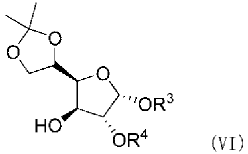 [wherein R³ and R⁴, which may be the same or different, each independently represent a hydroxyl protecting group, or together form a cyclic protecting group for a diol] to an oxidation reaction;
      Steps 5 to 7: steps of obtaining a compound represented by Formula (X): 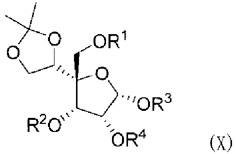 [wherein R³ and R⁴, which may be the same or different, each independently represent a hydroxyl protecting group, or together form a cyclic protecting group for a diol],
         by sequentially subjecting the compound represented by Formula (VII) to an aldol reaction (Step 5), a reduction reaction (Step 6), and a hydroxyl protecting reaction (Step 7); and
      Step 8: a step of subjecting the compound represented by Formula (X) to an oxidative cleavage reaction.

A second embodiment of the present invention is a method for producing a compound represented by the following Formula (XIII).
[4] A method for producing a compound represented by Formula (XIII): 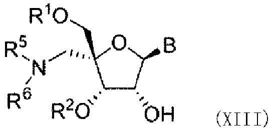 [wherein
   R¹ represents a hydroxyl protecting group;
   R² represents a hydroxyl protecting group;
   one of R⁵ and R⁶ represents a hydrogen atom or an amino protecting group, and the other represents an amino protecting group; and
   B represents a nucleic acid base moiety which may be substituted with one or more substituents] from a compound represented by Formula (IV): 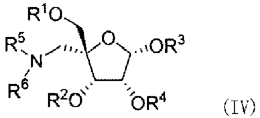 [wherein
   R¹ represents a hydroxyl protecting group;
   R² represents a hydroxyl protecting group;
   R³ and R⁴, which may be the same or different, each independently represent a hydroxyl protecting group, or together form a cyclic protecting group for a diol; and
   one of R⁵ and R⁶ represents a hydrogen atom or an amino protecting group, and the other represents an amino protecting group],
   the production method comprising the following Steps 9 to 11:
      Step 9: a step of obtaining a compound represented by Formula (XI): 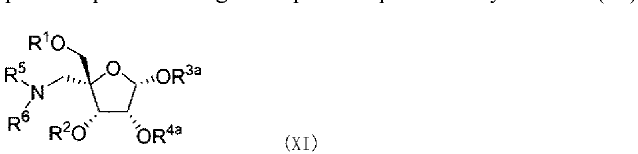 [wherein
         R¹ represents a hydroxyl protecting group;
         R² represents a hydroxyl protecting group;
         R^{3a} represents an acyl group;
         R^{4a} represents an acyl group; and
         one of R⁵ and R⁶ represents a hydrogen atom or an amino protecting group, and the other represents an amino protecting group],
         by subjecting a compound represented by Formula (IV), produced using the production method according to any one of the above [1] to [3], to a conversion reaction of R³ and R⁴ into acyl groups, as necessary;
      Step 10: a step of obtaining a compound represented by Formula (XII): 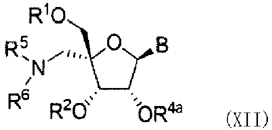 [wherein
         R¹ represents a hydroxyl protecting group;
         R² represents a hydroxyl protecting group;
         R^{4a} represents an acyl group;
         one of R⁵ and R⁶ represents a hydrogen atom or an amino protecting group, and the other represents an amino protecting group; and
         B represents a nucleic acid base moiety which may be substituted with one or more substituents], by subjecting the compound represented by Formula (XI) to a glycosylation reaction; and
      Step 11: a step of subjecting the compound represented by Formula (XII) to a deprotection reaction of an acyl group.
[5] The production method according to the above [4], wherein B is represented by Formula (XXIV): 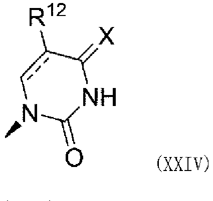 [wherein
   R¹² represents a hydrogen atom, an alkyl group, an alkoxy group, or an alkoxyalkyl group;
   X represents an oxygen atom or NR¹³ (wherein R¹³ represents a hydrogen atom or an amino protecting group); and
   a double line consisting of a solid line and a dotted line represents a single bond or a double bond].

A third embodiment of the present invention is a method for producing a compound represented by the following Formula (XVII).
A method for producing a compound represented by Formula (XVII): 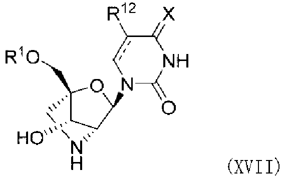 [wherein
R¹ represents a hydrogen atom or a hydroxyl protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond] from a compound represented by Formula (XIII'): 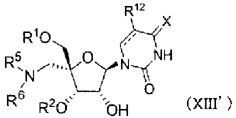 [wherein
R¹ represents a hydroxyl protecting group;
R² represents a hydroxyl protecting group;
one of R⁵ and R⁶ represents a hydrogen atom or an amino protecting group, and the other represents an amino protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond],
the production method comprising the following Steps 12 to 15:
   Step 12: a step of obtaining a compound represented by Formula (XIV): 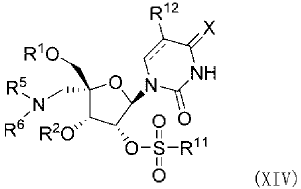 [wherein
      R¹ represents a hydroxyl protecting group;
      R² represents a hydroxyl protecting group;
      one of R⁵ and R⁶ represents a hydrogen atom or an amino protecting group, and the other represents an amino protecting group;
      R¹¹ represents an alkyl group or an aryl group;
      R¹² represents a hydrogen atom, an alkyl group, an alkoxy group, or an alkoxyalkyl group;
      X represents an oxygen atom or NR¹³ (wherein R¹³ represents a hydrogen atom or an amino protecting group); and
      a double line consisting of a solid line and a dotted line represents a single bond or a double bond],
      by subjecting a compound represented by Formula (XIII'), produced using the production method according to the above [4] or [5], to a sulfonylation reaction;
   Step 13: a step of obtaining a compound represented by Formula (XV): 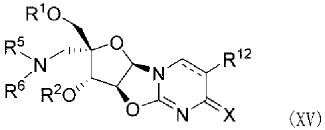 [wherein
      R¹ represents a hydroxyl protecting group;
      R² represents a hydroxyl protecting group;
      one of R⁵ and R⁶ represents a hydrogen atom or an amino protecting group, and the other represents an amino protecting group;
      R¹² represents a hydrogen atom, an alkyl group, an alkoxy group, or an alkoxyalkyl group;
      X represents an oxygen atom or NR¹³ (wherein R¹³ represents a hydrogen atom or an amino protecting group); and
      a double line consisting of a solid line and a dotted line represents a single bond or a double bond],
      by subjecting the compound represented by Formula (XIV) to a cyclization reaction;
   Step 14: a step of obtaining a compound represented by Formula (XVI): 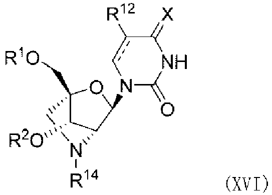 [wherein
      R¹ represents a hydroxyl protecting group;
      R² represents a hydroxyl protecting group;
      R¹² represents a hydrogen atom, an alkyl group, an alkoxy group, or an alkoxyalkyl group; R¹⁴ represents a hydrogen atom or an amino protecting group;
      X represents an oxygen atom or NR¹³ (wherein R¹³ represents a hydrogen atom or an amino protecting group); and
      a double line consisting of a solid line and a dotted line represents a single bond or a double bond],
      by subjecting the compound represented by Formula (XV) to a ring-closing reaction; and
   Step 15: a step of subjecting the compound represented by Formula (XVI) to a deprotection reaction, or to a deprotection reaction followed by a further hydroxyl protecting reaction.

Further several embodiments of the present invention are the following several compounds.
[7] A compound represented by Formula (II): 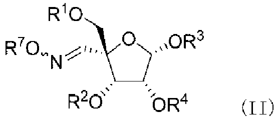
   [wherein R¹ represents a hydroxyl protecting group;
   R² represents a hydroxyl protecting group;
   R³ and R⁴, which may be the same or different, each independently represent a hydroxyl protecting group, or together form a cyclic protecting group for a diol; and
   R⁷ represents a hydrogen atom or an alkyl group].
[8] A compound represented by Formula (III): 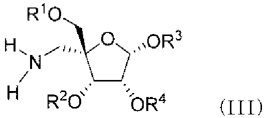 [wherein
   R¹ represents a hydroxyl protecting group;
   R² represents a hydroxyl protecting group; and
   R³ and R⁴, which may be the same or different, each independently represent a hydroxyl protecting group, or together form a cyclic protecting group for a diol].
[9] A compound represented by Formula (IV): 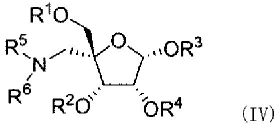 [wherein
   R¹ represents a hydroxyl protecting group;
   R² represents a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   R³ and R⁴, which may be the same or different, each independently represent a hydroxyl protecting group, or together form a cyclic protecting group for a diol; and
   one of R⁵ and R⁶ represents a hydrogen atom or an amino protecting group, and the other represents an amino protecting group].
[10] A compound represented by Formula (XI): 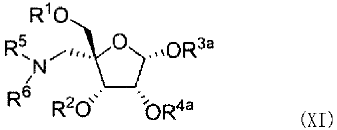 [wherein
   R¹ represents a hydroxyl protecting group;
   R² represents a hydroxyl protecting group;
   R^{3a} and R^{4a} each represent an acyl group; and
   one of R⁵ and R⁶ represents a hydrogen atom or an amino protecting group, and the other represents an amino protecting group].
[11] The compound according to the above [10], represented by Formula (XI): 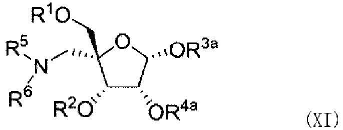 [wherein
   R¹ represents a hydroxyl protecting group;
   R² represents a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   R^{3a} and R^{4a} each represent an acyl group; and
   one of R⁵ and R⁶ represents a hydrogen atom or an amino protecting group, and the other represents an amino protecting group].
[12] A compound represented by Formula (XII): 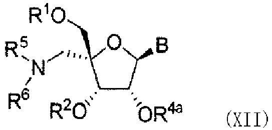 [wherein
   R¹ represents a hydroxyl protecting group;
   R² represents a hydroxyl protecting group;
   R^{4a} represents an acyl group;
   one of R⁵ and R⁶ represents a hydrogen atom or an amino protecting group, and the other represents an amino protecting group; and
   B represents a nucleic acid base moiety which may be substituted with one or more substituents].
[13] The compound according to the above [12], represented by Formula (XII): 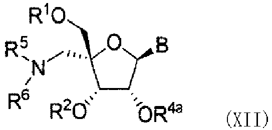 [wherein
   R¹ represents a hydroxyl protecting group;
   R² represents a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   R^{4a} represents an acyl group;
   one of R⁵ and R⁶ represents a hydrogen atom or an amino protecting group, and the other represents an amino protecting group; and
   B represents a nucleic acid base moiety which may be substituted with one or more substituents].
[14] The compound according to the above [12] or [13], wherein B is thyminyl.
[15] A compound represented by Formula (XIII): 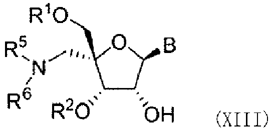 [wherein
   R¹ represents a hydroxyl protecting group;
   R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   one of R⁵ and R⁶ represents a hydrogen atom or an amino protecting group, and the other represents an amino protecting group; and
   B represents a nucleic acid base moiety which may be substituted with one or more substituents].
[16] The compound according to the above [15], wherein B is thyminyl.
[17] A compound represented by Formula (XIV): 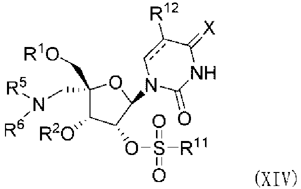 [wherein
   R¹ represents a hydroxyl protecting group;
   R² represents a hydroxyl protecting group;
   one of R⁵ and R⁶ represents a hydrogen atom or an amino protecting group, and the other represents an amino protecting group;
   R¹¹ represents an alkyl group or an aryl group;
   R¹² represents a hydrogen atom, an alkyl group, an alkoxy group, or an alkoxyalkyl group;
   X represents an oxygen atom or NR¹³ (wherein R¹³ represents a hydrogen atom or an amino protecting group); and
   a double line consisting of a solid line and a dotted line represents a single bond or a double bond].
[18] The compound according to the above [17], represented by Formula (XIV): 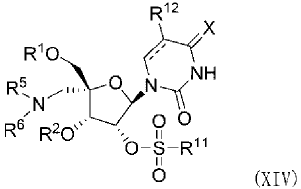 [wherein
   R¹ represents a hydroxyl protecting group;
   R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   one of R⁵ and R⁶ represents a hydrogen atom or an amino protecting group, and the other represents an amino protecting group;
   R¹¹ represents an alkyl group or an aryl group;
   R¹² represents a hydrogen atom, an alkyl group, an alkoxy group, or an alkoxyalkyl group;
   X represents an oxygen atom or NR¹³ (wherein R¹³ represents a hydrogen atom or an amino protecting group); and
   a double line consisting of a solid line and a dotted line represents a single bond or a double bond].
[19] The compound according to the above [17] or [18], wherein R¹² is a methyl group, and X is an oxygen atom.
[20] A compound represented by formula (XV): 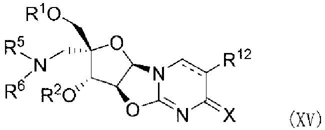 [wherein
   R¹ represents a hydroxyl protecting group;
   R² represents a hydroxyl protecting group;
   one of R⁵ and R⁶ represents a hydrogen atom or an amino protecting group, and the other represents an amino protecting group;
   R¹² represents a hydrogen atom, an alkyl group, an alkoxy group or an alkoxyalkyl group;
   X represents an oxygen atom or NR¹³ (wherein R¹³ represents a hydrogen atom or an amino protecting group); and
   a double line consisting of a solid line and a dotted line represents a single bond or a double bond].
[21] The compound according to [20], represented by Formula (XV): 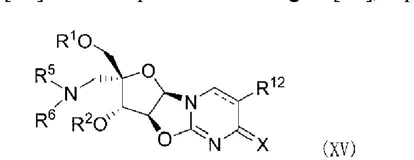 [wherein
   R¹ represents a hydroxyl protecting group;
   R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   one of R⁵ and R⁶ represents a hydrogen atom or an amino protecting group, and the other represents an amino protecting group;
   R¹² represents a hydrogen atom, an alkyl group, an alkoxy group, or an alkoxyalkyl group;
   X represents an oxygen atom or NR¹³ (wherein R¹³ represents a hydrogen atom or an amino protecting group); and
   a double line consisting of a solid line and a dashed line represents a single bond or a double bond].
[22] The compound according to the above [20] or [21], wherein R¹² is a methyl group, and X is an oxygen atom.
[23] The compound according to any one of the above [20] to [22], wherein R¹² is a methyl group; X is an oxygen atom; R¹ is a benzyl group; R² is a benzyl group; R⁵ is a benzyloxycarbonyl group; R⁶ is a hydrogen atom; and a double line consisting of a solid line and a dashed line is a single bond or a double bond.
[24] A crystal of the compound according to the above [23].
[25] The crystal of the compound according to the above [24], having peaks at diffraction angles, expressed in terms of 2θ, of 7.4±0.2°, 19.2±0.2°, 20.1±0.2°, 21.3±0.2°, and 24.7±0.2° in a powder X-ray diffraction spectrum measured using CuKα radiation.
[26] A compound represented by Formula (XVI): 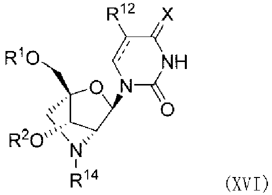 [wherein
   R¹ represents a hydroxyl protecting group;
   R² represents a hydroxyl protecting group;
   R¹² represents a hydrogen atom, an alkyl group, an alkoxy group, or an alkoxyalkyl group;
   R¹⁴ represents a hydrogen atom, a benzyloxycarbonyl group, an isobutyl group, a benzoyl group, a t-butyloxycarbonyl group, a trimethylsilylethoxycarbonyl (Teoc) group, an allyloxycarbonyl group, a 9-fluorenylmethoxycarbonyl group, a 2,2,2-trichloroethoxycarbonyl group, an acetyl group, a formyl group, a 4-methoxybenzyl group, an allyl group, a benzyl group, or a 2-nitrobenzenesulfonyl group;
   X represents an oxygen atom or NR¹³ (wherein R¹³ represents a hydrogen atom or an amino protecting group); and
   a double line consisting of a solid line and a dashed line represents a single bond or a double bond].
[27] The compound according to the above [26], represented by Formula (XVI) 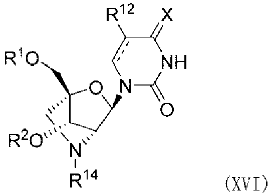 [wherein
   R¹ represents a hydroxyl protecting group;
   R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
   R¹² represents a hydrogen atom, an alkyl group, an alkoxy group, or an alkoxyalkyl group;
   R¹⁴ represents a hydrogen atom, a benzyloxycarbonyl group, an isobutyl group, a benzoyl group, a t-butyloxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a 4-methoxybenzyl group, an allyl group, a benzyl group, or a 2-nitrobenzenesulfonyl group;
   X represents an oxygen atom or NR¹³ (wherein R¹³ represents a hydrogen atom or an amino protecting group); and
   a double line consisting of a solid line and a dashed line represents a single bond or a double bond].

A further embodiment of the present invention is a method for producing a compound represented by the following Formula (XVII).
A method for producing a compound represented by Formula (XVII): 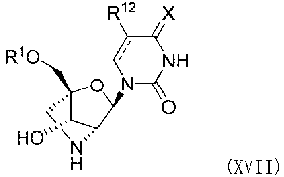 [wherein
R¹ represents a hydrogen atom or a hydroxyl protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond] from a compound represented by Formula (I): 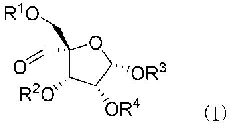 [wherein
R¹ represents a hydroxyl protecting group;
R² represents a hydroxyl protecting group; and
R³ and R⁴, which may be the same or different, each independently represent a hydroxyl protecting group, or together form a cyclic protecting group for a diol],
the production method comprising the following Steps 1 to 3 and 9 to 15:
   Step 1: a step of obtaining a compound represented by Formula (II): 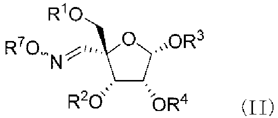 [wherein
      R¹ represents a hydroxyl protecting group;
      R² represents a hydroxyl protecting group;
      R³ and R⁴, which may be the same or different, each independently represent a hydroxyl protecting group, or together form a cyclic protecting group for a diol; and
      R⁷ represents a hydrogen atom or an alkyl group],
      by reacting a compound represented by Formula (I): 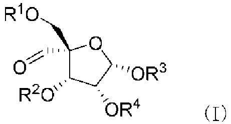 [wherein the symbols have the same meaning as described above] with a compound represented by Formula (V):

         R⁷O-NH₂ (V)

         [wherein R⁷ represents a hydrogen atom or an alkyl group] or a salt thereof;
   Step 2: a step of obtaining a compound represented by Formula (III): 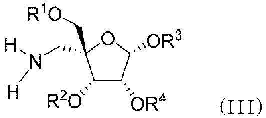 [wherein
      R¹ represents a hydroxyl protecting group;
      R² represents a hydroxyl protecting group; and
      R³ and R⁴, which may be the same or different, each independently represent a hydroxyl protecting group, or together form a cyclic protecting group for a diol],
      by subjecting the compound represented by Formula (II) to a reduction reaction with a reducing agent;
   Step 3: a step of obtaining a compound represented by Formula (IV): 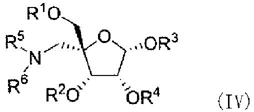 [wherein
      R¹ represents a hydroxyl protecting group;
      R² represents a hydroxyl protecting group;
      R³ and R⁴, which may be the same or different, each independently represent a hydroxyl protecting group, or together form a cyclic protecting group for a diol; and
      one of R⁵ and R⁶ represents a hydrogen atom or an amino protecting group, and the other represents an amino protecting group],
      by subjecting the compound represented by Formula (III) to a reaction for protecting an amino group;
   Step 9: a step of obtaining a compound represented by Formula (XI): 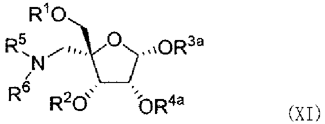 [wherein
      R¹ represents a hydroxyl protecting group;
      R² represents a hydroxyl protecting group;
      R^{3a} represents an acyl group;
      R^{4a} represents an acyl group; and
      one of R⁵ and R⁶ represents a hydrogen atom or an amino protecting group, and the other represents an amino protecting group],
      by subjecting the compound represented by Formula (IV) to a conversion reaction of R³ and R⁴ into acyl groups, as necessary; Step 10: a step of obtaining a compound represented by Formula (XII'): 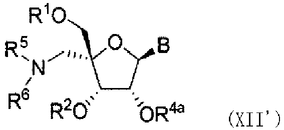 [wherein
      R¹ represents a hydroxyl protecting group;
      R² represents a hydroxyl protecting group;
      R^{4a} represents an acyl group;
      one of R⁵ and R⁶ represents a hydrogen atom or an amino protecting group, and the other represents an amino protecting group; and
      B represents a compound represented by Formula (XXIV): 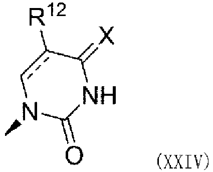 [wherein
      R¹² represents a hydrogen atom, an alkyl group, an alkoxy group, or an alkoxyalkyl group;
      X represents an oxygen atom or NR¹³ (wherein R¹³ represents a hydrogen atom or an amino protecting group); and
      a double line consisting of a solid line and a dotted line represents a single bond or a double bond]],
      by subjecting the compound represented by Formula (XI) to a glycosylation reaction;
   Step 11: a step of obtaining a compound represented by Formula (XIII'): 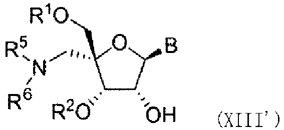 [wherein
      R¹ represents a hydroxyl protecting group;
      R² represents a hydroxyl protecting group;
      one of R⁵ and R⁶ represents a hydrogen atom or an amino protecting group, and the other represents an amino protecting group; and
      B represents a compound represented by Formula (XXIV): 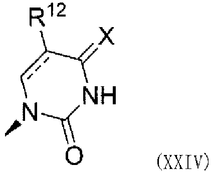
      [wherein the symbols have the same meaning as described above]], by subjecting the compound represented by Formula (XII') to a deprotection reaction of the acyl group;
   Step 12: a step of obtaining a compound represented by Formula (XIV): 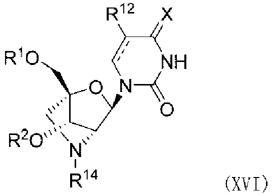 [wherein
      R¹ represents a hydroxyl protecting group;
      R² represents a hydroxyl protecting group;
      one of R⁵ and R⁶ represents a hydrogen atom or an amino protecting group, and the other represents an amino protecting group;
      R¹¹ represents an alkyl group or an aryl group;
      R¹² represents a hydrogen atom, an alkyl group, an alkoxy group, or an alkoxyalkyl group;
      X represents an oxygen atom or NR¹³ (wherein R¹³ represents a hydrogen atom or an amino protecting group); and
      a double line consisting of a solid line and a dotted line represents a single bond or a double bond],
      by subjecting the compound represented by Formula (XIII') to a sulfonylation reaction;
   Step 13: a step of obtaining a compound represented by Formula (XV): 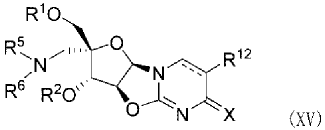 [wherein
      R¹ represents a hydroxyl protecting group;
      R² represents a hydroxyl protecting group;
      one of R⁵ and R⁶ represents a hydrogen atom or an amino protecting group, and the other represents an amino protecting group;
      R¹² represents a hydrogen atom, an alkyl group, an alkoxy group, or an alkoxyalkyl group;
      X represents an oxygen atom or NR¹³ (wherein R¹³ represents a hydrogen atom or an amino protecting group); and
      a double line consisting of a solid line and a dotted line represents a single bond or a double bond],
      by subjecting the compound represented by Formula (XIV) to a cyclization reaction;
   Step 14: a step of obtaining a compound represented by Formula (XVI): 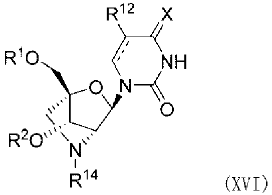 [wherein
      R¹ represents a hydroxyl protecting group;
      R² represents a hydroxyl protecting group;
      R¹² represents a hydrogen atom, an alkyl group, an alkoxy group, or an alkoxyalkyl group;
      R¹⁴ represents a hydrogen atom or an amino protecting group;
      X represents an oxygen atom or NR¹³ (wherein R¹³ represents a hydrogen atom or an amino protecting group); and
      a double line consisting of a solid line and a dotted line represents a single bond or a double bond],
      by subjecting the compound represented by Formula (XV) to a ring-closing reaction; and
   Step 15: a step of subjecting the compound represented by Formula (XVI) to a deprotection reaction, or to a deprotection reaction followed by a further hydroxyl protecting reaction.

A further embodiment of the present invention is a method for producing the following modified oligonucleotide or a salt thereof.
[29] A method for producing a modified oligonucleotide or a salt thereof represented by the following formula: 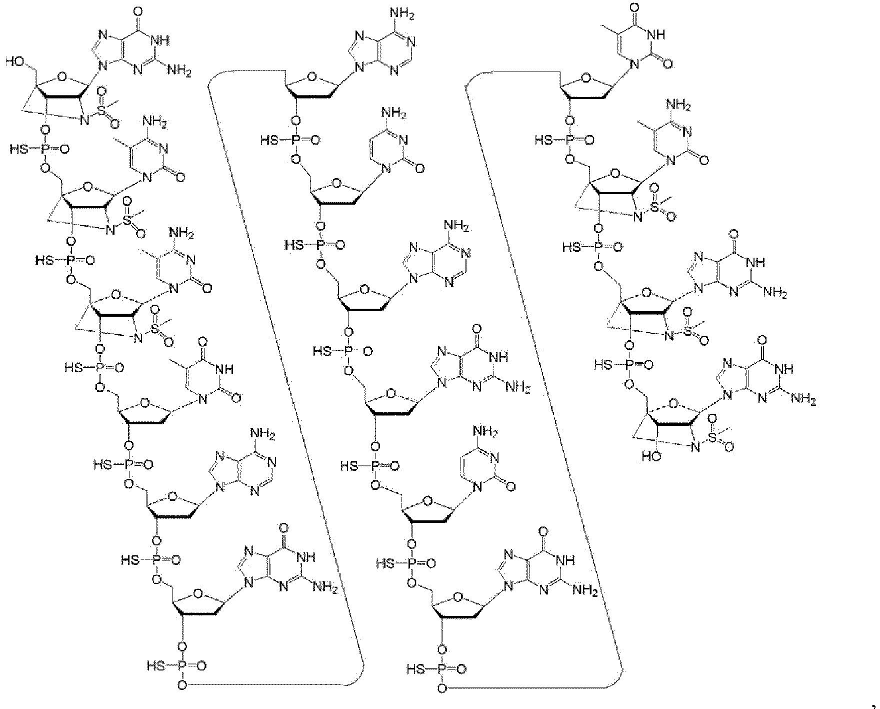 from a compound represented by Formula (XVII"): 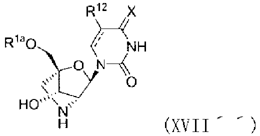 [wherein
   R^{1a} represents a hydroxyl protecting group;
   R¹² represents a hydrogen atom, an alkyl group, an alkoxy group, or an alkoxyalkyl group;
   X represents an oxygen atom or NR¹³ (wherein R¹³ represents a hydrogen atom or an amino protecting group); and
   a double line consisting of a solid line and a dotted line represents a single bond or a double bond],
   the production method comprising the following steps:
      a step of producing a compound represented by Formula (XVII") using the production method according to the above [6];
      a step of producing an ALNA[Ms] amidite from the compound represented by Formula (XVII");
      a step of subjecting the ALNA[Ms] amidite and an amidite for DNA synthesis to a phosphoramidite method; and,
      as necessary, a step of subjecting a phosphodiester bond to a thiation reaction.
[30] A method for producing a modified oligonucleotide or a salt thereof represented by the following formula: 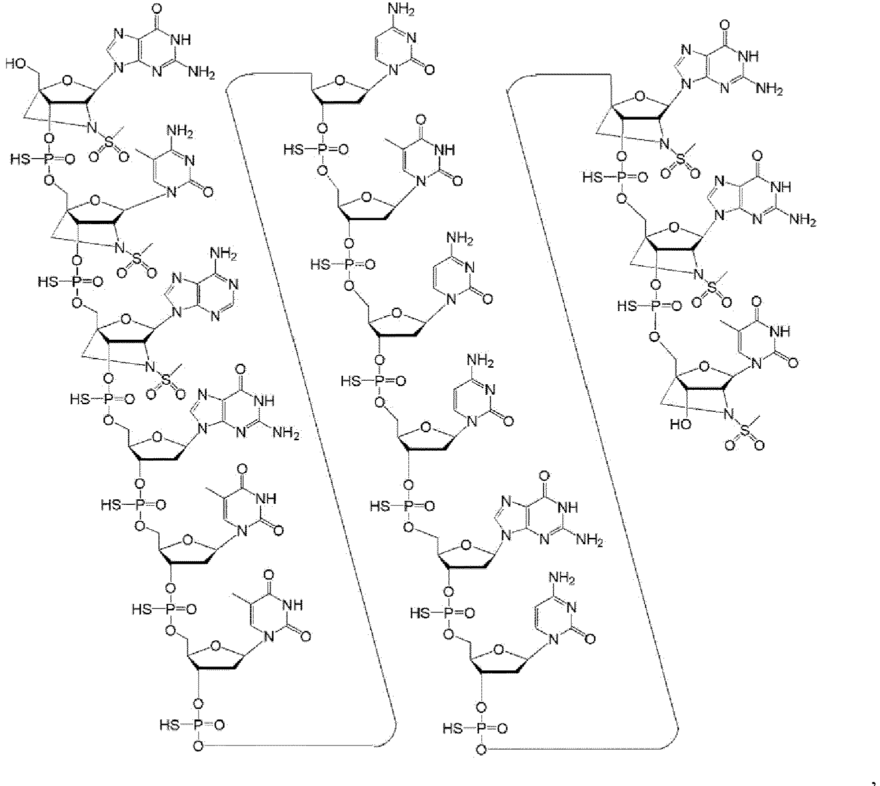 from a compound represented by Formula (XVII"): 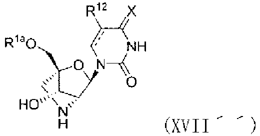 [wherein
   R^{1a} represents a hydroxyl protecting group;
   R¹² represents a hydrogen atom, an alkyl group, an alkoxy group, or an alkoxyalkyl group;
   X represents an oxygen atom or NR¹³ (wherein R¹³ represents a hydrogen atom or an amino protecting group); and
   a double line consisting of a solid line and a dotted line represents a single bond or a double bond],
   the production method comprising the following steps:
      a step of producing a compound represented by Formula (XVII") using the production method according to the above [6];
      a step of producing an ALNA[Ms] amidite from the compound represented by Formula (XVII");
      a step of subjecting the ALNA[Ms] amidite and an amidite for DNA synthesis to a phosphoramidite method; and,
      as necessary, a step of subjecting a phosphodiester bond to a thiation reaction.
[31] A modified oligonucleotide or a salt thereof represented by the following formula: 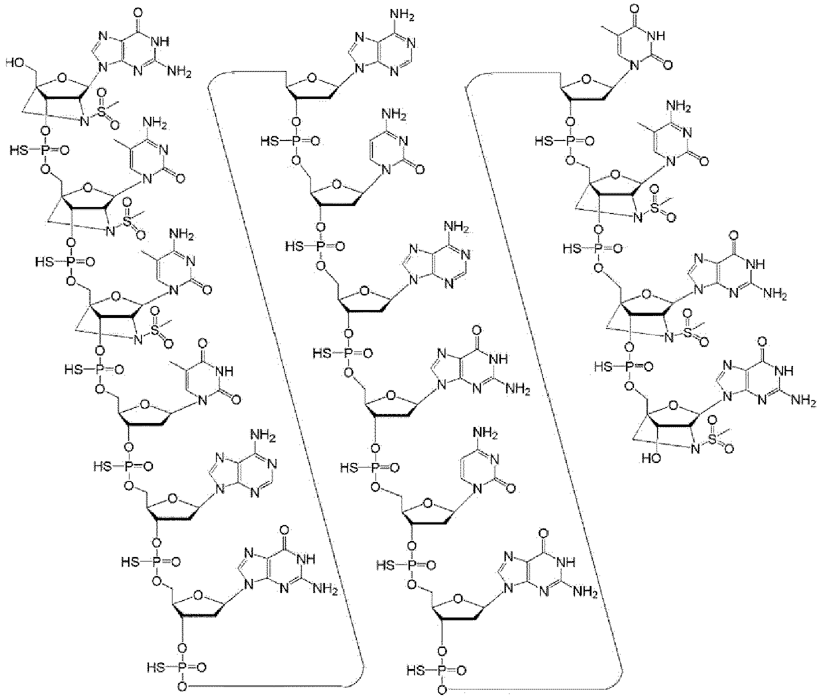 produced using the production method according to the above [29].
[32] A modified oligonucleotide or a salt thereof represented by the following formula: 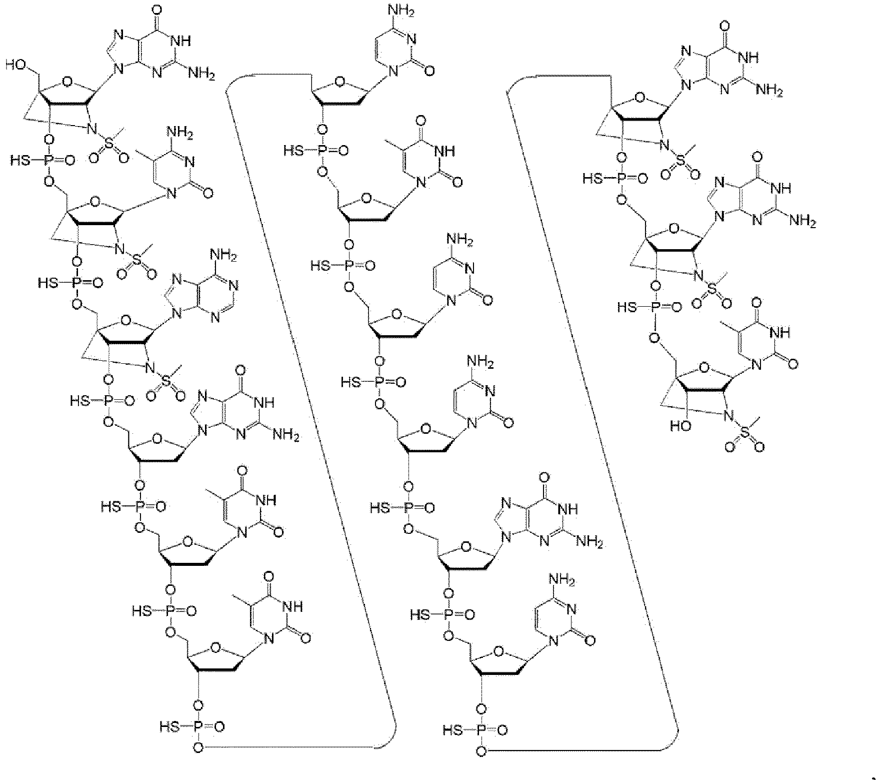 produced using the production method according to the above [30].

According to the method for producing a compound of Formula (IV), which is the first embodiment of the present invention, a production intermediate useful for producing a crosslinked artificial nucleic acid intermediate that is useful as an intermediate for a nucleic acid medicine or the like can be produced using a method suitable for industrial production. More specifically, by using a reductive amination reaction in producing a compound of Formula (III) from a compound of Formula (I), the compound of Formula (III) and the subsequent compound of Formula (IV) can be safely produced in good yield.
According to the method for producing a compound of Formula (XIII), which is the second embodiment of the present invention, the compound of Formula (XIII), which is another novel production intermediate, can be produced in good yield from the compound of Formula (IV) produced using the production method of the first embodiment described above.
Further, according to the method for producing a compound of Formula (XVII), which is the third embodiment of the present invention, a compound of Formula (XVII), which is one of desired crosslinked artificial nucleic acid intermediates, can be produced using a method suitable for industrial production from a compound of Formula (XIII') produced using the production method of the second embodiment described above. More specifically, the compound of Formula (XV), which is one of production intermediates, can be obtained as crystals, and by passing through this crystallization step, not only can the compound of Formula (XV) be produced in high purity and good yield by simple purification, but also, by using this compound in subsequent steps, can the compound of Formula (XVII), which is one of desired crosslinked artificial nucleic acid intermediates, be obtained in high quality.
While the production methods of the first to third embodiments described above are each independently industrially advantageous, combining them allows a desired crosslinked artificial nucleic acid intermediate to be produced safely, with good reproducibility, in good yield, and with high quality, and thus is extremely advantageous industrially. A combination of these production methods is also an embodiment of the present invention.
Several production intermediate compounds in the production methods of the first to third embodiments described above are also industrially advantageous intermediate compounds, and are each an embodiment of the present invention.
Furthermore, a method for producing a modified oligonucleotide or a salt thereof that includes any production method described above, and a modified oligonucleotide or a salt thereof produced using the production method are each an embodiment of the present invention.

### [Brief Description of the Drawings]

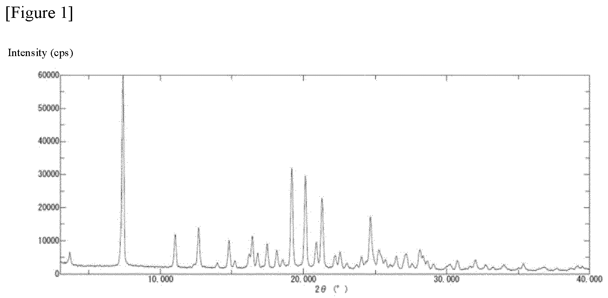

[Fig. 1] Powder X-ray diffraction pattern of crystals of a compound of Formula (XV') (Compound 14).

### [Mode for Carrying Out the Invention]

Definitions of the groups in the present specification are as follows.

In the present specification, the term "alkyl group" refers to a linear or branched saturated hydrocarbon group having 1 to 6 carbon atoms (C₁₋₆). A group having 1 to 4 carbon atoms (C₁₋₄) is particularly preferred. Specific examples thereof include a methyl group, an ethyl group, an n-propyl group, an i-propyl group, an n-butyl group, an i-butyl group, a 2-methyl-n-butyl group, an i-amyl (3-methyl-n-butyl) group, a 2-methyl-n-pentyl group, and the like, with the methyl group being preferred.

In the present specification, the term "alkoxy group" refers to a monovalent group in which the above alkyl group is bonded to an oxygen atom, and examples thereof include linear or branched alkyl-O- groups having 1 to 6 carbon atoms (C₁₋₆), with alkyl-O- groups having 1 to 4 carbon atoms (C₁₋₄) being preferred. Specific examples thereof include a methoxy group, an ethoxy group, an n-propoxy group, an i-propoxy group, an n-butoxy group, a t-butoxy group, a 2-methyl-n-propoxy group, a 3-methyl-n-butoxy group, and the like, with the methoxy group being preferred.

In the present specification, the term "alkoxyalkyl group" refers to the above alkyl group substituted with 1 or 2 alkoxy groups. Specific examples thereof include a methoxymethyl group, a methoxyethyl group, a methoxypropyl group, and the like, with the methoxymethyl group being preferred.

In the present specification, the term "alkylene group" refers to a divalent group derived from a linear or branched saturated hydrocarbon having 1 to 6 carbon atoms (C₁₋₆) by removing two hydrogen atoms, and specific examples thereof include a methylene group, an ethylene group, an n-propylene group, an isopropylidene group, a butylene group, a hexylene group, a cyclopentylene group, a cyclohexylene group, and the like.

In the present specification, the term "alkynyl group" refers to a linear or branched alkynyl group having 2 to 6 carbon atoms (C₂₋₆), preferably 2 to 4 carbon atoms (C₂₋₄), and more preferably 2 to 3 carbon atoms (C₂₋₃). Specific examples thereof include an ethynyl group, a 1-propynyl group, a 2-propynyl group, a 1-butynyl group, a 2-butynyl group, a 1-pentynyl group, a 2-pentynyl group, a 3-pentynyl group, a 4-pentynyl group, a 1-hexynyl group, a 1-methyl-2-propynyl group, a 1-methyl-2-butynyl group, and the like.

In the present specification, the term "aryl group" means a functional group or substituent derived from an aromatic hydrocarbon (monocyclic, bicyclic, or tricyclic). It preferably represents a monocyclic or bicyclic aromatic hydrocarbon group having 6 to 11 ring-forming carbon atoms, and more preferably represents a 5-membered or 6-membered aromatic hydrocarbon group. Among these, a phenyl group is preferred. Further, the aryl group may be substituted, and examples of substituents for such an aryl group include a hydroxyl group, a halogen atom, a nitro group, a cyano group, a C₁₋₆ alkyl group which may be substituted with one or more substituents, a C₂₋₆ alkenyl group which may be substituted with one or more substituents, a C₂₋₆ alkynyl group which may be substituted with one or more substituents, a C₁₋₆ alkoxy group, an aryloxy group, an amino group, an amino group which may be substituted with one or more C₁₋₃ alkyl groups, and an aryl group, from which one or more (preferably 1 to 3) identical or different groups are selected. Preferred examples include a C₁₋₆ alkyl group which may be substituted with one or more substituents, an amino group which may be substituted with one or more C₁₋₃ alkyl groups, and a halogen atom. More preferred examples include a trifluoromethyl group, a dimethylamino group, and a chloro atom.
Specific examples of an aryl group which may be substituted with one or more substituents include a 2-methylphenyl group, a 3-methylphenyl group, a 4-methylphenyl group, a 2,6-dimethylphenyl group, a 2,4-dimethylphenyl group, a 2-chlorophenyl group, a 3-chlorophenyl group, a 4-chlorophenyl group, a 2,4-dichlorophenyl group, a 2,5-dichlorophenyl group, a 2,6-dichlorophenyl group, a 2-bromophenyl group, a 4-methoxyphenyl group, a 4-chloro-2-nitrophenyl group, a 4-nitrophenyl group, a 2-nitrophenyl group, a 2,4-dinitrophenyl group, a 2-trifluoromethylphenyl group, a 3-trifluoromethylphenyl group, a 4-trifluoromethylphenyl group, a 2-dimethylaminophenyl group, a 3-dimethylaminophenyl group, a 4-dimethylaminophenyl group, a 2-biphenyl group, a 3-biphenyl group, a 4-biphenyl group, and the like.

In the present specification, the term "halogen atom" refers to, for example, a fluorine atom, a chlorine atom, a bromine atom, or an iodine atom.

In the present specification, the term "protecting group" as used in "hydroxyl protecting group" and "amino protecting group" refers to a group that is not particularly limited as long as it is capable of stably protecting an amino group or a hydroxyl group during nucleic acid synthesis. Specifically, it refers to a protecting group that is stable under acidic or neutral conditions and can be cleaved by chemical methods such as hydrogenolysis, hydrolysis, electrolysis, or photolysis.

In the present specification, the term "hydroxyl protecting group" refers to a protecting group commonly used in organic synthetic chemistry (particularly, in nucleic acid synthesis), and examples thereof include, for example, an acyl group; an alkyl group; a methyl group substituted with 1 to 3 aryl groups; a methyl group substituted with 1 to 3 aryl groups which are substituted with an alkyl group, an alkoxy group, a halogen atom, and/or a cyano group; a methyl group substituted with an alkoxy group; a silyl group; a silyloxymethyl group; and a carbonate group. Specific examples thereof include a benzyl (may be referred to as Bn) group, a 4,4'-dimethoxytrityl (may be referred to as DMTr) group, a 4-methoxytrityl group, a triphenylmethyl group, a 2-naphthylmethyl group, a cyanoethoxycarbonyl group, a cyanoethyl group, a methoxymethyl group, a methylthiomethyl group, a benzyloxymethyl group, a 4-methoxybenzyloxymethyl group, a t-butyloxymethyl group, a trimethylsilyloxymethyl group, a t-butyldimethylsilyloxymethyl group, a hexyldimethylsilyloxymethyl group, a t-butyldiphenylsilyloxymethyl group, a triethylsilyloxymethyl group, a triisopropylsilyloxymethyl group, a 2-methoxyethoxymethyl group, a 2,2,2-trichloroethoxymethyl group, a 2-(trimethylsilyl)ethoxymethyl group, a 1-ethoxyethyl group, a tetrahydropyranyl group, a trimethylsilyl (may be referred to as TMS) group, a triethylsilyl group, a triisopropylsilyl group, a t-butyldimethylsilyl group, a t-butyldiphenylsilyl group, a triphenylsilyl group, a 4-methoxybenzyl group, a 3,4-dimethoxybenzyl group, a 2,6-dimethoxybenzyl group, a p-phenylbenzyl group, a benzoyl (may be referred to as Bz) group, a phenoxyacetyl group, a benzyloxycarbonyl (may be referred to as Cbz) group, a t-butyloxycarbonyl (may be referred to as Boc) group, a 9-fluorenylmethyloxycarbonyl (may be referred to as Fmoc) group, and an acetyl (may be referred to as Ac) group. For example, a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group is preferred, and a benzyl group, a 4,4'-dimethoxytrityl group, or an acetyl group is more preferred.

In the present specification, the term "cyclic protecting group for a diol" means a cyclic protecting group for a hydroxyl group (diol) formed by oxygen atoms of two hydroxyl groups of a diol together with an alkylene group. Specific examples thereof include cyclic acetal-type protecting groups such as a methoxymethylene acetal group, an isopropylidene ketal group, a methylene acetal group, an ethylidene acetal group, a t-butylmethylidene acetal group, a 1-t-butylethylidene ketal group, a 1-phenylethylidene ketal group, a 1-(4-methoxyphenyl)ethylidene ketal group, a 2,2,2-trichloroethylidene acetal group, a benzylidene acetal group, a 4-methoxybenzylidene acetal group, a 2,4-methoxybenzylidene acetal group, a 3,4-methoxybenzylidene acetal group, a cyclopentylidene ketal group, a cyclohexylidene ketal group, a cycloheptylidene ketal group, a benzophenone ketal group, an ethoxymethylene acetal group, a 1-methoxyethylidene orthoester group, a 1-ethoxyethylidene orthoester group, a 1-benzyloxybenzylidene orthoester group, a di-t-butylsilylene group, a diisopropylsilylene group, a methylcyclohexylsilylene group, a dicyclohexylsilylene group, a 1,3-(1,1,3,3-tetraisopropyl)disiloxanylidene group, a 1,1,3,3-tetra-t-butoxydisiloxanylidene group, and a cyclic carbonate group; among these, an isopropylidene ketal group, a methylene acetal group, an ethylidene acetal group, a benzylidene acetal group, a 4-methoxybenzylidene acetal group, or a 1-benzyloxybenzylidene orthoester group is preferred, and an isopropylidene ketal group is more preferred. The above specific examples of cyclic protecting groups are, in accordance with conventions of organic synthetic chemistry, names of groups that include oxygen atoms forming a cyclic structure. For example, names of specific examples of a cyclic protecting group for a diol formed by R³ and R⁴ together are names of groups that include oxygen atoms to which R³ and R⁴ are respectively bonded.

In the present specification, the term "amino protecting group" means a protecting group commonly used in organic synthetic chemistry (particularly, in nucleic acid synthesis), and examples thereof include, for example, an aliphatic acyl group; an aromatic acyl group; a sulfonyl group; an alkyl group; a methyl group substituted with 1 to 3 aryl groups; and a methyl group substituted with 1 to 3 aryl groups which are substituted with a halogen atom and/or a cyano group. Specific examples thereof include an acetyl (Ac) group, a formyl group, a phenoxyacetyl group, a t-butylphenoxyacetyl group, a p-isopropylphenoxyacetyl group, a trifluoroacetyl group, a propionyl group, an isobutyryl group, a benzoyl (Bz) group, a methoxycarbonyl group, an ethoxycarbonyl group, a t-butoxycarbonyl group, a trimethylsilylethoxycarbonyl group, a cyanoethoxycarbonyl group, a benzyloxycarbonyl (Cbz) group, an allyloxycarbonyl group, a 9-fluorenylmethoxycarbonyl group, a dimethylaminomethylenyl group, a 2,2,2-trichloroethoxycarbonyl group, a t-amyloxycarbonyl group, a 4-methoxybenzyl group, a triphenylmethyl group, a 2-nitrobenzenesulfonyl group, a 4-nitrobenzenesulfonyl group, a 2,4-dinitrobenzenesulfonyl group, a 2-(trimethylsilyl)ethoxymethyl group, a benzyl (Bn) group, and an allyl group. For example, a benzyloxycarbonyl (Cbz) group, an isobutyryl group, a benzoyl (Bz) group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl (Ac) group, a formyl group, a trifluoroacetyl group, a 4-methoxybenzyl group, an allyl group, a benzyl (Bn) group, or a 2-nitrobenzenesulfonyl group is preferred, and a benzyloxycarbonyl (Cbz) group, an acetyl (Ac) group, a 4-methoxybenzyl group, or an allyl group is more preferred.

In the present specification, examples of the term "acyl group" include an aliphatic acyl group and an aromatic acyl group. Specific examples of an aliphatic acyl group include alkylcarbonyl groups such as a formyl group, an acetyl group, a propionyl group, a butyryl group, an isobutyryl group, a pentanoyl group, a pivaloyl group, a valeryl group, an isovaleryl group, an octanoyl group, a nonanoyl group, a decanoyl group, a 3-methylnonanoyl group, an 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an eicosanoyl group, and a heneicosanoyl group; aryloxyalkylcarbonyl groups such as a phenoxyacetyl group; carboxylated alkylcarbonyl groups such as a succinoyl group, a glutaroyl group, and an adipoyl group; carbonyl groups having 1 to 6 carbon atoms and substituted with an alkyl group substituted with a halogen atom, such as a chloroacetyl group, a dichloroacetyl group, a trichloroacetyl group, and a trifluoroacetyl group; alkoxyalkylcarbonyl groups having 1 to 6 carbon atoms, such as a methoxyacetyl group; and unsaturated alkylcarbonyl groups such as an (E)-2-methyl-2-butenoyl group. Further, examples of an aromatic acyl group include arylcarbonyl groups such as a benzoyl group, an α-naphthoyl group, and a β-naphthoyl group; halogenoarylcarbonyl groups such as a 2-bromobenzoyl group and a 4-chlorobenzoyl group; arylcarbonyl groups substituted with an alkyl group having 1 to 6 carbon atoms, such as a 2,4,6-trimethylbenzoyl group and a 4-toluoyl group; arylcarbonyl groups substituted with an alkoxy group having 1 to 6 carbon atoms, such as a 4-anisoyl group; carboxylated arylcarbonyl groups such as a 2-carboxybenzoyl group, a 3-carboxybenzoyl group, and a 4-carboxybenzoyl group; nitrated arylcarbonyl groups such as a 4-nitrobenzoyl group and a 2-nitrobenzoyl group; carbonylated arylcarbonyl groups substituted with an alkoxy group having 1 to 6 carbon atoms, such as a 2-(methoxycarbonyl)benzoyl group; arylated arylcarbonyl groups such as a 4-phenylbenzoyl group; and the like.

In the present specification, examples of the term "silyl group" include silyl groups substituted with an alkyl group having 1 to 6 carbon atoms, such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group; silyl groups substituted with three alkyl groups having 1 to 6 carbon atoms and substituted with1 to 2 aryl groups, such as a t-butyldiphenylsilyl group, a diphenylmethylsilyl group, a butyldiphenylbutylsilyl group, a diphenylisopropylsilyl group, and a phenyldiisopropylsilyl group; and a triphenylsilyl group.

In the present specification, examples of the term "silyloxy group" include silyloxy groups substituted with an alkyl group, such as a trimethylsilyloxymethyl group, a t-butyldimethylsilyloxymethyl group, a hexyldimethylsilyloxymethyl group, a triethylsilyloxymethyl group, and a triisopropylsilyloxymethyl group; and silyloxy groups substituted with an aryl group, such as a t-butyldiphenylsilyloxymethyl group.

In the present specification, the term "nucleic acid base moiety" in "nucleic acid base moiety which may be substituted with one or more substituents" includes natural nucleic acid base moieties and non-natural nucleic acid base moieties, encompassing aromatic heterocyclic groups (for example, monocyclic groups, bicyclic groups, tricyclic groups, and the like). Nucleic acid base moieties include not only commonly known purine and pyrimidine heterocycles, but also their heterocyclic analogs and tautomers. Specific examples of nucleic acid base moieties include adeninyl, guanyl, thyminyl, dihydrothyminyl, cytosinyl, uracinyl, dihydrouracyl, purinyl, xanthinyl, diaminopurinyl, 8-oxo-N⁶-methyladeninyl, 7-deazaxanthinyl, 7-deazaguanyl, 2-thiothyminyl, N⁴,N⁴-ethanocytosinyl, N⁶,N⁶-ethano-2,6-diaminopurinyl, 5-methylcytosinyl, 5-(C³ - C⁶)-alkynylcytosinyl, 5-fluorocytosinyl, 5-bromouracinyl, pseudoisocytosinyl, 2-hydroxy-5-methyl-4-triazolopyridinyl, isocytosinyl, isoguanyl, inosinyl, 6-aminopurinyl, 2-aminopurinyl, 6-chloro-2-aminopurinyl, 6-chloropurinyl, N⁶-allylpurinyl, N⁶-acylpurinyl, N⁶-benzylpurinyl, N⁶-halopurinyl, N⁶-vinylpurinyl, N⁶-acetylenic purinyl, N⁶-hydroxyalkylpurinyl, N⁶-thioalkylpurinyl, N²-alkylpurinyl, N⁴-alkylpyrimidinyl, N⁴-acylpyrimidinyl, N⁴-benzylpurinyl, N⁴-halopyrimidinyl, N⁴-vinylpyrimidinyl, N⁴-acetylpyrimidinyl, N⁴-hydroxyalkylpyrimidinyl, N⁶-thioalkylpyrimidinyl, 6-azapyrimidinyl, 6-azacytosinyl, 2- and/or 4-mercaptopyrimidinyl, uracinyl, C⁵-alkylpyrimidinyl, C⁵-benzylpyrimidinyl, C⁵-halopyrimidinyl, C⁵-vinylpyrimidinyl, C⁵-acetylenic pyrimidinyl, C⁵-acylpyrimidinyl, C⁵-hydroxyalkylpurinyl, C⁵-amidopyrimidinyl, C⁵-cyanopyrimidinyl, C⁵-nitropyrimidinyl, C⁵-aminopyrimidinyl, N²-alkylpurinyl, N²-alkyl-6-thiopurinyl, 5-cytidinyl, 5-azauracinyl, triazolopyridinyl, imidazopyridinyl, pyrrolopyrimidinyl, pyrazolopyrimidinyl, and the like. Preferred examples of nucleic acid base moieties include adeninyl, guanyl, 2,6-diaminopurinyl, thyminyl, dihydrothyminyl, 2-thiothyminyl, cytosinyl, 5-methylcytosinyl, uracinyl, dihydrouracyl, 5-fluorocytosinyl, xanthinyl, 6-aminopurinyl, 2-aminopurinyl, 6-chloro-2-aminopurinyl, and 6-chloropurinyl. These nucleic acid base moieties may be substituted with one or more substituents, and preferably may be substituted with one or two substituents. Examples of the substituents include a hydroxyl group, an alkoxy group, a mercapto group, an alkylthio group, an amino group, an amino group substituted with an alkyl group, an alkyl group, an alkynyl group, an oxo group, a thioxo group, and a halogen atom. Functional oxygen, sulfur, and nitrogen groups on the base moieties can be protected and/or deprotected as necessary. Suitable protecting groups are well known to those skilled in the art, and examples thereof include, for example, the above-described hydroxyl protecting group and amino protecting group, and the like; and preferred examples include a diphenylaminocarbonyl group, silyl groups (for example, a trimethylsilyl group, a dimethylhexylsilyl group, a t-butyldimethylsilyl group, and a t-butyldiphenylsilyl group), a trityl group, an alkyl group, acyl groups (for example, an acetyl group, a propionyl group, an isobutyryl group, a benzoyl group, and a phenoxyacetyl group), alkoxycarbonyl groups (for example, a t-butoxycarbonyl group, a benzyloxycarbonyl group, a diphenylaminocarbonyl group (may be referred to as DPC), and a cyanoethoxycarbonyl group), sulfonyl groups (for example, a methanesulfonyl group and a p-toluenesulfonyl group), a dimethylaminomethylenyl group, and the like.

### <Production method and compound aspects>

Several production methods and several compounds, which are embodiments of the present invention, include one or more production method aspects and one or more compound aspects as follows.

As one aspect of the above embodiments, the present invention includes a production method and compounds used in the production method, the production method comprising one or more steps among Steps 1 to 3 in the present specification using the compounds, wherein, in Formulas (I) to (V) in Steps 1 to 3, R¹ is a hydroxyl protecting group, R² is a hydroxyl protecting group, R³ and R⁴ together form a cyclic protecting group for a diol, R⁵ and R⁶ are amino protecting groups, and R⁷ is an alkyl group or a hydrogen atom.

As one aspect of the above embodiments, the present invention includes a production method and compounds used in the production method, the production method comprising one or more steps among Steps 1 to 3 in the present specification using the compounds, wherein, in Formulas (I) to (V) in Steps 1 to 3, R¹ is a hydroxyl protecting group, R² is a hydroxyl protecting group, R³ and R⁴ together form a cyclic protecting group for a diol, R⁵ is an amino protecting group, R⁶ is a hydrogen atom, and R⁷ is a hydrogen atom.

As one aspect of the above embodiments, the present invention includes a production method and compounds used in the production method, the production method comprising one or more steps among Steps 1 to 3 in the present specification using the compounds, wherein, in Formulas (I) to (V) in Steps 1 to 3, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, and R⁷ is a hydrogen atom.

As one aspect of the above embodiments, the present invention includes a production method and compounds used in the production method, the production method comprising one or more steps among Steps 1 to 3 in the present specification using the compounds, wherein, in Formulas (I) to (V) in Steps 1 to 3, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R⁵ is a benzyloxycarbonyl group, an acetyl group, a 4-methoxybenzyl group, or an allyl group, R⁶ is a hydrogen atom, and R⁷ is a hydrogen atom.

As one aspect of the above embodiments, the present invention includes a production method and compounds used in the production method, the production method comprising one or more steps among Steps 1 to 8 in the present specification using the compounds, wherein, in Formulas (I) to (X) in Steps 1 to 8, R¹ is a hydroxyl protecting group, R² is a hydroxyl protecting group, R³ and R⁴ together form a cyclic protecting group for a diol, R⁵ and R⁶ are amino protecting groups, and R⁷ is an alkyl group or a hydrogen atom.

As one aspect of the above embodiments, the present invention includes a production method and compounds used in the production method, the production method comprising one or more steps among Steps 1 to 8 in the present specification using the compounds, wherein, in Formulas (I) to (X) in Steps 1 to 8, R¹ is a hydroxyl protecting group, R² is a hydroxyl protecting group, R³ and R⁴ together form a cyclic protecting group for a diol, R⁵ is an amino protecting group, R⁶ is a hydrogen atom, and R⁷ is a hydrogen atom.

As one aspect of the above embodiments, the present invention includes a production method and compounds used in the production method, the production method comprising one or more steps among Steps 1 to 8 in the present specification using the compounds, wherein, in Formulas (I) to (X) in Steps 1 to 8, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, and R⁷ is a hydrogen atom.

As one aspect of the above embodiments, the present invention includes a production method and compounds used in the production method, the production method comprising one or more steps among Steps 1 to 8 in the present specification using the compounds, wherein, in Formulas (I) to (X) in Steps 1 to 8, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R⁵ is a benzyloxycarbonyl group, an acetyl group, a 4-methoxybenzyl group, or an allyl group, R⁶ is a hydrogen atom, and R⁷ is a hydrogen atom.

As one aspect of the above embodiments, the present invention includes a production method and compounds used in the production method, the production method comprising one or more steps among Steps 1 to 11 in the present specification using the compounds, wherein, in Formulas (I) to (XIII) in Steps 1 to 11, R¹ is a hydroxyl protecting group, R² is a hydroxyl protecting group, R³ and R⁴ together form a cyclic protecting group for a diol, R^{3a} and R^{4a} are acyl groups, R⁵ and R⁶ are amino protecting groups, R⁷ is an alkyl group or a hydrogen atom, and B is a nucleic acid base moiety.

As one aspect of the above embodiments, the present invention includes a production method and compounds used in the production method, the production method comprising one or more steps among Steps 1 to 11 in the present specification using the compounds, wherein, in Formulas (I) to (XIII) in Steps 1 to 11, R¹ is a hydroxyl protecting group, R² is a hydroxyl protecting group, R³ and R⁴ together form a cyclic protecting group for a diol, R^{3a} and R^{4a} are acetyl groups, R⁵ is an amino protecting group, R⁶ is a hydrogen atom, R⁷ is a hydrogen atom, and B is a nucleic acid base moiety.

As one aspect of the above embodiments, the present invention includes a production method and compounds used in the production method, the production method comprising one or more steps among Steps 1 to 11 in the present specification using the compounds, wherein, in Formulas (I) to (XIII) in Steps 1 to 11, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R^{3a} and R^{4a} are acetyl groups, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, R⁷ is a hydrogen atom, and B is a nucleic acid base moiety.

As one aspect of the above embodiments, the present invention includes a production method and compounds used in the production method, the production method comprising one or more steps among Steps 1 to 11 in the present specification using the compounds, wherein, in Formulas (I) to (XIII) in Steps 1 to 11, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, and B is a nucleic acid base moiety.

As one aspect of the above embodiments, the present invention includes a production method and compounds used in the production method, the production method comprising one or more steps among Steps 1 to 11 in the present specification using the compounds, wherein, in Formulas (I) to (XIII) in Steps 1 to 11, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, and B is adeninyl, guanyl, 2,6-diaminopurinyl, thyminyl, dihydrothyminyl, 2-thiothyminyl, cytosinyl, 5-methylcytosinyl, uracinyl, dihydrouracyl, 5-fluorocytosinyl, xanthinyl, 6-aminopurinyl, 2-aminopurinyl, 6-chloro-2-aminopurinyl, or 6-chloropurinyl.

As one aspect of the above embodiments, the present invention includes a production method and compounds used in the production method, the production method comprising one or more steps among Steps 1 to 11 in the present specification using the compounds, wherein, in Formulas (I) to (XIII) in Steps 1 to 11, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, and B is thyminyl.

As one aspect of the above embodiments, the present invention includes a production method and compounds used in the production method, the production method comprising one or more steps among Steps 1 to 3 and Steps 9 to 11 in the present specification using the compounds, wherein, in Formulas (I) to (XIII) in Steps 1 to 3 and Steps 9 to 11, R¹ is a hydroxyl protecting group, R² is a hydroxyl protecting group, R³ and R⁴ together form a cyclic protecting group for a diol, R^{3a} and R^{4a} are acyl groups, R⁵ and R⁶ are amino protecting groups, R⁷ is an alkyl group or a hydrogen atom, and B is a nucleic acid base moiety.

As one aspect of the above embodiments, the present invention includes a production method and compounds used in the production method, the production method comprising one or more steps among Steps 1 to 3 and Steps 9 to 11 in the present specification using the compounds, wherein, in Formulas (I) to (XIII) in Steps 1 to 3 and Steps 9 to 11, R¹ is a hydroxyl protecting group, R² is a hydroxyl protecting group, R³ and R⁴ together form a cyclic protecting group for a diol, R^{3a} and R^{4a} are acetyl groups, R⁵ is an amino protecting group, R⁶ is a hydrogen atom, R⁷ is a hydrogen atom, and B is a nucleic acid base moiety.

As one aspect of the above embodiments, the present invention includes a production method and compounds used in the production method, the production method comprising one or more steps among Steps 1 to 3 and Steps 9 to 11 in the present specification using the compounds, wherein, in Formulas (I) to (XIII) in Steps 1 to 3 and Steps 9 to 11, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R^{3a} and R^{4a} are acetyl groups, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, R⁷ is a hydrogen atom, and B is a nucleic acid base moiety.

As one aspect of the above embodiments, the present invention includes a production method and compounds used in the production method, the production method comprising one or more steps among Steps 1 to 3 and Steps 9 to 11 in the present specification using the compounds, wherein, in Formulas (I) to (XIII) in Steps 1 to 3 and Steps 9 to 11, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, and B is a nucleic acid base moiety.

As one aspect of the above embodiments, the present invention includes a production method and compounds used in the production method, the production method comprising one or more steps among Steps 1 to 3 and Steps 9 to 11 in the present specification using the compounds, wherein, in Formulas (I) to (XIII) in Steps 1 to 3 and Steps 9 to 11, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, and B is adeninyl, guanyl, 2,6-diaminopurinyl, thyminyl, dihydrothyminyl, 2-thiothyminyl, cytosinyl, 5-methylcytosinyl, uracinyl, dihydrouracyl, 5-fluorocytosinyl, xanthinyl, 6-aminopurinyl, 2-aminopurinyl, 6-chloro-2-aminopurinyl, or 6-chloropurinyl.

As one aspect of the above embodiments, the present invention includes a production method and compounds used in the production method, the production method comprising one or more steps among Steps 1 to 3 and Steps 9 to 11 in the present specification using the compounds, wherein, in Formulas (I) to (XIII) in Steps 1 to 3 and Steps 9 to 11, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, and B is thyminyl.

As one aspect of the above embodiments, the present invention includes a production method and compounds used in the production method, the production method comprising one or more steps among Steps 1 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 14, Step 15-1, and Step 15-2, R¹ is a hydroxyl protecting group, R^{1a} is a hydroxyl protecting group, R² is a hydroxyl protecting group, R³ and R⁴ together form a cyclic protecting group for a diol, R^{3a} and R^{4a} are acyl groups, R⁵ and R⁶ are amino protecting groups, R⁷ is an alkyl group or a hydrogen atom, R¹¹ is an alkyl group, R¹² is a hydrogen atom, an alkyl group, an alkoxy group, or an alkoxyalkyl group, R¹⁴ is a hydrogen atom or an amino protecting group, X is an oxygen atom or NR¹³ (wherein R¹³ represents a hydrogen atom or an amino protecting group), and a double line consisting of a solid line and a dotted line represents a single bond or a double bond.

As one aspect of the above embodiments, the present invention includes a production method and compounds used in the production method, the production method comprising one or more steps among Steps 1 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 14, Step 15-1, and Step 15-2, R¹ is a hydroxyl protecting group, R^{1a} is a hydroxyl protecting group, R² is a hydroxyl protecting group, R³ and R⁴ together form a cyclic protecting group for a diol, R^{3a} and R^{4a} are acetyl groups, R⁵ is an amino protecting group, R⁶ is a hydrogen atom, R⁷ is a hydrogen atom, R¹¹ is an alkyl group, R¹² is a hydrogen atom or a methyl group, R¹⁴ is a hydrogen atom or an amino protecting group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 14, Step 15-1, and Step 15-2, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R^{1a} is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R^{3a} and R^{4a} are acetyl groups, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, R⁷ is a hydrogen atom, R¹¹ is a methyl group, R¹² is a hydrogen atom or a methyl group, R¹⁴ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 14, Step 15-1, and Step 15-2, R¹ is a benzyl group, a 4,4'-dimethoxytrityl group, or an acetyl group, R^{1a} is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, R¹¹ is a methyl group, R¹² is a hydrogen atom or a methyl group, R¹⁴ is a benzyloxycarbonyl group, an acetyl group, a 4-methoxybenzyl group, or an allyl group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 14, Step 15-1, and Step 15-2, R¹ is a benzyl group, a 4,4'-dimethoxytrityl group, or an acetyl group, R^{1a} is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, R¹¹ is a methyl group, R¹² is a methyl group, R¹⁴ is a benzyloxycarbonyl group, an acetyl group, a 4-methoxybenzyl group, or an allyl group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 3, Steps 9 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 3, Steps 9 to 14, Step 15-1, and Step 15-2, R¹ is a hydroxyl protecting group, R^{1a} is a hydroxyl protecting group, R² is a hydroxyl protecting group, R³ and R⁴ together form a cyclic protecting group for a diol, R^{3a} and R^{4a} are acyl groups, R⁵ and R⁶ are amino protecting groups, R⁷ is an alkyl group or a hydrogen atom, R¹¹ is an alkyl group, R¹² is a hydrogen atom, an alkyl group, an alkoxy group, or an alkoxyalkyl group, R¹⁴ is a hydrogen atom or an amino protecting group, X is an oxygen atom or NR¹³ (wherein R¹³ is a hydrogen atom or an amino protecting group), and a double line consisting of a solid line and a dotted line represents a single bond or a double bond.

As one aspect of the above embodiments, the present invention includes a production method and compounds used in the production method, the production method comprising one or more steps among Steps 1 to 3, Steps 9 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 3, Steps 9 to 14, Step 15-1, and Step 15-2, R¹ is a hydroxyl protecting group, R^{1a} is a hydroxyl protecting group, R² is a hydroxyl protecting group, R³ and R⁴ together form a cyclic protecting group for a diol, R^{3a} and R^{4a} are acetyl groups, R⁵ is an amino protecting group, R⁶ is a hydrogen atom, R⁷ is a hydrogen atom, R¹¹ is an alkyl group, R¹² is a hydrogen atom or a methyl group, R¹⁴ is a hydrogen atom or an amino protecting group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 3, Steps 9 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 3, Steps 9 to 14, Step 15-1, and Step 15-2, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R^{1a} is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R^{3a} and R^{4a} are acetyl groups, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, R⁷ is a hydrogen atom, R¹¹ is a methyl group, R¹² is a hydrogen atom or a methyl group, R¹⁴ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 3, Steps 9 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 3, Steps 9 to 14, Step 15-1, and Step 15-2, R¹ is a benzyl group, a 4,4'-dimethoxytrityl group, or an acetyl group, R^{1a} is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, R¹¹ is a methyl group, R¹² is a hydrogen atom or a methyl group, R¹⁴ is a benzyloxycarbonyl group, an acetyl group, a 4-methoxybenzyl group, or an allyl group, X is an oxygen atom, and a double line consisting of a solid line and a dotted line represents a double bond.

As one aspect of the above embodiments, the present invention includes a production method and compounds used in the production method, the production method comprising one or more steps among Steps 1 to 3, Steps 9 to 14, Step 15-1, and Step 15-2 in the present specification using the compounds, wherein, in Formulas (I) to (XI), Formula (XII'), Formula (XIII'), Formulas (XIV) to (XVII), Formula (XVII'), and Formula (XVII") in Steps 1 to 3, Steps 9 to 14, Step 15-1, and Step 15-2, R¹ is a benzyl group, a 4,4'-dimethoxytrityl group, or an acetyl group, R^{1a} is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R^{3a} and R^{4a} are acetyl groups, R⁵ is a benzyloxycarbonyl group, an acetyl group, a 4-methoxybenzyl group, or an allyl group, R⁶ is a hydrogen atom, R⁷ is a hydrogen atom, R¹¹ is a methyl group, R¹² is a methyl group, R¹⁴ is a benzyloxycarbonyl group, an acetyl group, a 4-methoxybenzyl group, or an allyl group, X is an oxygen atom, and a double line consisting of a solid line and a dotted line represents a double bond.

As one aspect of the above embodiments, the present invention includes a production method and compounds used in the production method, the production method comprising one or more steps among Steps 16 to 18 in the present specification using the compounds, wherein, in Formula (I), Formula (IV'), and Formulas (XVIII) to (XX) in Steps 16 to 18, R¹ is a hydroxyl protecting group, R² is a hydroxyl protecting group, R³ and R⁴ together form a cyclic protecting group for a diol, R⁵ is an amino protecting group, and R⁸ is an alkoxy group.

As one aspect of the above embodiments, the present invention includes a production method and compounds used in the production method, the production method comprising one or more steps among Steps 16 to 18 in the present specification using the compounds, wherein, in Formula (I), Formula (IV'), and Formulas (XVIII) to (XX) in Steps 16 to 18, R¹ is a hydroxyl protecting group, R² is a hydroxyl protecting group, R³ and R⁴ together form a cyclic protecting group for a diol, R⁵ is an amino protecting group, and R⁸ is a para-methoxy group.

As one aspect of the above embodiments, the present invention includes a production method and compounds used in the production method, the production method comprising one or more steps among Steps 16 to 18 in the present specification using the compounds, wherein, in Formula (I), Formula (IV'), and Formulas (XVIII) to (XX), in Steps 16 to 18, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, and R⁸ is a para-methoxy group.

As one aspect of the above embodiments, the present invention includes a production method and compounds used in the production method, the production method comprising one or more steps among Steps 16 to 18 in the present specification using the compounds, wherein, in Formulas (I), Formula (IV'), and Formulas (XVIII) to (XX) in Steps 16 to 18, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R⁵ is a benzyloxycarbonyl group, an acetyl group, a 4-methoxybenzyl group, or an allyl group, and R⁸ is a para-methoxy group.

As one aspect of the above embodiments, the present invention includes a production method and compounds used in the production method, the production method comprising one or more steps among Steps 19 to 21 in the present specification using the compounds, wherein, in Formula (I), Formula (IV), and Formulas (XXI) to (XXIII) in Steps 19 to 21, R¹ is a hydroxyl protecting group, R² is a hydroxyl protecting group, R³ and R⁴ together form a cyclic protecting group for a diol, R⁵ and R⁶ are amino protecting groups, R⁹ is an alkyl group or a hydrogen atom, and R¹⁰ is an alkyl group or a hydrogen atom.

As one aspect of the above embodiments, the present invention includes a production method and compounds used in the production method, the production method comprising one or more steps among Steps 19 to 21 in the present specification using the compounds, wherein, in Formula (I), Formula (IV), and Formulas (XXI) to (XXIII) in Steps 19 to 21, R¹ is a hydroxyl protecting group, R² is a hydroxyl protecting group, R³ and R⁴ together form a cyclic protecting group for a diol, R⁵ is an amino protecting groups, R⁶ is a hydrogen atom, R⁹ is a hydrogen atom, and R¹⁰ is a hydrogen atom.

As one aspect of the above embodiments, the present invention includes a production method and compounds used in the production method, the production method comprising one or more steps among Steps 19 to 21 in the present specification using the compounds, wherein, in Formulas (I), Formula (IV), and Formulas (XXI) to (XXIII) in Steps 19 to 21, R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, R⁹ is a hydrogen atom, and R¹⁰ is a hydrogen atom.

As one aspect of the above embodiments, the present invention includes a production method and compounds used in the production method, the production method comprising one or more steps among Steps 19 to 21 in the present specification using the compounds, wherein, in Formula (I), Formula (IV), and Formulas (XXI) to (XXIII) in Steps 19 to 21, R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R⁵ is a benzyloxycarbonyl group, an acetyl group, a 4-methoxybenzyl group, or an allyl group, R⁶ is a hydrogen atom, R⁹ is a hydrogen atom, and R¹⁰ is a hydrogen atom.

### <Other Aspects of Compounds>

As another aspect of the compounds of the above embodiments, the present invention includes compounds wherein, in Formula (IV), R¹ is a hydroxyl protecting group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form a cyclic protecting group for a diol, and R⁵ and R⁶ are amino protecting groups.

As another aspect of the compounds of the above embodiments, the present invention includes compounds wherein, in Formula (IV), R¹ is a hydroxyl protecting group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form a cyclic protecting group for a diol, R⁵ is an amino protecting group, and R⁶ is a hydrogen atom.

As another aspect of the compounds of the above embodiments, the present invention includes compounds wherein, in Formula (IV), R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R³ and R⁴ together form an isopropylidene ketal group, a methylene acetal group, an ethylidene acetal group, a benzylidene acetal group, a 4-methoxybenzylidene acetal group, or a 1-benzyloxybenzylidene orthoester group, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, and R⁶ is a hydrogen atom.

As another aspect of the compounds of the above embodiments, the present invention includes compounds wherein, in Formula (IV), R¹ is a benzyl group, a 4,4'-dimethoxytrityl group, or an acetyl group, R² is a benzyl group, a 4,4'-dimethoxytrityl group, or an acetyl group, R³ and R⁴ together form an isopropylidene ketal group, R⁵ is a benzyloxycarbonyl group, an acetyl group, a 4-methoxybenzyl group, or an allyl group, and R⁶ is a hydrogen atom.

As another aspect of the compounds of the above embodiments, the present invention includes compounds wherein, in Formula (XIII), R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a hydroxyl protecting group, R⁵ and R⁶ are amino protecting groups, and B is a nucleic acid base moiety.

As another aspect of the compounds of the above embodiments, the present invention includes compounds wherein, in Formula (XIII), R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a hydroxyl protecting group, R⁵ is an amino protecting group, R⁶ is a hydrogen atom, and B is a nucleic acid base moiety.

As another aspect of the compounds of the above embodiments, the present invention includes compounds wherein, in Formula (XIII), R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R⁵ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, R⁶ is a hydrogen atom, and B is a nucleic acid base moiety.

As another aspect of the compounds of the above embodiments, the present invention includes compounds wherein, in Formula (XIII), R¹ is a benzyl group, a 4,4'-dimethoxytrityl group, or an acetyl group, R² is a benzyl group, a 4,4'-dimethoxytrityl group, or an acetyl group, R⁵ is a benzyloxycarbonyl group, an acetyl group, a 4-methoxybenzyl group, or an allyl group, R⁶ is a hydrogen atom, and B is a nucleic acid base moiety.

As another aspect of the compounds of the above embodiments, the present invention includes compounds wherein, in Formula (XIII), R¹ is a benzyl group, a 4,4'-dimethoxytrityl group, or an acetyl group, R² is a benzyl group, a 4,4'-dimethoxytrityl group, or an acetyl group, R⁵ is a benzyloxycarbonyl group, an acetyl group, a 4-methoxybenzyl group, or an allyl group, R⁶ is a hydrogen atom, and B is adeninyl, guanyl, 2,6-diaminopurinyl, thyminyl, dihydrothyminyl, 2-thiothyminyl, cytosinyl, 5-methylcytosinyl, uracinyl, dihydrouracyl, 5-fluorocytosinyl, xanthinyl, 6-aminopurinyl, 2-aminopurinyl, 6-chloro-2-aminopurinyl, or 6-chloropurinyl.

As another aspect of the compounds of the above embodiments, the present invention includes compounds wherein, in Formula (XIII), R¹ is a benzyl group, a 4,4'-dimethoxytrityl group, or an acetyl group, R² is a benzyl group, a 4,4'-dimethoxytrityl group, or an acetyl group, R⁵ is a benzyloxycarbonyl group, an acetyl group, a 4-methoxybenzyl group, or an allyl group, R⁶ is a hydrogen atom, and B is thyminyl.

As another aspect of the compounds of the above embodiments, the present invention includes compounds wherein, in Formula (XVI), R¹ is a hydroxyl protecting group, R² is a hydroxyl protecting group, R¹² is a hydrogen atom, an alkyl group, an alkoxy group, or an alkoxyalkyl group, R¹⁴ is a hydrogen atom, a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, X is an oxygen atom or NR¹³ (wherein R¹³ is a hydrogen atom or an amino protecting group), and a double line consisting of a solid line and a dotted line represents a single bond or a double bond.

As another aspect of the compounds of the above embodiments, the present invention includes compounds wherein, in Formula (XVI), R¹ is a hydroxyl protecting group, R² is a hydroxyl protecting group, R¹² is a hydrogen atom or a methyl group, R¹⁴ is a hydrogen atom, a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, X is an oxygen atom, and a double line consisting of a solid line and a dotted line represents a double bond.

As another aspect of the compounds of the above embodiments, the present invention includes compounds wherein, in Formula (XVI), R¹ is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R² is a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group, R¹² is a hydrogen atom or a methyl group, R¹⁴ is a benzyloxycarbonyl group, an isobutyryl group, a benzoyl group, a t-butoxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a trifluoroacetyl group, a 4-methoxybenzyl group, an allyl group, a benzyl group, or a 2-nitrobenzenesulfonyl group, X is an oxygen atom, and a double line consisting of a solid line and a dotted line represents a double bond.

As another aspect of the compounds of the above embodiments, the present invention includes compounds wherein, in Formula (XVI), R¹ is a benzyl group, a 4,4'-dimethoxytrityl group, or an acetyl group, R² is a benzyl group, a 4,4'-dimethoxytrityl group, or an acetyl group, R¹² is a hydrogen atom or a methyl group, R¹⁴ is a benzyloxycarbonyl group, an acetyl group, a 4-methoxybenzyl group, or an allyl group, X is an oxygen atom, and a double line consisting of a solid line and a dotted line represents a double bond.

As another aspect of the compounds of the above embodiments, the present invention includes compounds wherein, in Formula (XVI), R¹ is a benzyl group, a 4,4'-dimethoxytrityl group, or an acetyl group, R² is a benzyl group, a 4,4'-dimethoxytrityl group, or an acetyl group, R¹² is a methyl group, R¹⁴ is a benzyloxycarbonyl group, an acetyl group, a 4-methoxybenzyl group, or an allyl group, X is an oxygen atom, and a double line consisting of a solid line and a dotted line represents a double bond.

### <Production methods>

The above production methods are specifically described below. In the present specification, a compound represented by a certain formula, such as a compound represented by Formula (I) or a compound represented by Formula (II), is also referred to as a compound of a certain formula, such as a compound of Formula (I) or a compound of Formula (II).

### (1) Method for producing a compound represented by Formula (IV)

A compound represented by Formula (IV) can be produced as follows: 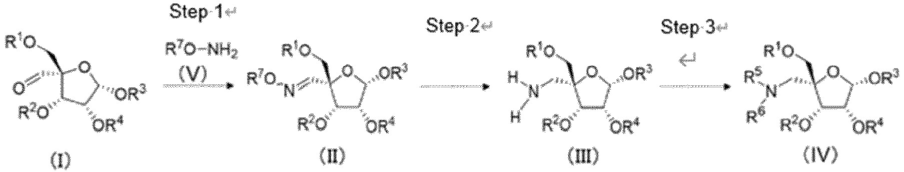 (wherein the symbols have the same meaning as described above).

### <Step 1>

This is a step of obtaining a compound of Formula (II) by subjecting a compound of Formula (I) and a compound of Formula (V) or a salt thereof (such as hydrochloride, sulfate, or phosphate, preferably hydrochloride) to an oximation reaction in an appropriate solvent.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations.

When a salt of a compound of Formula (V) is used, the reaction can be carried out in the presence of a base. Examples of the base include pyridine, sodium acetate, sodium hydroxide, potassium hydroxide, sodium bicarbonate, sodium carbonate, potassium carbonate, potassium bicarbonate, and the like, with pyridine being preferred. The solvent is the same as described above.

Further, when a salt of a compound of Formula (V) is used, it may not be necessary to add a base when an alcohol, a combination of tetrahydrofuran and water, N,N-dimethylformamide, or dioxane is used as the solvent.

An amount of the compound of Formula (V) or the salt thereof used is preferably 1 to 3 molar equivalents relative to 1 mole of the compound of Formula (I). An amount of the base used is preferably 1 to 10 molar equivalents relative to 1 mole of the compound of Formula (I).

The reaction temperature is preferably 40°C to 80°C, particularly 50°C to 70°C.

### <Step 2>

This is a step of obtaining a compound of Formula (III) by subjecting a compound of Formula (II) to a reduction reaction with a reducing agent in an appropriate solvent.

As the solvent, any solvent that does not adversely affect the present reaction may be used. Example thereof include aromatic hydrocarbons (such as benzene, toluene, and xylene), ethers (such as diethyl ether, tetrahydrofuran, and dimethoxyethane), halogenated solvents (such as dichloromethane, and chloroform), and the like. These solvents may also be used in appropriate combinations. Among these, ethers are preferred, with tetrahydrofuran being more preferred.

As the reducing agent, sodium bis(2-methoxyethoxy)aluminum hydride (Red-Al) or lithium aluminum hydride is preferred. When sodium bis(2-methoxyethoxy)aluminum hydride is used, the reaction proceeds without producing by-products, and thus, it is more preferred.

An amount of the reducing agent used is preferably 2 to 3 molar equivalents relative to 1 mole of the compound of Formula (II).

The reaction temperature is preferably 20°C to 60°C, particularly 30°C to 50°C.

### <Step 3>

This is a step of obtaining a compound of Formula (IV) by subjecting a compound of Formula (III) to an amino group protection reaction in an appropriate solvent.

The amino group protection reaction can be carried out under reaction conditions generally known in organic synthetic chemistry (for example, nucleic acid synthesis), for example, can be carried out according to a method described in Theodora W. Greene, Peter G. M. Wuts, "Protective Groups in Organic Synthesis," 4th Ed., John Wiley & Sons, Inc., 2007. For example, it can be carried out using N-carbobenzoxyoxysuccinimide in a solvent in the presence of a base.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Among these, when sodium hydroxide is used as the base, esters, particularly ethyl acetate, are preferred. Further, when potassium carbonate is used as the base, ethers, particularly tetrahydrofuran, are preferred.

Examples of the base include basic inorganic salts such as sodium hydroxide, potassium hydroxide, sodium hydrogen carbonate, sodium carbonate, potassium carbonate, and potassium hydrogen carbonate, with sodium hydroxide or potassium carbonate being preferred. An amount of the base used may be such that the pH of the reaction solution is weakly basic.

An amount of carbobenzoxyoxysuccinimide used is preferably 1 to 1.5 molar equivalents relative to 1 mole of the compound of Formula (III).

The reaction temperature is preferably 0°C to 40°C, particularly 10°C to 30°C.

### (2) Method for producing a compound of Formula (I)

A compound of Formula (I) can be produced as follows: 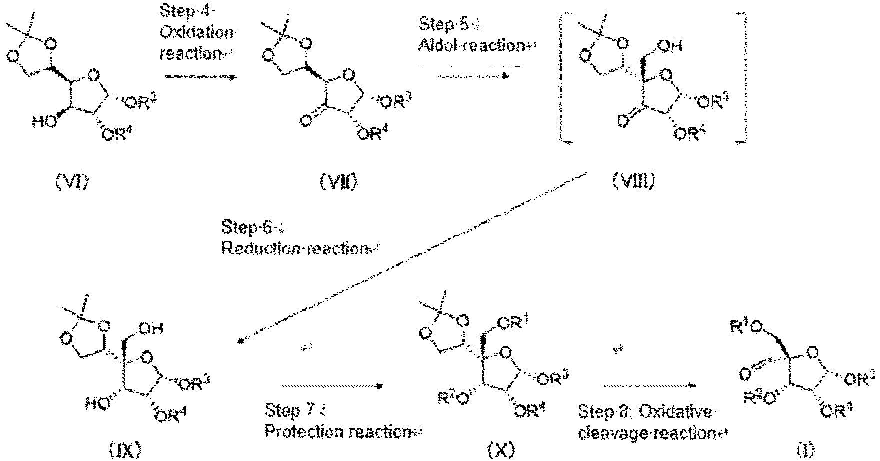 (wherein the symbols have the same meaning as described above).

A compound of Formula (VI) is commercially available, and Steps 4 to 8 can be carried out according to commonly known methods, for example, methods described in Kei Fukuyama et al., Org. Lett., 2015, 17, 828-831.

### (3) Method for producing a compound represented by Formula (XIII) from a compound represented by Formula (IV)

A compound represented by Formula (XIII) can be produced from a compound represented by Formula (IV) or Formula (IV') as follows: 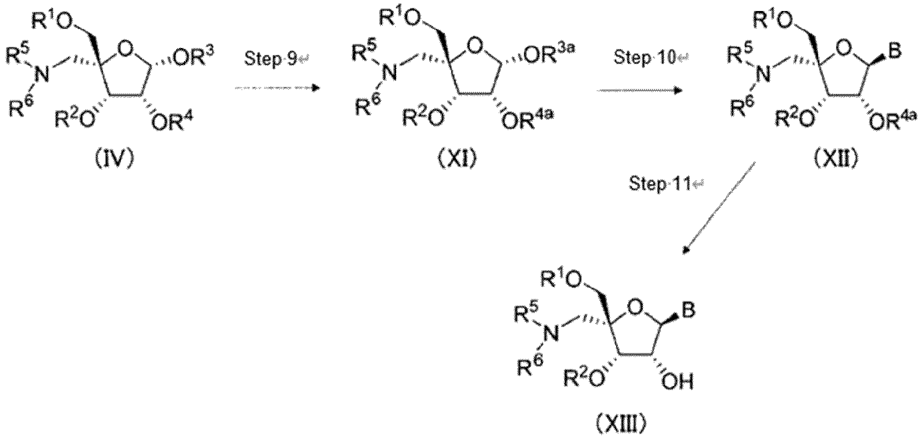 (wherein the symbols have the same meaning as described above).

A compound represented by Formula (IV') is a compound represented by Formula (IV) wherein R⁶ is a hydrogen atom.

### <Step 9>

This is a step of obtaining a compound of Formula (XI) by subjecting a compound of Formula (IV) or Formula (IV') to an acyl conversion reaction of R³ and R⁴ in an appropriate solvent to convert R³ and R⁴ into acyl groups.

The acyl conversion reaction of R³ and R⁴ can be carried out according to methods described in J. Org. Chem., 2011, 76, 9891-9899, WO2017/047816, and the like.

For example, a compound of Formula (XI) can be produced by reacting a compound of Formula (IV) in an appropriate solvent in the presence of an acid anhydride with an acid catalyst. An acid may be added as necessary.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Among these, ethyl acetate is preferably used.

A preferred example of the acid anhydride is acetic anhydride, and an amount thereof is preferably 1 to 10 molar equivalents relative to 1 mole of the compound of Formula (IV).

A preferred example of the acid is acetic acid, and an amount thereof is preferably 1 to 20 molar equivalents relative to 1 mole of the compound of Formula (IV).

A preferred example of the acid catalyst is sulfuric acid, and an amount thereof is preferably 0.01 to 0.20 molar equivalents relative to 1 mole of the compound of Formula (IV).

The reaction temperature is preferably 10 to 40°C.

### <Step 10>

This is a step of obtaining a compound of Formula (XII) by subjecting a compound of Formula (XI) to a glycosylation reaction in an appropriate solvent to introduce a nucleic acid base moiety.

The introduction of a nucleic acid base moiety into a compound of Formula (XI) via a glycosylation reaction can be carried out according to methods described in J. Org. Chem., 2011, 76, 9891-9899, WO2017/047816, and the like.

For example, a compound of Formula (XII) can be produced by reacting a compound of Formula (XI) in an appropriate solvent with N,O-bis(trimethylsilyl)acetamide and a nucleic acid base in the presence of trimethylsilyl trifluoromethanesulfonate.

The nucleic acid base is, for example, thymine (T). In contrast to reacting thymine (T) as the nucleic acid base, various nucleic acid bases other than thymine can be introduced by reacting a different nucleic acid base in place of thymine (T). As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Among these, acetonitrile is preferably used.

The reaction temperature is preferably 60 to 100°C.

### <Step 11>

This is a step of obtaining a compound of Formula (XIII) by subjecting a compound of Formula (XII) to a deprotection reaction of an acyl group in an appropriate solvent.

The deprotection reaction can be carried out according to methods described in J. Org. Chem., 2011, 76, 9891-9899, WO2017/047816, and the like.

For example, the deprotection reaction can be carried out in an appropriate solvent in the presence of a base.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations. Among these, alcohols (such as methanol, ethanol) are preferred.

As the base, methylamine is preferred. An amount of the base used is preferably 3 to 30 molar equivalents relative to 1 mole of the compound of Formula (VIII).

The reaction temperature is preferably 0°C to 50°C, particularly 15°C to 30°C.

Further, the deprotection reaction can also be carried out by hydrolysis.

### (4) Method for producing a compound represented by Formula (XVII') or a compound represented by Formula (XVII") from a compound represented by Formula (XIII')

A compound represented by Formula (XVII') or a compound represented by Formula (XVII") can be produced from a compound represented by Formula (XIII') as follows: 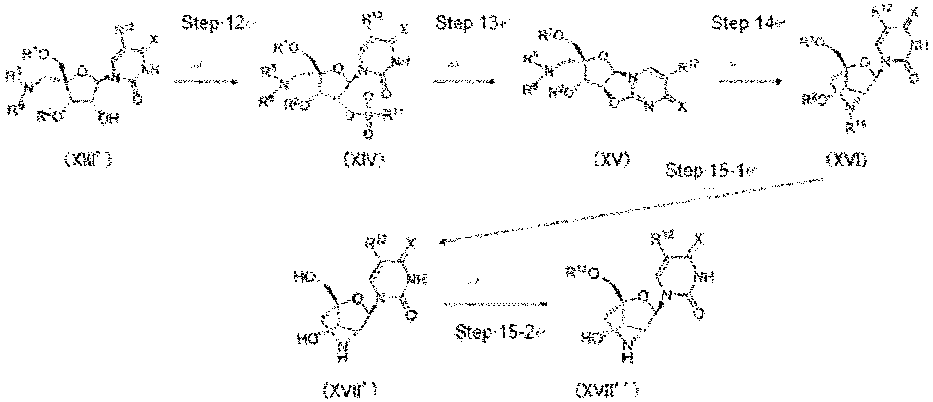 (wherein the symbols have the same meaning as described above)
A compound represented by Formula (XIII') is a compound represented by Formula (XIII) wherein B is represented by Formula (XXIV): 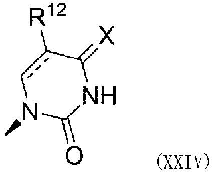 [wherein
R¹² represents a hydrogen atom, an alkyl group, an alkoxy group, or an alkoxyalkyl group,
X represents an oxygen atom or NR¹³ (wherein R¹³ represents a hydrogen atom or an amino protecting group), and
a double line consisting of a solid line and a dotted line represents a single bond or a double bond].

A compound represented by Formula (XVII') is a compound represented by Formula (XVII) wherein R¹ is a hydrogen atom.

A compound represented by Formula (XVII") is a compound represented by Formula (XVII) wherein R¹ is R^{1a}.

### <Step 12>

This is a step of obtaining a compound of Formula (XIV) by subjecting a compound of Formula (XIII') to a sulfonylation reaction in an appropriate solvent.

The sulfonylation reaction can be carried out according to methods described in WO2017/047816 and the like. For example, a compound of Formula (XIV) can be produced by reacting a compound of Formula (XIII') in an appropriate solvent in the presence of a hydroxyl group activating agent and a base.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Among these, ethers are preferred, with tetrahydrofuran being more preferred.

Examples of the hydroxyl group activating agent include methanesulfonyl chloride, trifluoromethanesulfonyl chloride, p-toluenesulfonyl chloride, 2-nitrobenzenesulfonyl chloride, methanesulfonic anhydride, and trifluoromethanesulfonic anhydride, with methanesulfonyl chloride being preferred.

Examples of the base include triethylamine, pyridine, diisopropylethylamine, 2,6-lutidine, 4-dimethylaminopyridine, and the like, with triethylamine or pyridine being preferred.

An amount of the hydroxyl group activating agent used is preferably 1 to 2 molar equivalents relative to 1 mole of the compound of Formula (XIII'). An amount of the base used is preferably 1 to 4 molar equivalents relative to 1 mole of the compound of Formula (XIII').

The reaction temperature is preferably 5°C to 45°C, particularly 15°C to 35°C.

### <Step 13>

This is a step of obtaining a compound of Formula (XV) by subjecting a compound of Formula (XIV) to a cyclization reaction in an appropriate solvent.

The cyclization reaction can be carried out in an appropriate solvent in the presence of a base.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Further, pyridine can also be used as the solvent.

Examples of the base include diazabicycloundecene, sodium hydride, and the like, with diazabicycloundecene being preferred.

An amount of the base used is preferably 1 to 4 molar equivalents for diazabicycloundecene and 1 molar equivalent for sodium hydride relative to 1 mole of the compound of Formula (XIV).

The reaction temperature is preferably 5°C to 45°C, particularly 15°C to 35°C.

The compound of Formula (XV) obtained by this step can be isolated as crystals. However, among these, particularly, a compound of Formula (XV') (Compound 14 in Example 1 (11) to be described later) wherein R¹ is a benzyl group, R² is a benzyl group, R⁵ is a benzyloxycarbonyl group, R⁶ is a hydrogen atom, X is an oxygen atom, R¹² is a methyl group, and a double line consisting of a solid line and a dotted line represents a double bond can be preferably isolated as crystals. Therefore, in this step, it is preferred to use a corresponding compound of Formula (XIV') (wherein R¹¹ is a methyl group, and the like) to produce the compound of Formula (XV').

Examples of methods for crystallizing a compound of Formula (XV'), among compounds of Formula (XV), include a method of crystallizing by adding a poor solvent, a method of crystallizing from a mixture of a good solvent and a poor solvent, a method of crystallizing by reducing solubility of the compound by cooling, or a method combining these. The crystallization in this step can be efficiently carried out by adding water dropwise as a poor solvent and cooling to about 10°C.

The powder X-ray diffraction pattern of the crystals of the compound of Formula (XV'), measured using CuKα radiation, is as shown in Table 1 and Fig. 1. However, regarding the powder X-ray diffraction pattern, it should be noted that the diffraction peak intensities in the experimental pattern can vary and fluctuate due to preferred orientation in a prepared sample, as is commonly known in the art.

### <Step 14>

This is a step of obtaining a compound of Formula (XVI) by subjecting a compound of Formula (XV) to a ring-closing reaction in an appropriate solvent.

The ring-closing reaction can be carried out in an appropriate solvent in the presence of a base.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Among these, an aprotic polar solvent is preferred, with N,N-dimethylformamide being more preferred.

An example of the base is sodium hydride.

An amount of the base used is preferably 1 to 3 molar equivalents relative to 1 mole of the compound of Formula (XV).

The reaction temperature is preferably -10°C to 30°C, particularly 0°C to 10°C.

### <Step 15-1>

This is a step of obtaining a compound of Formula (XVII') by subjecting a compound of Formula (XVI) to a deprotection reaction in an appropriate solvent.

The deprotection reaction can be carried out under reaction conditions generally known in organic synthetic chemistry (for example, nucleic acid synthesis), for example, can be carried out according to a method described in Theodora W. Greene, Peter G. M. Wuts, "Protective Groups in Organic Synthesis," 4th Ed., John Wiley & Sons, Inc., 2007. For example, it can be carried out in an appropriate solvent in the presence of palladium hydroxide on carbon under a hydrogen atmosphere.

As the solvent, any solvent that does not adversely affect the present reaction may be used. Example thereof include aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations. Among these, alcohols are preferred, with methanol being more preferred.

An amount of palladium hydroxide on carbon used is preferably 1 to 30% by weight relative to the compound of Formula (XVI).

The reaction temperature is preferably 0°C to 30°C.

### <Step 15-2>

This is a step of subjecting a compound of Formula (XVII') to a hydroxyl group protection reaction in an appropriate solvent.

The hydroxyl group protection reaction can be carried out under reaction conditions generally known in organic synthetic chemistry (for example, nucleic acid synthesis), for example, can be carried out according to a method described in WO2017/047816. The present reaction can be carried out, for example, using 4,4'-dimethoxytrityl chloride in a solvent in the presence of a base.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations. Further, pyridine can also be used as the solvent.

Examples of the base include pyridine, sodium hydroxide, potassium hydroxide, sodium bicarbonate, sodium carbonate, potassium carbonate, potassium bicarbonate, and the like, with pyridine being preferred.

The reaction temperature is preferably 0°C to 50°C, particularly 15°C to 30°C.

### (5) Method for producing a compound represented by Formula (IV) from a compound represented by Formula (I) (alternative method)

The production of a compound represented by Formula (IV) or Formula (IV') from a compound represented by Formula (I) is a method different from the method described in (3), and can also be carried out, for example, by the following Scheme 1 or Scheme 2:
<Alternative Method 1> 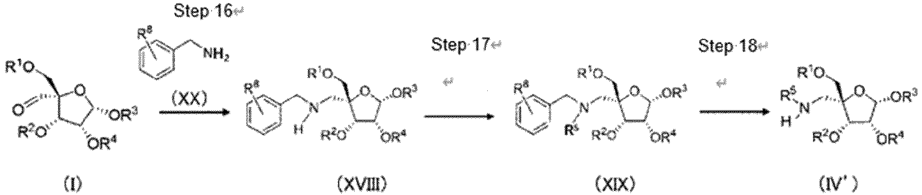 (wherein the symbols have the same meaning as described above).
<Alternative Method 2> 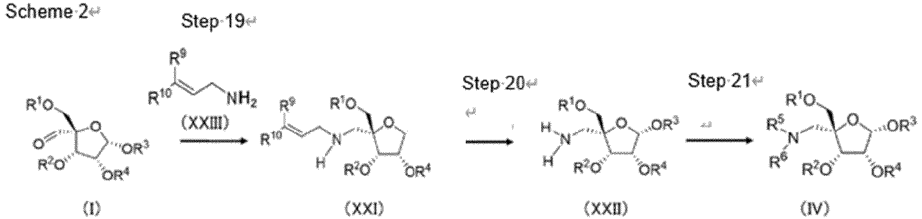 (wherein the symbols have the same meaning as described above)

### <Step 16>

This is a step of obtaining a compound of Formula (XVIII) by reacting a compound of Formula (I) with a compound of Formula (XX) in an appropriate solvent, followed by a treatment with a reducing agent.

As the solvent, any solvent that does not adversely affect the present reaction may be used. Examples thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations.

As the reducing agent, sodium cyanoborohydride, sodium triacetoxyborohydride, and sodium borohydride are preferred, with sodium triacetoxyborohydride being more preferred.

An amount of the compound of Formula (XX) used is preferably 1 to 2 molar equivalents relative to 1 mole of the compound of Formula (I). An amount of the reducing agent used is preferably 1 to 3 molar equivalents relative to 1 mole of the compound of Formula (I).

The reaction temperature is preferably 0°C to 50°C, particularly 15°C to 30°C.

### <Step 17>

This is a step of obtaining a compound of Formula (XIX) by subjecting a compound of Formula (XVIII) to an amino group protection reaction in an appropriate solvent. The protection reaction can be carried out according to a method described in Theodora W. Greene, Peter G. M. Wuts, "Protective Groups in Organic Synthesis," 4th Ed., John Wiley & Sons, Inc., 2007, or the like. For example, it can be carried out using benzyl chloroformate or N-carbobenzoxyoxysuccinimide in a solvent.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations.

An amount of benzyl chloroformate or N-carbobenzoxyoxysuccinimide used is preferably 1 to 2 molar equivalents relative to 1 mole of the compound of Formula (XVIII).

The reaction temperature is preferably 0°C to 50°C, particularly 15°C to 30°C.

### <Step 18>

This is a step of obtaining a compound of Formula (IV') by subjecting a compound of Formula (XIX) to a deprotection reaction in an appropriate solvent. The deprotection reaction can be carried out according to a method described in Theodora W. Greene, Peter G. M. Wuts, "Protective Groups in Organic Synthesis," 4th Ed., John Wiley & Sons, Inc., 2007, or the like. For example, it can be carried out using ammonium hexanitratocerate (IV) in a solvent.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations.

An amount of ammonium hexanitratocerate (IV) used is preferably 1 to 3 molar equivalents relative to the compound of Formula (XIX).

The reaction temperature is preferably 0°C to 50°C, particularly 0°C to 10°C, for favorable progress.

### <Step 19>

This is a step of obtaining a compound of Formula (XXI) by reacting a compound of Formula (I) with a compound of Formula (XXIII) in an appropriate solvent, followed by a treatment with a reducing agent.

As the solvent, any solvent that does not adversely affect the present reaction may be used. Examples thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations.

As the reducing agent, sodium cyanoborohydride, sodium triacetoxyborohydride, and sodium borohydride are preferred, with sodium triacetoxyborohydride being more preferred.

An amount of the compound of Formula (XXIII) or a salt thereof used is preferably 1 to 2 molar equivalents relative to the compound of Formula (I). An amount of the reducing agent used is preferably 1 to 3 molar equivalents relative to 1 mole of the compound of Formula (I).

The reaction temperature is preferably -25°C to 50°C, particularly 15°C to 30°C.

### <Step 20>

This is a step of obtaining a compound of Formula (XXII) by subjecting a compound of Formula (XXI) to a deprotection reaction. The deprotection reaction can be carried out according to a method described in Theodora W. Greene, Peter G. M. Wuts, "Protective Groups in Organic Synthesis," 4th Ed., John Wiley & Sons, Inc., 2007, or the like. For example, a compound of Formula (XXI) can be subjected to a deprotection reaction in an appropriate solvent in the presence of 1,3-dimethylbarbituric acid or a combination of polymethylhydrosiloxane and zinc(II) chloride, using a catalyst (tetrakis(triphenylphosphine)palladium(0)). Further, it can be carried out in the presence of 2-mercaptobenzoic acid using a catalyst (1,4-bis(diphenylphosphino)butane-palladium(II) chloride or bis(dibenzylideneacetone)palladium(0)).

Or, a deprotection reaction can be carried out using a catalyst (tris(triphenylphosphine)rhodium(I) chloride or benzylidene-bis(tricyclohexylphosphine)dichlororuthenium(II)).

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), alcohols (such as methanol, ethanol, and isopropanol), and the like. These solvents may also be used in appropriate combinations.

An amount of 1,3-dimethylbarbituric acid, polymethylhydrosiloxane, zinc(II) chloride, or 2-mercaptobenzoic acid used is preferably 1 to 3 molar equivalents relative to the compound of Formula (XXI). An amount of the catalyst used is preferably 0.05 to 0.1 molar equivalents relative to the compound of Formula (XXI).

The reaction temperature is preferably 0°C to 50°C, particularly 30°C to 50°C. The reaction temperature for the reaction using a catalyst (tris(triphenylphosphine)rhodium(I) chloride or benzylidene-bis(tricyclohexylphosphine)dichlororuthenium(II)) is preferably 80°C to 120°C.

### <Step 21>

This is a step of obtaining a compound of Formula (IV) by subjecting a compound of Formula (XXII) to an amino group protection reaction. The protection reaction can be carried out according to a method described in Theodora W. Greene, Peter G. M. Wuts, "Protective Groups in Organic Synthesis," 4th Ed., John Wiley & Sons, Inc., 2007, or the like. For example, a compound of Formula (IV) can be produced by reacting a compound of Formula (XXII) in an appropriate solvent with benzyl chloroformate or N-carbobenzoxyoxysuccinimide.

As the solvent, any solvent that does not adversely affect the present reaction may be used. Example thereof include aromatic hydrocarbons (such as benzene, toluene, and xylene), aprotic polar solvents (such as N,N-dimethylformamide, dimethyl sulfoxide, and N-methyl-2-pyrrolidone), nitriles (such as acetonitrile), ketones (such as acetone, and methyl ethyl ketone), esters (such as ethyl acetate, and isopropyl acetate), ethers (such as diethyl ether, tetrahydrofuran, and dimethoxyethane), halogenated solvents (such as dichloromethane, and chloroform), and the like. These solvents may also be used in appropriate combinations.

An amount of benzyl chloroformate or N-carbobenzoxyoxysuccinimide used is preferably 1 to 2 molar equivalents relative to the compound of Formula (XVIII).

The reaction temperature is preferably 0°C to 50°C, particularly 15°C to 30°C.

### (6) Method for producing a compound represented by Formula (XVII') or a compound represented by Formula (XVII") from a compound represented by Formula (VI)

A compound represented by Formula (XVII') or a compound represented by Formula (XVII") can be produced from a compound represented by Formula (VI) as follows: 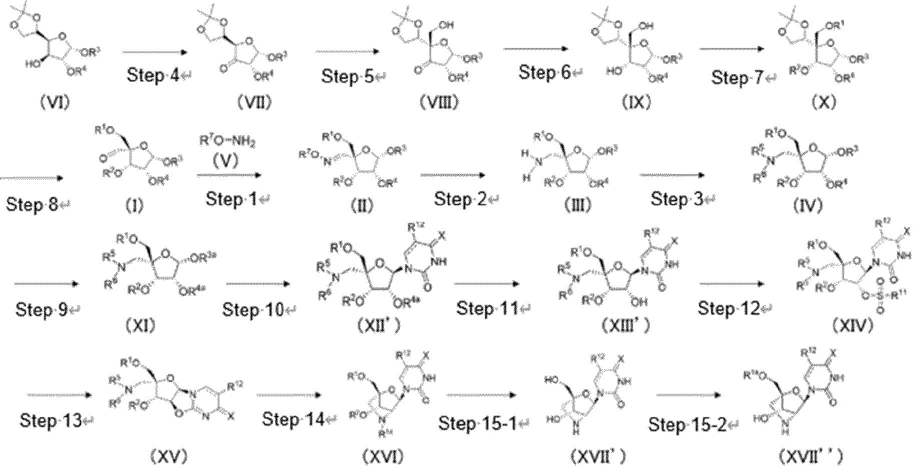 (wherein the symbols have the same meaning as described above).

Steps 1 to 15-2 are as described in (1) to (4) above.

In the above production method, Steps 1 to 13 each allow for isolation of a target compound. Further, in each of Steps 1 to 13, without isolating a reaction product, a reaction solution containing a crude reaction product can be used as is in the next step, or a concentrated reaction solution can be used in the next step. The compound of Formula (XV) obtained in this manner can be isolated and purified as crystals. Further, Steps 14 to 15-2 each allow for isolation of a target compound. Further, in each of Steps 14 to 15-2, without isolating a reaction product, a reaction solution containing a crude reaction product can be used as is in the next step, or a concentrated reaction solution can be used in the next step.

In the present specification, when compounds, intermediate compounds, starting material compounds, and the like described above have a functional group (for example, a hydroxyl group, an amino group, a carboxyl group, or the like), it can be protected with a protecting group commonly used in organic synthetic chemistry according to a method described in Theodora W. Greene, Peter G. M. Wuts, "Protective Groups in Organic Synthesis," 4th ed., John Wiley & Sons, Inc., 1999, or the like, and after the reaction, a target compound can be obtained by removing the protecting group.

### (7) Compounds obtained in the production methods of (1) to (6)

In the production methods of (1) to (6) described above, the compounds represented by Formula (II), Formula (III), Formula (IV), Formula (XI), Formula (XII), Formula (XII'), Formula (XIII), Formula (XIII'), Formula (XIV), Formula (XIV'), Formula (XV), Formula (XV'), and Formula (XVI) are each a novel compound.

When isolating compounds obtained in the steps of the above production methods, all compounds can also be obtained as salts. The salts may be any salts that can be commonly used industrially, and examples thereof include inorganic acid salts such as hydrochloride, sulfate, phosphate, and hydrobromide; organic acid salts such as acetate, fumarate, oxalate, citrate, methanesulfonate, benzenesulfonate, tosylate, and maleate; alkali metal salts such as a sodium salt and a potassium salt; and the like. Conversion to such salts can be carried out according to a conventional method.

### (8) Method for producing a modified oligonucleotide or a salt thereof from a compound represented by Formula (XVII")

From a compound represented by Formula (XVII"), ALNA[Ms] amidite can be produced according to a known method (for example, as described in WO2017/047816, WO2020/100826, or the like). A modified oligonucleotide or a salt thereof represented by the following formula: Formula: 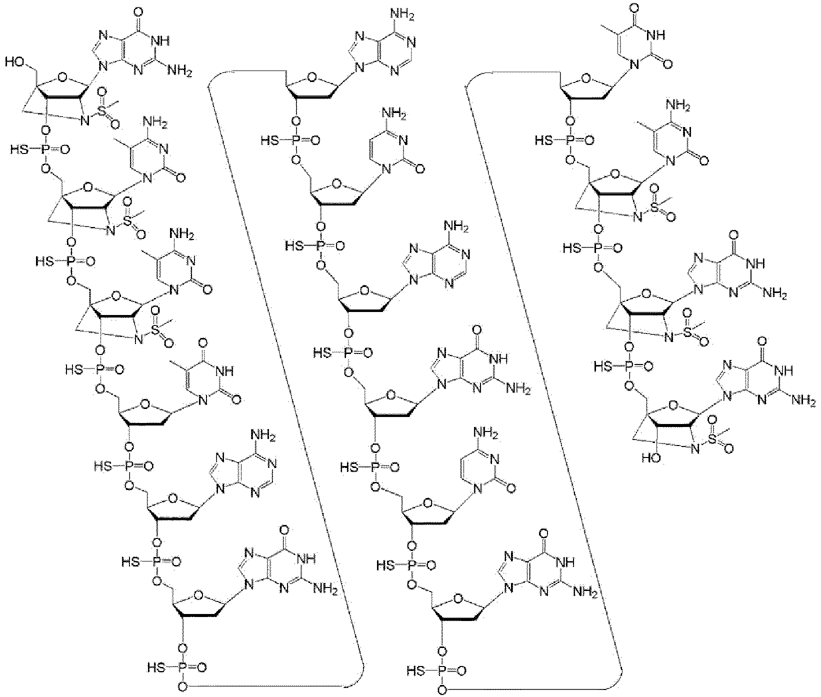 or Formula: 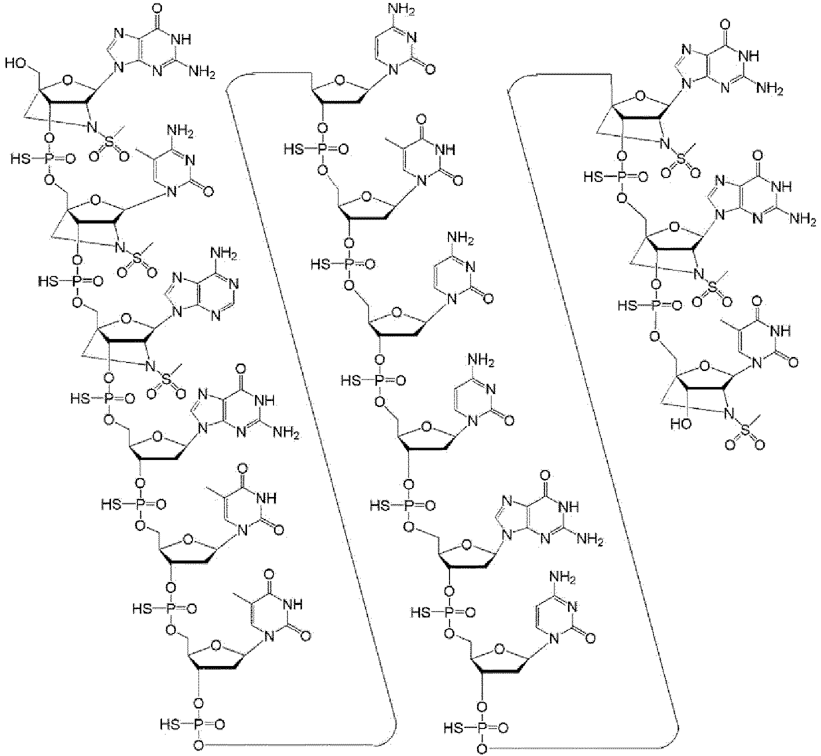 can be produced by subjecting the obtained ALNA[Ms] amidite and a commercially available amidite for DNA synthesis (for example, N6-Benzoyl-DMT-2'-deoxyadenosine-3'-CE Phosphoramidite, N2-Isobutyryl-5'-O-(4,4'-dimethoxytrityl)-2'-deoxyguanosine-3'-cyanoethyl Phosphoramidite, N2-Dimethylformamidine-5'-O-(4,4'-dimethoxytrityl)-2'-deoxyguanosine-3'-cyanoethyl Phosphoramidite, N4-Benzoyl-5'-O-(4,4'-dimethoxytrityl)-2'-deoxycytidine-3'-cyanoethyl Phosphoramidite, 5'-O-(4,4'-Dimethoxytrityl)-thymidine-3'-cyanoethyl Phosphoramidite) to a phosphoramidite method according to a commonly known method (for example, as described in WO2020/100826, WO2020/203880, or the like), and, as necessary, subjecting the product to a thioation reaction of a phosphodiester bond according to a commonly known method. Specific examples of the salt include a sodium salt and a potassium salt, with a sodium salt being preferred.

A step of producing ALNA[Ms] amidite from a compound represented by Formula (XVII"), for example, includes a step of subjecting a compound represented by Formula (XVII") to (a) introduction of a hydroxyl protecting group and any nucleic acid base exchange reaction (transglycosylation reaction), (b) sulfonamidation on an imino nitrogen atom, (c) deprotection of the hydroxyl protecting group, and (d) phosphoramidite formation to produce the desired ALNA[Ms] amidite.

ALNA[Ms] is represented by the following structural formula:

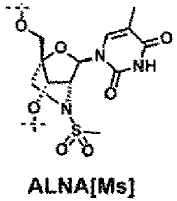

### <(a) Introduction of a hydroxyl protecting group and any nucleic acid base exchange reaction (transglycosylation reaction)>

The introduction of a hydroxyl protecting group can be carried out under reaction conditions well known in organic synthetic chemistry (for example, nucleic acid synthesis), depending on the type of protecting group. For example, when the hydroxyl protecting group is a silyl-type protecting group (for example, TMS), a silylating agent (for example, BSA, hexamethyldisilazane, or TMS chloride) can be used. In this case, a Lewis acid (for example, TMSOTf, TBSOTf, or tin chloride) may be added.

Relative to a reaction substrate, the silylating agent can be used in an amount of about 1 to about 20 molar equivalents, and the Lewis acid can be used in a catalytic amount (about 0.05 molar equivalents) to 2 molar equivalents.

As the solvent, any solvent that does not adversely affect the reaction may be used, and the reaction can be carried out in an appropriate solvent (for example, ethers such as THF; halogenated hydrocarbons such as dichloromethane, dichloroethane, or chloroform; hydrocarbons such as benzene or toluene; acetonitrile; water; mixtures thereof; or the like). The reaction temperature is preferably from 0°C to high temperatures, particularly from room temperature to about 60°C, for favorable progress.

Further, by a nucleic acid base exchange reaction (transglycosylation reaction), a nucleic acid base moiety (thymine (T)) of the compound represented by Formula (XVII") can be substituted with another optionally protected nucleic acid base moiety (for example, adenine (A), guanine (G), uracil (U), cytosine (C), or 5-methylcytosine (MeC)). This base exchange reaction can be carried out in the presence of a Lewis acid (for example, TMSOTf, or TBSOTf), and the reaction can be promoted by reacting a silylating agent.

### <(b) Sulfonamidation on an imino nitrogen atom>

Examples of a sulfonamidation reagent used in the sulfonamidation reaction include alkylsulfonyl halide reagents (for example, methanesulfonyl chloride (MsCl)), alkylsulfonic anhydrides (for example, methanesulfonic anhydride), aromatic sulfonyl halide reagents (for example, benzenesulfonyl chloride), and aromatic sulfonic anhydrides (for example, benzenesulfonic anhydride), and these can be used in the presence of a base (for example, triethylamine, or DIPEA).

Relative to a reaction substrate, the sulfonamidation reagent can be used in an amount ranging from an approximately equivalent molar amount to a somewhat excess equivalent molar amount (for example, about 1.0 molar equivalent).

As the solvent, any solvent that does not adversely affect the reaction may be used, and the reaction can be carried out in an appropriate solvent (ethers such as THF; halogenated hydrocarbons such as dichloromethane, dichloroethane, or chloroform; hydrocarbons such as benzene or toluene; acetonitrile; water; mixtures thereof; or the like). The reaction temperature is preferably from -25°C to room temperature, particularly from 0°C to room temperature, for favorable progress.

### <(c) Deprotection of the hydroxyl protecting group>

The deprotection reaction of the hydroxyl group can be carried out under appropriate reaction conditions (for example, reagents and the like) depending on the type of protecting group. For example, when the hydroxyl protecting group is a silyl-type protecting group (for example, TMS), it can be carried out by a treatment via hydrolysis under acidic conditions (for example, acetic acid-THF-water) or with a fluoride ion-donating reagent (for example, TBAF).

As the solvent, any solvent that does not adversely affect the reaction may be used, and the reaction can be carried out in an appropriate solvent (for example, ethers such as THF; halogenated hydrocarbons such as dichloromethane; water; mixtures thereof; or the like). The reaction temperature is preferably -25°C to 100°C, particularly 0°C to 50°C, for favorable progress.

### <(d) Phosphoramidite formation>

The phosphoramidite formation can be carried out under reaction conditions (for example, reagents) generally known in organic synthesis (particularly, nucleic acid synthesis).

Examples of a phosphoramidite formation reagent include, but are not limited to, the following formula:

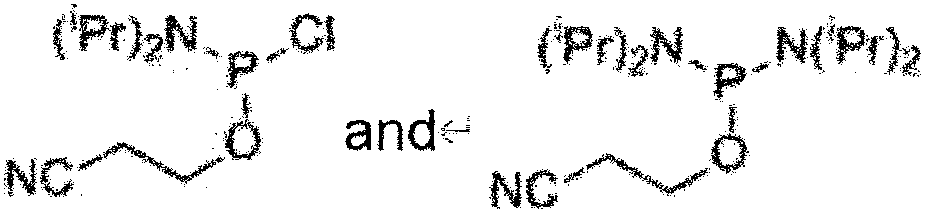

The phosphoramidite formation reaction may be carried out, as appropriate, in the presence of a base (for example, DIPEA) or an acid (for example, diisopropylammonium tetrazolide, or 4,5-dicyanoimidazole).

Relative to a reaction substrate, the phosphoramidite formation reagent can be used in an amount of 1 to 10 molar equivalents, preferably 1 to 5 molar equivalents (for example, 3 molar equivalents).

As the solvent, any solvent that does not adversely affect the reaction may be used, and the reaction can be carried out in an appropriate solvent (for example, ethers such as THF; halogenated hydrocarbons such as dichloromethane, dichloroethane, or chloroform; acetonitrile; mixtures thereof; or the like). The reaction temperature is preferably from -25°C to 60°C, particularly from 0°C to room temperature, for favorable progress.

In the step of subjecting the ALNA[Ms] amidite and the amidite for DNA synthesis to the phosphoramidite method, an oligomerization reaction is carried out. After carrying out the oligomerization reaction, a modified oligonucleotide or a salt thereof can be produced by, as necessary, deprotecting an amino protecting group and a hydroxyl protecting group.

In the synthesis of an oligonucleotide using the phosphoramidite method, an oxidizing agent is used when converting a phosphite group to a phosphate group. When DDTT (((dimethylamino-methylidene)amino)-3H-1,2,4-dithiazoline-3-thione) or the Beaucage reagent (3H-1,2-benzodithiol-3-one-1,1-dioxide) is used instead of the oxidizing agent at this stage, a phosphorothioate oligomer having a protecting group is obtained, in which an oxygen atom at P=O of a phosphate group is replaced with sulfur as in P=S to form a thiophosphate group.

In the step of subjecting a phosphodiester bond to a thioation reaction as necessary, a commonly known thioation reagent can be used. Examples of commercially available thioation reagents include, but are not limited to, Sulfurizing Reagent II (Glen Research Corporation).

All documents, patent applications, and technical standards mentioned in the present specification are incorporated herein by reference to the same extent as when each individual document, patent application, and technical standard is specifically and individually indicated to be incorporated by reference.

### [Examples]

The present invention is specifically described below by way of Examples and the like. However, the present invention is not limited thereto in any way.

Abbreviations to be used respectively represent the following meanings.
Bn: Benzyl
Cbz: Benzyloxycarbonyl
Ac: Acetyl
DMTr: 4,4'-Dimethoxytrityl

Identification of compounds was performed by LCMS, NMR spectroscopy, high-performance liquid chromatography (HPLC), or the like. In NMR, for proton nuclear magnetic resonance (¹H-NMR), an instrument with a resonance frequency of 400 MHz was used. Symbols used in NMR are as follows: s represents a singlet, d represents a doublet, dd represents a double doublet, and J represents a coupling constant.

### Example 1: Methods for producing Compounds 2 to 17 and these compounds

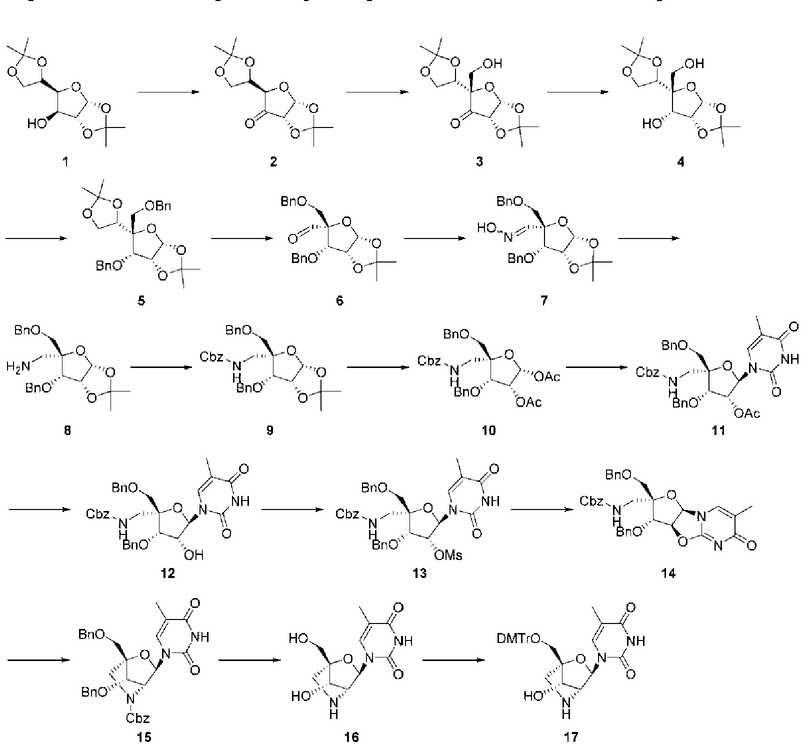

### (1) Synthesis of Compound 2

(1)-1: Under a nitrogen atmosphere, Compound 1 (44.2 g, 170 mmol) and nor-AZADO (1.2 mg, 0.0086 mmol) were dissolved in dichloromethane (22.8 mL), and a saturated aqueous sodium bicarbonate solution (114 mL) and potassium bromide (2.00 g, 16.9 mmol) were added. Compound 1 was obtained as a commercially available product. The mixture was cooled to 0°C, and a mixed solution of a 12% (w/w) aqueous sodium hypochlorite solution (178 mL) and a saturated aqueous sodium bicarbonate solution (91 mL) was added dropwise over 20 minutes, followed by stirring at 0°C for 40 minutes. A mixed solution of nor-AZADO (1.2 mg, 0.0086 mmol), dichloromethane (22.8 mL), a 12% (w/w) aqueous sodium hypochlorite solution (33 mL), and a saturated aqueous sodium bicarbonate solution (17 mL) was added dropwise, and the mixture was further stirred at the same temperature for 30 minutes. At 0°C, a 30% (w/w) aqueous sodium thiosulfate solution (50 mL) and dichloromethane (400 mL) were added, and an organic layer was separated. An aqueous layer was extracted with dichloromethane (6 × 200 mL), and organic layers were combined and dried over anhydrous magnesium sulfate. After filtration, the filtrate was concentrated under reduced pressure to obtain a crude product of Compound 2 (43.1 g) as a pale yellow viscous material. The obtained crude product was used as is in the next step (2)-1.

MS (ESI): m/z = 259 (M+H)⁺
(1)-2 (Non-Isolation Method): Under a nitrogen atmosphere, Compound 1 (14.8 g, 56.9 mmol), *ⁿ*Bu₄HSO₄ (978 mg, 2.88 mmol), and nor-AZADO (9.5 mg, 0.069 mmol) were dissolved in dichloromethane (30 mL). The mixture was cooled to 0°C, and an aqueous sodium hypochlorite solution, prepared from sodium hypochlorite pentahydrate (15.1 g, 91.8 mmol) and tap water (7.8 mL), was added dropwise over 30 minutes, followed by stirring at 0°C for 1 hour. A 30% (w/w) aqueous sodium thiosulfate solution (30 mL) was added, and an organic layer was separated. Tap water (30 mL) was added, an aqueous layer was extracted with dichloromethane (3 × 30 mL), and organic layers were combined and concentrated under reduced pressure with solvent exchange to tetrahydrofuran, yielding a solution of Compound 2 as a yellow solution. The obtained solution was used as is in the next step (2)-2.

### (2) Synthesis of Compound 4

(2)-1: Under a nitrogen atmosphere, the crude product of Compound 2 (43.1 g) obtained in (1)-1 was dissolved in tetrahydrofuran (340 mL). Paraformaldehyde (10.2 g, 340 mmol) and potassium carbonate (47.0 g, 340 mmol) were added, and the mixture was stirred at room temperature for 3.5 hours, followed by further stirring at 30°C for 3 hours. The mixture was cooled to room temperature, insoluble matter was removed by filtration, and a solid on a filter paper was washed with tetrahydrofuran (50 mL). The filtrates were combined, and tap water (69 mL) was added. After that, the mixture was cooled to 0°C and sodium borohydride (12.9 g, 340 mmol) was added over 15 minutes. After stirring at 0°C for 30 minutes, the reaction mixture was allowed to stand, and an organic layer was separated. An aqueous layer was extracted with tetrahydrofuran (3 × 80 mL), and organic layers were combined and dried over anhydrous magnesium sulfate. After filtration, the filtrate was concentrated under reduced pressure, and the residue was dissolved in ethyl acetate (200 mL). The solution was passed through a column packed with silica gel 60 N (neutral, 90 g), followed by elution with ethyl acetate (600 mL). The eluates were combined and concentrated under reduced pressure to obtain a crude product of Compound 4 (44.0 g) as a white solid. The obtained crude product was suspended in a mixed solution of n-hexane (85 mL) and diethyl ether (15 mL), and stirred at room temperature for 2 hours, and then, the solid was collected by filtration. The solid was washed with *n*-hexane-diethyl ether (9:1, v/v) (2 × 50 mL) and dried under reduced pressure to obtain Compound 4 (41.3 g, 142 mmol, yield 83% over two steps ((1)-1 and (2)-1) from Compound 1) as a white solid.

MS (ESI): m/z = 291 (M+H)⁺
¹H NMR (400 MHz, CDCl₃) δ 5.92 (d, J = 4.0 Hz, 1H), 4.73 (dd, J = 6.4, 4.0 Hz, 1H), 4.58 (dd, J = 7.4, 6.8 Hz, 1H), 4.32 (d, J = 6.8 Hz, 1H), 4.16 (dd, J = 9.4, 7.4 Hz, 1H), 3.92 (dd, J = 9.2, 6.4 Hz, 1H), 3.79 (dd, J = 11.6, 4.4 Hz, 1H), 3.61 (dd, J = 11.8, 7.8 Hz, 1H), 2.74 (d, J = 6.8 Hz, 1H), 1.98 (dd, J = 7.8, 4.6 Hz, 1H), 1.63 (s, 3H), 1.47 (s, 3H), 1.41 (s, 3H), 1.35 (s, 3H).

(2)-2 (Non-Isolation Method): Under a nitrogen atmosphere, tetrahydrofuran (73.5 mL) was added to a tetrahydrofuran solution containing Compound 2 obtained in (1)-2. Paraformaldehyde (3.6 g, 102 mmol) and potassium carbonate (15.7 g, 114 mmol) were added, and the mixture was stirred at 30°C for 21 hours. Further, paraformaldehyde (1.8 g, 51 mmol) and potassium carbonate (7.9 g, 57 mmol) were added, and the mixture was stirred at 30°C for 21 hours, and then cooled to 20°C. Insoluble matter was removed by filtration, and the solid on the filter paper was washed with tetrahydrofuran (7 × 30 mL). The filtrates were combined, and concentrated under reduced pressure, and tetrahydrofuran (30 mL) and tap water (22 mL) were added. After that, the mixture was cooled to 0°C, and sodium borohydride (4.3 g, 114 mmol) was added over 16 minutes. The mixture was warmed to 20°C, stirred for 1 hour, and then allowed to stand, and an organic layer was separated. An aqueous layer was extracted with tetrahydrofuran (2 × 30 mL), and organic layers were combined and concentrated under reduced pressure, and the residue was dissolved in ethyl acetate (84 mL), and the solution was passed through a short column packed with silica gel (40 µm, 13.96 g). After elution with ethyl acetate (168 mL), the eluate was concentrated under reduced pressure to obtain a crude product of Compound 4 (10.3 g) as a yellow-green solid.

### (3) Synthesis of Compound 5

(3)-1: Under a nitrogen atmosphere, tetrahydrofuran (140 mL) and 60% (w/w) sodium hydride (dispersion in paraffin liquid) (14.3 g, 357 mmol) were mixed at 0°C. Compound 4 (41.3 g, 142 mmol), dissolved in tetrahydrofuran (140 mL), was added dropwise at 0°C, and the mixture was washed with tetrahydrofuran (35 mL). After stirring at 0°C for 10 minutes, benzyl bromide (37.5 mL, 315 mmol) was added, and the mixture was further stirred at 50°C for 4 hours. The reaction mixture was cooled to 0°C and added dropwise to tap water (700 mL) cooled to 0°C. Ethyl acetate (700 mL) was added, and an organic layer was separated. An aqueous layer was extracted with ethyl acetate (2 × 350 mL), and organic layers were combined and washed with saturated brine (150 mL). An organic layer was dried over anhydrous magnesium sulfate, filtered, and the filtrate was concentrated under reduced pressure. The residue was dissolved in n-hexane (400 mL), and the solution was passed through a column packed with silica gel 60 N (neutral, 201 g). After elution with *n*-hexane (800 mL), ethyl acetate (1.60 L) was passed through, and only the ethyl acetate eluate was concentrated under reduced pressure to obtain Compound 5 (65.0 g, 138 mmol, yield 97%) as a pale yellow solid.

MS (ESI): m/z = 471 (M+H)⁺
¹H NMR (400 MHz, CDCl₃) δ 7.36-7.24 (m, 10H), 5.84 (d, J = 4.0 Hz, 1H), 4.81-4.74 (m, 2H), 4.67 (dd, J = 5.2, 4.0 Hz, 1H), 4.54 (d, J = 11.6 Hz, 1H), 4.48-4.38 (m, 2H), 4.26 (d, J = 5.2 Hz, 1H), 4.05 (dd, J = 9.2, 7.7 Hz, 1H), 3.77 (dd, J = 9.2, 6.4 Hz, 1H), 3.68 (d, J = 10.4 Hz, 1H), 3.58 (d, J = 10.4 Hz, 1H), 1.61 (s, 3H), 1.40 (s, 3H), 1.38 (s, 3H), 1.30 (s, 3H).

(3)-2 (Non-Isolation Method): Under a nitrogen atmosphere, the crude product of Compound 4 (8.56 g) obtained in (2)-2 was dissolved in tetrahydrofuran (26 mL), and sodium hydroxide (3.0 g, 75 mmol) was added. The mixture was stirred at 20°C for 30 minutes, and then cooled to 0°C. Benzyl bromide (11.2 g, 65 mmol) was added, and the mixture was further stirred at 50°C for 2 hours and 30 minutes. The reaction mixture was cooled to 20°C, tap water (17 mL) and ethyl acetate (17 mL) were added, and an organic layer was separated. An aqueous layer was extracted with ethyl acetate (17 mL), and then, organic layers were combined and washed with a saturated aqueous sodium bicarbonate solution (26 mL). An organic layer was concentrated under reduced pressure and dried under reduced pressure to obtain a crude product of Compound 5 (14.3 g) as a yellow viscous material.

### (4) Synthesis of Compound 6

(4)-1: Under a nitrogen atmosphere, Compound 5 (10.0 g, 21.2 mmol) was dissolved in acetonitrile (64 mL), and iodine (1.62 g, 6.38 mmol), sodium periodate (9.10 g, 42.5 mmol), and tap water (21 mL) were added at room temperature. The mixture was stirred at 70°C for 4.5 hours, and then, additional sodium periodate (1.00 g, 4.67 mmol) was added, and the mixture was further stirred for 1.5 hours. The reaction mixture was cooled to room temperature, ethyl acetate (175 mL) was added, insoluble matter was removed by filtration, and the filtered residue was washed with ethyl acetate (175 mL). The filtrates were combined. A 30% (w/w) aqueous sodium thiosulfate solution was added to the filtrate, and an organic layer was separated. An aqueous layer was extracted with ethyl acetate (2 × 100 mL), and organic layers were combined and washed with saturated brine (60 mL). An organic layer was dried over anhydrous magnesium sulfate, filtered, and the filtrate was concentrated under reduced pressure. The residue was dissolved in *n*-hexane-ethyl acetate (4:1, v/v) and loaded onto a column packed with silica gel 60 N (neutral, 26 g). Elution was performed with *n*-hexane-ethyl acetate (4:1, v/v) (200 mL), and the eluate was concentrated under reduced pressure to obtain Compound 6 (7.00 g, 17.5 mmol, yield 82%) as a pale yellow viscous material.

MS (ESI): m/z = 421 (M+Na)⁺
¹H NMR (400 MHz, CDCl₃) δ 9.91 (s, 1H), 7.35-7.22 (m, 10H), 5.84 (d, J = 3.2 Hz, 1H), 4.71 (d, J = 12.4 Hz, 1H), 4.61-4.57 (m, 2H), 4.51-4.47 (m, 2H), 4.37 (d, J = 4.4 Hz, 1H), 3.68 (d, J = 10.8 Hz, 1H), 3.61 (d, J = 10.8 Hz, 1H), 1.60 (s, 3H), 1.35 (s, 3H).

(4)-2 (Non-Isolation Method): Under a nitrogen atmosphere, the crude product of Compound 5 (14.3 g) obtained in (3)-2 was dissolved in acetonitrile (42 mL), and iodine (2.29 g, 9.02 mmol), sodium periodate (12.7 g, 59.4 mmol), and tap water (29 mL) were added at room temperature. The mixture was stirred at 70°C for 5 hours, then acetonitrile (42 mL), iodine (4.58 g, 18.0 mmol), and sodium periodate (8.85 g, 41.4 mmol) were added, and the mixture was further stirred for 16 hours and 30 minutes. The reaction mixture was cooled to 20°C, ethyl acetate (28 mL) was added, insoluble matter was removed by filtration, and the solid on the filter paper was washed with ethyl acetate (2 × 28 mL). A 30% (w/w) aqueous sodium thiosulfate solution (36 g) was added to the filtrate, and an organic layer was separated. An aqueous layer was extracted with ethyl acetate (2 × 28 mL), and organic layers were combined and concentrated under reduced pressure and dried under reduced pressure to obtain a crude product of Compound 6 (11.3 g) as a brown liquid.

### (5) Synthesis of Compound 7

(5)-1: Under a nitrogen atmosphere, Compound 6 (5.50 g, 13.8 mmol) was dissolved in tetrahydrofuran (55 mL), and pyridine (5.5 mL) and hydroxylamine hydrochloride (1.15 g, 16.5 mmol) were added. The mixture was stirred at 40°C for 1.5 hours, and then the mixture was further stirred at 60°C for 1.5 hours. The reaction mixture was concentrated under reduced pressure, and tap water (40 mL) and ethyl acetate (40 mL) were added to the concentrated residue, followed by liquid-liquid separation. An aqueous layer was extracted with ethyl acetate (40 mL × 3), and combined organic layers were washed with saturated brine (20 mL). An organic layer was dried over anhydrous magnesium sulfate, filtered, and the filtrate was concentrated under reduced pressure. The residue was purified by normal-phase chromatography (Biotage Isolera, stationary phase; SNAP Ultra 50 g, mobile phase; hexane/ethyl acetate = 90/10 to 60/40), and the eluate containing the target product was concentrated under reduced pressure. Toluene (20 mL) was added to the concentrated residue, followed by concentration under reduced pressure, and this operation was repeated twice to obtain Compound 7 (4.79 g, 11.6 mmol, yield 84%) as a pale yellow viscous material.

MS (ESI): m/z = 414 (M+H)⁺
(5)-2 (Non-Isolation Method): Using the crude product of Compound 6 (11.3 g) obtained in (4)-2, the same reaction and workup procedures as in (5)-1 were performed to obtain a crude product of Compound 7 (10.5 g) as a yellow viscous material. The obtained crude product was used as is in the next step (6)-2.

### (6) Synthesis of Compound 8

(6)-1: Under a nitrogen atmosphere, the crude product of Compound 7 (7.67 g) was dissolved in tetrahydrofuran (34 mL). The solution was warmed to 40°C, and then, a 60% (w/w) sodium bis(2-methoxyethoxy)aluminum hydride toluene solution (14.4 g, 42.6 mmol) was added dropwise over 32 minutes, followed by stirring at 40°C for 80 minutes. The reaction mixture was cooled to room temperature, and then, a 2 M aqueous sodium hydroxide solution (28 mL) was added dropwise over 10 minutes, followed by stirring at room temperature for 15 minutes. Ethyl acetate (34 mL) was added, an organic layer was separated, and the organic layer was washed with a 10% (w/w) aqueous sodium chloride solution (2 × 17 mL), and then concentrated under reduced pressure. Toluene (50 mL) was added to the residue, and the mixture was concentrated under reduced pressure, and this operation was repeated twice. The residue was dissolved in toluene (20 mL), and a 10% (w/w) aqueous citric acid solution (68 mL) and tap water (21 mL) were added, followed by separation of an aqueous layer containing Compound 8. The obtained aqueous layer was used as is in the next step (7)-1.

(6)-2 (Non-Isolation Method): Using the crude product of Compound 7 (10.5 g) obtained in (5)-2, the same reaction and workup procedures as above were performed to obtain an aqueous layer containing Compound 8. The obtained aqueous layer was used as is in the next step (7)-2.

MS (ESI): m/z = 400 (M+H)⁺

### (7) Synthesis of Compound 9

(7)-1: Under a nitrogen atmosphere, a 5 M aqueous sodium hydroxide solution (20 mL) was added at 25°C to the above-described aqueous layer containing Compound 8 obtained in (6)-1. Ethyl acetate (15 mL) was added at room temperature, and a solution of *N-*carbobenzoxyoxysuccinimide (4.28 g, 17.0 mmol) in ethyl acetate (25 mL) was added dropwise over 6 minutes, followed by stirring at room temperature for 40 minutes. An organic layer was separated, *N*-methylpiperazine (0.94 mL, 8.5 mmol) was added, and the mixture was stirred at room temperature for 30 minutes. The reaction mixture was sequentially washed with a 10% (w/w) aqueous sodium chloride solution (14 mL), a 10% (w/w) aqueous citric acid solution (14 mL), a 10% (w/w) aqueous sodium chloride solution (14 mL), a 5% (w/w) aqueous sodium bicarbonate solution (14 mL), and a 10% (w/w) aqueous sodium chloride solution (14 mL). An organic layer was dried over anhydrous magnesium sulfate, and filtered, and then, the filtrate was concentrated under reduced pressure to obtain a crude product of Compound 9 (9.93 g) as a pale yellow viscous material. The obtained crude product was used as is in the next step (same step as (8)-2).

¹H NMR (400 MHz, CDCl₃) δ 7.38-7.21 (m, 15H), 5.75 (d, J = 4.0 Hz, 1H), 5.30-5.29 (m, 1H), 5.08 (s, 2H), 4.75 (d, J = 12 Hz, 1H), 4.62 (dd, J = 4.8 Hz, 4.4 Hz, 1H), 4.52-4.47 (m, 2H), 4.37 (d, J = 12 Hz, 1H), 4.22 (d, J = 5.2 Hz, 1H), 3.71 (dd, J = 14 Hz, 4 Hz, 1H), 3.60 (dd, J = 14.4 Hz, 8.4 Hz, 1H), 3.49 (d, J = 10.8 Hz, 1H), 3.40 (d, J = 10.8 Hz, 1H), 1.57 (s, 3H), 1.32 (s, 3H).

(7)-2 (Non-Isolation Method): Using the above-described aqueous layer containing Compound 8 obtained in (6)-2, the same reaction and workup procedures as in (7)-1 were performed to obtain a crude product of Compound 9 (7.26 g). The obtained crude product was used as is in the next step (8)-2.

### (8) Synthesis of Compound 10

(8)-1: Under a nitrogen atmosphere, Compound 9 (2.56 g, 4.79 mmol) was dissolved in acetic acid (5.12 mL), and acetic anhydride (2.56 mL) and sulfuric acid (13 µL) were added at room temperature, followed by stirring at the same temperature for 1 hour. Under ice-cooling, a saturated aqueous sodium bicarbonate solution (75 mL) was added dropwise to the reaction mixture, followed by addition of ethyl acetate (75 mL) and liquid-liquid separation. An aqueous layer was extracted with ethyl acetate (75 mL × 3), and combined organic layers were sequentially washed with a saturated aqueous sodium bicarbonate solution (50 mL) and a saturated aqueous sodium bicarbonate solution (50 mL). An organic layer was dried over anhydrous magnesium sulfate, filtered, and the filtrate was concentrated under reduced pressure to obtain a crude product of Compound 10 (2.82 g). The obtained crude product was used as is in the next step (9)-1.

MS (ESI): m/z = 579 (M+H)⁺
(8)-2 (Non-Isolation Method): Using the crude product of Compound 9 (7.26 g) obtained in (7)-2, the same reaction and workup procedures as in (8)-1 were performed to obtain a crude product of Compound 10 (7.69 g) as a brown viscous material. The obtained crude product was used as is in the next step (9)-2.

### (9) Synthesis of Compound 11

(9)-1: Under a nitrogen atmosphere, the crude product of Compound 10 (3.15 g) obtained in (8)-1 was dissolved in acetonitrile (13.5 mL), and thymine (875 mg, 6.94 mmol) and *N,O-*bis(trimethylsilyl)acetamide (8.63 mL, 34.6 mmol) were added. The mixture was refluxed and stirred at 90°C for 1 hour. The reaction mixture was cooled to 0°C, and TMSOTf (1.15 mL, 6.36 mmol) was added dropwise over 2 minutes, followed by refluxing and stirring at 90°C for 1 hour. The reaction mixture was cooled to 0°C, and a saturated aqueous sodium bicarbonate solution (20 mL) was added dropwise, and then, ethyl acetate (40 mL) was added, insoluble matter was filtered off, and the filtered residue was washed with ethyl acetate (40 mL × 3). Combined filtrates were concentrated under reduced pressure, the concentrate was extracted with ethyl acetate (40 mL × 2), and combined organic layers were washed with saturated brine (20 mL). An organic layer was dried over anhydrous magnesium sulfate, and then, a drying agent was filtered off, and the filtrate was concentrated under reduced pressure to obtain a crude product of Compound 11 (3.78 g) as a pale yellow solid. The obtained crude product was used as is in the next step (10)-1.

MS (ESI): m/z = 645 (M+H)⁺
(9)-2 (Non-Isolation Method): Using the crude product of Compound 10 (7.69 g) obtained in (8)-2, the same reaction and workup procedures as in (9)-1 were performed to obtain a crude product of Compound 11 (8.17 g). The obtained crude product was used as is in the next step (10)-2.

### (10) Synthesis of Compound 12

(10)-1: Under a nitrogen atmosphere, the crude product of Compound 11 (3.78 g) obtained in (9)-1 was dissolved in tetrahydrofuran (11.6 mL), and then, a 40% aqueous methylamine solution (7.56 mL) was added at room temperature, followed by stirring at the same temperature for 1 hour. The reaction mixture was concentrated under reduced pressure, and tap water (20 mL) and a saturated aqueous ammonium chloride solution (20 mL) were added to the concentrate, followed by extraction with ethyl acetate (50 mL × 3). Combined organic layers were washed with saturated brine (25 mL). An organic layer was dried over anhydrous magnesium sulfate, and then, a drying agent was filtered off, and the filtrate was concentrated under reduced pressure to obtain a crude product (3.14 g). The crude product was purified by normal-phase chromatography (stationary phase: Kanto Chemical Silica Gel 60N, 41 g; mobile phase: hexane/ethyl acetate = 50/50), and the eluate containing the target product was concentrated under reduced pressure to obtain Compound 12 (2.28 g, 3.78 mmol, yield 70% over three steps ((8)-1, (9)-1, and (10)-1) from Compound 9) as a colorless solid.

MS (ESI): m/z = 603 (M+H)⁺
(10)-2 (Non-Isolation Method): Using the crude product of Compound 11 (8.17 g) obtained in (9)-2, the same reaction and workup procedures as in (10)-1 were performed to obtain a crude product of Compound 12 (6.79 g) as a brown viscous material. The obtained crude product was used as is in the next step (11)-2.

### (11) Synthesis of Compound 14

(11)-1: Under a nitrogen atmosphere, the crude product of Compound 12 (16.9 g) was dissolved in pyridine (30 mL), and methanesulfonyl chloride (2.33 g, 20.5 mmol) was added dropwise over 2 minutes at 0°C. After stirring at room temperature for 2 hours and 10 minutes, 1,8-diazabicyclo[5.4.0]undec-7-ene (7.82 g, 51.2 mmol) was added dropwise over 8 minutes. After stirring at room temperature for 3 hours, ethyl acetate (100 mL) and tap water (30 mL) were added. An organic layer was separated, and then, washed with tap water (2 × 30 mL), and concentrated under reduced pressure. The residue was suspended in ethyl acetate (30 mL), and then, toluene (60 mL) was added, and after stirring at room temperature, the solid was collected by filtration. The solid was washed with ethyl acetate-toluene (1:2, v/v) (2 × 10 mL) and dried under reduced pressure to obtain Compound 14 (6.22 g, 10.7 mmol, yield 62% over seven steps ((5)-1, (6)-1, (7)-1, (8)-1, (9)-1, (10)-1, and (11)-1) from Compound 6) as a pale yellow solid.

¹H NMR (400 MHz, CDCl₃) δ 7.39-7.26 (m, 13H), 7.17-7.10 (m, 3H), 5.98 (d, J = 6.4 Hz, 1H), 5.19 (dd, J = 6.4, 1.6 Hz, 1H), 5.12-5.04 (m, 3H), 4.74 (d, J = 12 Hz, 1H), 4.55 (d, J = 12 Hz, 1H), 4.36-4.28 (m, 3H), 3.57-3.51 (m, 1H), 3.40-3.34 (m, 1H), 3.23 (s, 2H), 1.95 (s, 3H).

(11)-2 (Non-Isolation Method): Under a nitrogen atmosphere, the crude product of Compound 12 (6.79 g) obtained in (10)-2 was dissolved in tetrahydrofuran (34 mL). Triethylamine (3.33 g, 32.9 mmol) and methanesulfonyl chloride (1.86 g, 16.2 mmol) were added at 10°C, and the mixture was stirred at 25°C for 2 hours and 30 minutes. At 25°C, 1,8-diazabicyclo[5.4.0]undec-7-ene (4.92 g, 32.3 mmol) was added to the reaction mixture, and after stirring at the same temperature for 3 hours and 30 minutes, tap water (65 mL) was added dropwise over 5 minutes. The mixture was cooled to 10°C, and then, stirred for 1 hour, and the solid was collected by filtration and dried under reduced pressure. The dried solid was suspended in ethyl acetate (7 mL), and then, toluene (14 mL) was added, and after stirring at 20°C, the solid was collected by filtration. The solid was washed with ethyl acetate-toluene (1:2, v/v) (9 mL) and dried under reduced pressure to obtain Compound 14 (2.80 g, 4.8 mmol, yield 8% over eleven steps ((1)-2, (2)-2, (3)-2, (4)-2, (5)-2, (6)-2, (7)-2, (8)-2, (9)-2, (10)-2, and (11)-2) from Compound 1) as a white solid.

(11)-3 (Method including isolation of Compound 13): Under a nitrogen atmosphere, Compound 12 (113 mg, 0.187 mmol) was dissolved in tetrahydrofuran (2.26 mL), and pyridine (60 µL, 0.748 mmol) was added, and then, methanesulfonyl chloride (17.5 µL, 0.224 mmol) and 4-dimethylaminopyridine (30 mg, 0.245 mmol) were added at room temperature, and the mixture was stirred at room temperature for 17 hours. The reaction mixture was concentrated under reduced pressure, and ethyl acetate (5 mL) and tap water (5 mL) were added to the concentrated residue, followed by liquid-liquid separation. An aqueous layer was extracted with ethyl acetate (5 mL × 3), and combined organic layers were washed with saturated brine. An organic layer was dried over anhydrous magnesium sulfate, a drying agent was filtered off, and the filtrate was concentrated under reduced pressure to obtain a crude product (0.14 g) as a pale yellow solid. The crude product was purified by normal-phase chromatography (Biotage Isolera, stationary phase: sfär HD 10 g, mobile phase: hexane/ethyl acetate = 90/10 to 40/60), and the eluate containing the target product was concentrated under reduced pressure. Toluene (5 mL) was added to the concentrated residue, followed by concentration under reduced pressure, and this operation was repeated twice to obtain a crude product of Compound 13 (0.14 g) as a colorless solid.

MS (ESI): m/z = 681 (M+H)⁺
Under a nitrogen atmosphere, the crude product of Compound 13 (0.14 g) was dissolved in DMF (2.54 mL), and then, 60% (w/w) sodium hydride (dispersion in paraffin liquid) (7.4 mg, 0.185 mmol) was added at 0°C, followed by stirring at the same temperature for 16 hours. At 0°C, tap water (5 mL) and ethyl acetate (5 mL) were added to the reaction mixture, followed by liquid-liquid separation. An aqueous layer was extracted with ethyl acetate (5 mL × 2), and combined organic layers were washed with saturated brine (3 mL). An organic layer was dried over anhydrous magnesium sulfate, a drying agent was filtered off, and the filtrate was concentrated under reduced pressure. The concentrated residue was purified by normal-phase chromatography (Biotage Isolera, stationary phase: sfär HD 10 g, mobile phase: hexane/ethyl acetate/methanol = 90/10/0 to 0/100/0 to 0/90/10). The eluates containing Compound 14 and Compound 15 were separately concentrated under reduced pressure to obtain Compound 14 (0.10 g, 0.171 mmol) and Compound 15 (0.01 g, 0.017 mmol), each as a colorless solid.

### <Powder X-ray diffraction measurements of crystals of compound 14>

Powder X-ray diffraction (XRD) measurements were performed using a powder X-ray diffractometer: MiniFlex600 (Rigaku Corporation) with CuKα radiation under the following conditions:
X-ray generator: X-ray tube (copper, tube voltage: 40 kV, tube current: 15 mA)
Detector: D/teX Ultra 2 (standard mode)
Measurement range: 3.0 to 40.0°
Scan speed: 10.0°/min
Sampling width: 0.020°

The results of the powder X-ray diffraction pattern (XRD) for Compound 14 are shown in Fig. 1 and Table 1. It was confirmed that Compound 14 was isolated as a crystal.

**[Table 1]**

| Table 1: XRD peaks of Compound 14 | | |
|---|---|---|
| 2*θ* | d value | Relative intensity |
| 3.66 | 24.1214 | 6 |
| 7.36 | 12.0014 | 100 |
| 11.02 | 8.0223 | 18 |
| 12.34 | 7.167 | 2 |
| 12.66 | 6.9865 | 22 |
| 13.98 | 6.3297 | 3 |
| 14.8 | 5.9808 | 15 |
| 15.2 | 5.8243 | 5 |
| 16.16 | 5.4804 | 7 |
| 16.4 | 5.4007 | 18 |
| 16.78 | 5.2792 | 8 |
| 17.44 | 5.0809 | 14 |
| 18.12 | 4.8917 | 10 |
| 18.52 | 787 | 4 |
| 19.16 | 4.6285 | 54 |
| 20.12 | 4.4098 | 51 |
| 20.88 | 4.2509 | 13 |
| 21.28 | 4.1719 | 39 |
| 22.2 | 4.0011 | 7 |
| 22.56 | 3.938 | 9 |
| 23.02 | 3.8604 | 3 |
| 23.68 | 3.7543 | 2 |
| 24.06 | 3.6958 | 6 |
| 24.34 | 3.6539 | 3 |
| 24.66 | 3.6072 | 29 |
| 25.24 | 3.5256 | 9 |
| 25.7 | 3.4636 | 5 |
| 26.02 | 3.4217 | 2 |
| 26.46 | 3.3658 | 7 |
| 27.16 | 3.2806 | 8 |
| 27.56 | 3.2339 | 3 |
| 28.1 | 3.173 | 10 |
| 28.36 | 3.1445 | 8 |
| 28.64 | 3.1144 | 5 |
| 29.06 | 3.0703 | 3 |
| 30.2 | 2.9569 | 3 |
| 30.74 | 2.9062 | 6 |
| 31.6 | 2.8291 | 2 |
| 32 | 2.7946 | 6 |
| 32.72 | 2.7347 | 3 |
| 33.2 | 2.6963 | 2 |
| 34 | 2.6346 | 3 |
| 35.02 | 2.5602 | 2 |
| 35.36 | 2.5364 | 5 |
| 36.7 | 2.4468 | 2 |
| 36.86 | 2.4365 | 2 |
| 37.72 | 2.3829 | 2 |
| 38.66 | 2.3271 | 2 |
| 39.06 | 2.3042 | 3 |
| 39.18 | 2.2974 | 3 |
| 39.48 | 2.2807 | 2 |

Characteristic peaks in the powder X-ray diffraction pattern of crystals of Compound 14 were observed at diffraction angles, expressed as 2θ, of 7.4 ± 0.2°, 19.2 ± 0.2°, 20.1 ± 0.2°, 21.3 ± 0.2°, and 24.7 ± 0.2°.

### (12) Synthesis of Compound 15

(12)-1: Under a nitrogen atmosphere, Compound 15 (0.01 g, 0.017 mmol) was added to Compound 14 (0.10 g, 0.17 mmol), and the mixture was dissolved in N,N-dimethylformamide (2.5 mL) under a nitrogen atmosphere. At 0°C, 60% (w/w) sodium hydride (dispersion in paraffin liquid) (7.4 mg, 0.19 mmol) was added, and the mixture was stirred at the same temperature for 16 hours. At 0°C, tap water (5 mL) and ethyl acetate (5 mL) were added, and an aqueous layer was separated. An aqueous layer was extracted with ethyl acetate (5 mL × 3), and combined organic layers were washed with saturated brine (3 mL). An organic layer was dried over anhydrous magnesium sulfate, filtered, and the filtrate was concentrated under reduced pressure. The concentrated residue was purified by normal-phase chromatography (Biotage Isolera, stationary phase: sfär HD 10 g, mobile phase: hexane/ethyl acetate/methanol = 90/10/0 to 0/100/0 to 0/80/20) to obtain a crude product of Compound 15 (0.10 g) as a colorless solid. The obtained crude product was used as is in the next step (13).

MS (ESI): m/z = 585 (M+H)⁺
(12)-2 (Method without adding Compound 15 as a starting material): Under a nitrogen atmosphere, Compound 14 (2.46 g, 4.28 mmol) was dissolved in N,Ndimethylformamide (13 mL). After cooling to 0°C, 60% (w/w) sodium hydride (dispersion in paraffin liquid) (254 mg, 6.4 mmol) was added, and the mixture was stirred at the same temperature for 3 hours and 50 minutes. Additional 60% (w/w) sodium hydride (dispersion in paraffin liquid) (38 mg, 0.95 mmol) was added, and the mixture was stirred at 0°C for 1 hour and 40 minutes. Further, 60% (w/w) sodium hydride (dispersion in paraffin liquid) (55 mg, 1.4 mmol) was added, and the mixture was stirred at 0°C for 17 hours and 50 minutes. After warming to 10°C, tap water (13 mL) and ethyl acetate (12 mL) were added. After warming to 20°C, the mixture was stirred for 10 minutes. An aqueous layer was separated, and then, extracted with ethyl acetate (3 × 12 mL). Organic layers were combined and concentrated under reduced pressure to obtain a crude product of Compound 15 (2.21 g) as a yellow viscous material.

### (13) Synthesis of Compound 16

(13): Under a nitrogen atmosphere, the crude product of Compound 15 (0.10 g) obtained in (12)-1 was dissolved in methanol (2.0 mL). At room temperature, Pd(OH)₂/C (20% (w/w) Pd, 50% (w/w) wet) (12 mg) was added, and the mixture was stirred under a hydrogen atmosphere for 7 hours. Acetic acid (1.0 mL) was added to the reaction mixture, and stirring was continued under a hydrogen atmosphere for an additional 16 hours, followed by removal of insoluble matter by filtration. The solid on the filter paper was washed with acetic acid (3 × 1 mL), and combined filtrates were concentrated under reduced pressure. The residue was dissolved in toluene (5 mL) and concentrated under reduced pressure; this operation was repeated twice to obtain a crude product of Compound 16 (0.060 g) as a pale yellow viscous material. The obtained crude product was used as is in the next step (14).

MS (ESI): m/z = 270 (M+H)⁺

### (14) Synthesis of Compound 17

(14): Under a nitrogen atmosphere, the crude product of Compound 16 (0.060 g) obtained in (13) was dissolved in pyridine (0.5 mL), and 4,4'-dimethoxytrityl chloride (58 mg, 0.17 mmol) was added at room temperature, followed by stirring at the same temperature for 21 hours. Additional 4,4'-dimethoxytrityl chloride (12 mg, 0.035 mmol) was added, and the mixture was stirred at room temperature for 20 hours. Further, 4,4'-dimethoxytrityl chloride (23 mg, 0.067 mmol) was added, and the mixture was stirred at room temperature for 24 hours. Methanol was added to the reaction mixture, and then, the solvent was removed under reduced pressure. Dichloromethane (10 mL) and a saturated aqueous sodium bicarbonate solution (5 mL) were added to the residue, and an aqueous layer was separated. An aqueous layer was extracted with dichloromethane (2 × 10 mL), and combined organic layers were washed with saturated brine (5 mL). An organic layer was dried over anhydrous magnesium sulfate, filtered, and the filtrate was concentrated under reduced pressure. The obtained crude product of Compound 17 was purified by column chromatography (neutral silica gel, dichloromethane-methanol (9:1, v/v)) to obtain Compound 17 (42.4 mg, 0.074 mmol, yield 40% over three steps ((12)-1, (13), and (14)) from Compound 14 and Compound 15) as a white solid.

¹H NMR (400 MHz, DMSO-d₆) δ 11.50-11.20 (br, 1H), 7.62 (d, J = 1.2 Hz, 1H), 7.46-7.43 (m, 2H), 7.35-7.22 (m, 7H), 6.93-6.89 (m, 4H), 5.50-5.35 (br, 1H), 5.35 (s, 1H), 4.06 (s, 1H), 3.74 (s, 6H), 3.36-3.26 (m, 3H), 2.84 (d, J = 10 Hz, 1H), 2.63 (d, J = 9.6 Hz, 1H), 1.50 (s, 3H).

MS (ESI): m/z = 573 (M+H)⁺

### Example 2: Method for producing Compound 9 (Alternative Method 1)

Compound 9 was produced from Compound 6 via Compound 18 and Compound 19 using the following method:

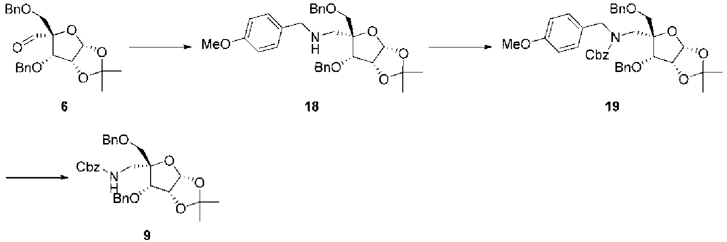

### (1) Synthesis of Compound 19

Under a nitrogen atmosphere, Compound 6 (479 mg, 1.20 mmol) was dissolved in dichloromethane (9.6 mL), and 4-methoxybenzylamine (188 µL, 1.45 mmol) was added, followed by stirring at room temperature for 30 minutes. Sodium triacetoxyborohydride (356 mg, 1.67 mmol) was added to the reaction mixture, and the mixture was stirred at room temperature for 14 hours. A saturated aqueous sodium bicarbonate solution (4.8 mL) and benzyl chloroformate (250 µL, 1.77 mmol) were added to the reaction mixture, and the mixture was further stirred at room temperature for 3 hours. A saturated aqueous sodium bicarbonate solution (4.8 mL) was added to the reaction mixture, and an organic layer was separated. An aqueous layer was extracted with dichloromethane (3 × 10 mL), and combined organic layers were washed with saturated brine (4 mL). An organic layer was dried over anhydrous magnesium sulfate, filtered, and then, the filtrate was concentrated under reduced pressure to obtain a crude product of Compound 19 (931 mg) as a pale yellow viscous material. The obtained crude product was used as is in the next step (2).
Compound 18 MS (ESI): m/z = 521 (M+H)⁺
Compound 19 MS (ESI): m/z = 654 (M+H)⁺

### (2) Synthesis of Compound 9

Under a nitrogen atmosphere, the crude product of Compound 19 (931 mg) obtained in step (1) was dissolved in acetonitrile (14 mL), and tap water (2.8 mL) was added. (NH₄)₂Ce(NO₃)₆ (2.37 g, 4.32 mmol) was added, and the mixture was stirred at room temperature for 30 minutes.

Under ice-cooling, a saturated aqueous sodium bicarbonate solution (40 mL) and ethyl acetate (40 mL) were added to the reaction mixture, and insoluble matter was removed by filtration. The solid on the filter paper was sequentially washed with ethyl acetate (2 × 20 mL), tap water (20 mL), and a saturated aqueous sodium bicarbonate solution (2 × 20 mL), and an organic layer of the filtrate was separated. An aqueous layer was extracted with ethyl acetate (3 × 80 mL), and combined organic layers were washed with saturated brine (60 mL). An organic layer was dried over anhydrous magnesium sulfate, filtered, and the filtrate was concentrated under reduced pressure. The obtained crude product of Compound 9 was purified by column chromatography (neutral silica gel, hexane-ethyl acetate (9:1 to 3:1, v/v)) to obtain Compound 9 (379 mg, 0.710 mmol, yield 59% over two steps ((1) and (2)) from Compound 6) as a pale yellow viscous material.

¹H NMR (400 MHz, CDCl₃) δ 7.39-7.20 (m, 15H), 5.75 (d, J = 4.0 Hz, 1H), 5.32-5.27 (m, 1H), 5.08 (s, 2H), 4.77-4.72 (m, 1H), 4.63-4.59 (m, 1H), 4.52-4.47 (m, 2H), 4.40-4.35 (m, 1H), 4.25-4.19 (m, 1H), 3.75-3.69 (m, 1H), 3.63-3.57 (m, 1H), 3.52-3.48 (m, 1H), 3.43-3.38 (m, 1H), 1.57 (s, 3H), 1.32 (s, 3H).

MS (ESI): m/z = 534 (M+H)⁺

Example 3: Method for producing Compound 9 (Alternative Method 2)

Compound 9 was produced from Compound 6 via Compound 20 and Compound 21 using the following method:

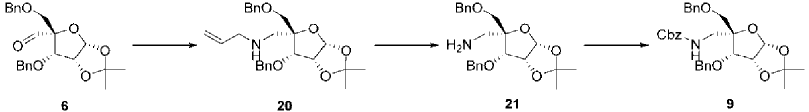

### (1) Synthesis of Compound 20

Under a nitrogen atmosphere, Compound 6 (1.49 g, 3.74 mmol) was dissolved in dichloromethane (15 mL). At room temperature, allylamine (256 mg, 4.49 mmol) and sodium triacetoxyborohydride (1.11 g, 5.24 mmol) were added, and the mixture was stirred at the same temperature overnight. The reaction was quenched by adding a 2 M aqueous sodium hydroxide solution, and an organic layer was separated. An organic layer was washed twice with saturated brine, and combined aqueous layers were extracted once with ethyl acetate. Combined organic layers were dried over anhydrous sodium sulfate, and filtered, and then, the filtrate was concentrated under reduced pressure to obtain a crude product of Compound 20 (1.61 g). The obtained crude product was used as is in the next step (2).

¹H NMR (400 MHz, CDCl₃) δ 7.35-7.23 (m, 10H), 5.92-5.82 (m, 1H), 5.77 (d, J = 4.0 Hz, 1H), 5.18-5.04 (m, 2H), 4.75 (d, J = 12 Hz, 1H), 4.61 (dd, J = 5.2 Hz, 4 Hz, 1H), 4.53 (d, J = 12 Hz, 2H), 4.41 (d, J = 12 Hz, 1H), 4.24 (d, J = 5.2 Hz, 1H), 3.77 (d, J = 10.4 Hz, 1H), 3.52 (d, J = 10.8 Hz, 1H), 3.28-3.16 (m, 3H), 2.77 (d, J = 12 Hz, 1H), 2.05 (s, 1H),1.61 (s, 3H), 1.33 (s, 3H).

### (2) Synthesis of Compound 21

Under a nitrogen atmosphere, the crude product of Compound 20 (1.25 g) was dissolved in dichloromethane (13 mL). At 40°C, N,N-dimethylbarbituric acid (680 mg, 4.36 mmol) and tetrakis(triphenylphosphine)palladium(0) (168 mg, 0.145 mmol) were added, and the mixture was stirred at the same temperature for 75 minutes. Isopropyl acetate was added, and the mixture was sequentially washed with an aqueous sodium hydroxide solution and an aqueous sodium chloride solution. An organic layer was concentrated under reduced pressure, and then, the residue was dissolved in toluene and extracted with an aqueous citric acid solution (13 mL). An aqueous layer containing a crude product of Compound 21 was used as is in the next step (3).

### (3) Synthesis of Compound 9

Under a nitrogen atmosphere, to the entire aqueous layer obtained in step (2), a 5 M aqueous sodium hydroxide solution (4 mL) and ethyl acetate (3 mL) were added. Next, N-(benzyloxycarbonyloxy)succinimide (724 mg, 2.90 mmol) dissolved in ethyl acetate (5 mL) was added dropwise at room temperature, and the reaction mixture was stirred. N-Methylpiperazine (100.17 g, 2.90 mmol) was added to quench the reaction, and then, an organic layer was separated, and sequentially washed with 10% (w/w) aqueous citric acid, a 10% (w/w) aqueous sodium chloride solution, a 5% (w/w) aqueous sodium bicarbonate solution, and a 10% (w/w) aqueous sodium chloride solution. An organic layer was dried over anhydrous sodium sulfate, and filtered, and then, the filtrate was concentrated under reduced pressure to obtain a crude product of Compound 9 (1.01 g, HPLC purity 73.0% (220 nm) over three steps ((1), (2), and (3)) from Compound 6).

¹H NMR (400 MHz, CDCl₃) δ 7.40-7.21 (m, 15H), 5.75 (d, J = 4.0 Hz, 1H), 5.32-5.27 (m,, 1H), 5.08 (s, 2H), 4.75 (d, J = 12 Hz, 1H), 4.61 (dd, J = 4.4 Hz, 4.4 Hz, 1H), 4.52-4.47 (m, 2H), 4.37 (d, J = 12 Hz, 1H), 4.22 (d, J = 5.2 Hz, 1H), 3.71 (dd, J = 14.4 Hz, 4 Hz, 1H), 3.60 (dd, J = 14.4 Hz, 8 Hz, 1H), 3.49 (d, J = 10.4 Hz, 1H), 3.40 (d, J = 10.8 Hz, 1H), 1.56 (s, 3H), 1.32 (s, 3H).

The disclosure of Japanese Patent Application No. 2022-204199 (filing date: December 21, 2022) is incorporated herein by reference in its entirety.

All documents, patent applications, and technical standards mentioned in the present specification are incorporated herein by reference to the same extent as when each individual document, patent application, and technical standard is specifically and individually indicated to be incorporated by reference.

## Claims

1. A method for producing a compound represented by Formula (IV): 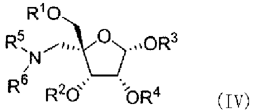 [wherein
R¹ represents a hydroxyl protecting group;
R² represents a hydroxyl protecting group;
R³ and R⁴, which may be the same or different, each independently represent a hydroxyl protecting group, or together form a cyclic protecting group for a diol; and
one of R⁵ and R⁶ represents a hydrogen atom or an amino protecting group, and the other represents an amino protecting group]
from a compound represented by Formula (I): 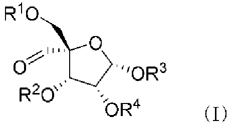 [wherein
R¹ represents a hydroxyl protecting group;
R² represents a hydroxyl protecting group; and
R³ and R⁴, which may be the same or different, each independently represent a hydroxyl protecting group, or together form a cyclic protecting group for a diol],
the production method comprising the following Steps 1 to 3:
Step 1: a step of obtaining a compound represented by Formula (II): 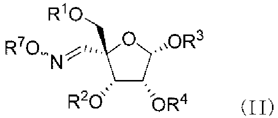 [wherein
R¹ represents a hydroxyl protecting group;
R² represents a hydroxyl protecting group;
R³ and R⁴, which may be the same or different, each independently represent a hydroxyl protecting group, or together form a cyclic protecting group for a diol; and
R⁷ represents a hydrogen atom or an alkyl group],
by reacting the compound represented by Formula (I): 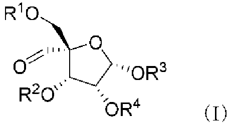
[wherein the symbols have the same meaning as described above] with a compound represented by Formula (V):
R⁷O-NH₂ (V)
[wherein R⁷ represents a hydrogen atom or an alkyl group] or a salt thereof;
Step 2: a step of obtaining a compound represented by Formula (III): 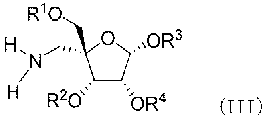 [wherein
R¹ represents a hydroxyl protecting group;
R² represents a hydroxyl protecting group; and
R³ and R⁴, which may be the same or different, each independently represent a hydroxyl protecting group, or together form a cyclic protecting group for a diol],
by subjecting a compound represented by Formula (II) to a reduction reaction with a reducing agent; and
Step 3: a step of subjecting the compound represented by Formula (III) to a reaction for protecting an amino group.

2. The production method according to claim 1, wherein the reducing agent used in Step 2 is sodium bis(2-methoxyethoxy)aluminum hydride (Red-Al).

3. The production method according to claim 1 or 2, wherein the compound represented by Formula (I): 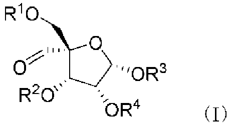 [wherein
R¹ represents a hydroxyl protecting group;
R² represents a hydroxyl protecting group; and
R³ and R⁴, which may be the same or different, each independently represent a hydroxyl protecting group, or together form a cyclic protecting group for a diol] is produced by a process including the following Steps 4 to 8:
Step 4: a step of obtaining a compound represented by Formula (VII): 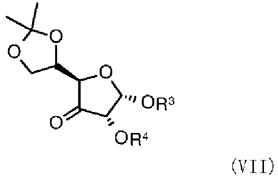 [wherein R³ and R⁴, which may be the same or different, each independently represent a hydroxyl protecting group, or together form a cyclic protecting group for a diol],
by subjecting a compound represented by Formula (VI): 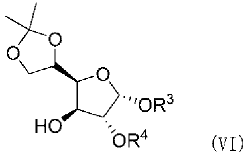 [wherein R³ and R⁴, which may be the same or different, each independently represent a hydroxyl protecting group, or together form a cyclic protecting group for a diol] to an oxidation reaction;
Steps 5 to 7: steps of obtaining a compound represented by Formula (X): 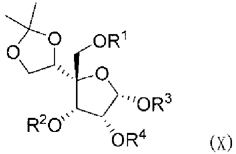 [wherein R³ and R⁴, which may be the same or different, each independently represent a hydroxyl protecting group, or together form a cyclic protecting group for a diol],
by sequentially subjecting the compound represented by Formula (VII) to an aldol reaction (Step 5), a reduction reaction (Step 6), and a hydroxyl protecting reaction (Step 7); and
Step 8: a step of subjecting the compound represented by Formula (X) to an oxidative cleavage reaction.

4. A method for producing a compound represented by Formula (XIII): 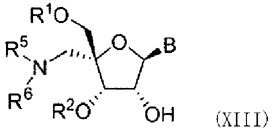 [wherein
R¹ represents a hydroxyl protecting group;
R² represents a hydroxyl protecting group;
one of R⁵ and R⁶ represents a hydrogen atom or an amino protecting group, and the other represents an amino protecting group; and
B represents a nucleic acid base moiety which may be substituted with one or more substituents] from a compound represented by Formula (IV): 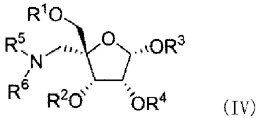 [wherein
R¹ represents a hydroxyl protecting group;
R² represents a hydroxyl protecting group;
R³ and R⁴, which may be the same or different, each independently represent a hydroxyl protecting group, or together form a cyclic protecting group for a diol; and
one of R⁵ and R⁶ represents a hydrogen atom or an amino protecting group, and the other represents an amino protecting group],
the production method comprising the following Steps 9 to 11:
Step 9: a step of obtaining a compound represented by Formula (XI): 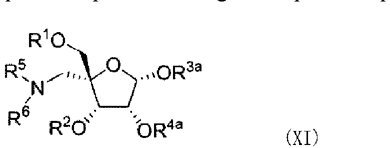 [wherein
R¹ represents a hydroxyl protecting group;
R² represents a hydroxyl protecting group;
R^{3a} represents an acyl group;
R^{4a} represents an acyl group; and
one of R⁵ and R⁶ represents a hydrogen atom or an amino protecting group, and the other represents an amino protecting group],
by subjecting a compound represented by Formula (IV), produced using the production method according to any one of claims 1 to 3, to a conversion reaction of R³ and R⁴ into acyl groups, as necessary;
Step 10: a step of obtaining a compound represented by Formula (XII): 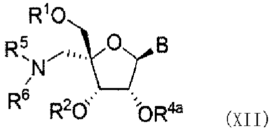 [wherein
R¹ represents a hydroxyl protecting group;
R² represents a hydroxyl protecting group;
R^{4a} represents an acyl group;
one of R⁵ and R⁶ represents a hydrogen atom or an amino protecting group, and the other represents an amino protecting group; and
B represents a nucleic acid base moiety which may be substituted with one or more substituents], by subjecting the compound represented by Formula (XI) to a glycosylation reaction; and
Step 11: a step of subjecting the compound represented by Formula (XII) to a deprotection reaction of an acyl group.

5. The production method according to claim 4, wherein B is represented by Formula (XXIV): 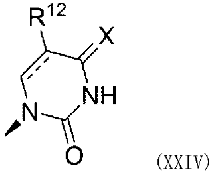 [wherein
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond].

6. A method for producing a compound represented by Formula (XVII): 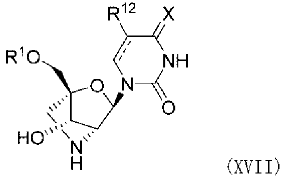 [wherein
R¹ represents a hydrogen atom or a hydroxyl protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond] from a compound represented by Formula (XIII'): 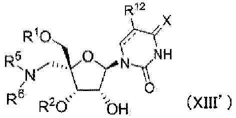 [wherein
R¹ represents a hydroxyl protecting group;
R² represents a hydroxyl protecting group;
one of R⁵ and R⁶ represents a hydrogen atom or an amino protecting group, and the other represents an amino protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond],
the production method comprising the following Steps 12 to 15:
Step 12: a step of obtaining a compound represented by Formula (XIV): 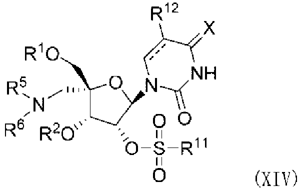 [wherein
R¹ represents a hydroxyl protecting group;
R² represents a hydroxyl protecting group;
one of R⁵ and R⁶ represents a hydrogen atom or an amino protecting group, and the other represents an amino protecting group;
R¹¹ represents an alkyl group or an aryl group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond],
by subjecting a compound represented by Formula (XIII'), produced using the production method according to claim 4 or 5, to a sulfonylation reaction;
Step 13: a step of obtaining a compound represented by Formula (XV): 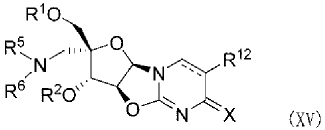 [wherein
R¹ represents a hydroxyl protecting group;
R² represents a hydroxyl protecting group;
one of R⁵ and R⁶ represents a hydrogen atom or an amino protecting group, and the other represents an amino protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond],
by subjecting the compound represented by Formula (XIV) to a cyclization reaction;
Step 14: a step of obtaining a compound represented by Formula (XVI): 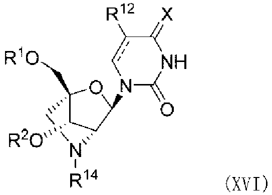 [wherein
R¹ represents a hydroxyl protecting group;
R² represents a hydroxyl protecting group;
R¹² represents a hydrogen atom, an alkyl group, an alkoxy group, or an alkoxyalkyl group;
R¹⁴ represents a hydrogen atom or an amino protecting group;
X represents an oxygen atom or NR¹³ (wherein R¹³ represents a hydrogen atom or an amino protecting group); and
a double line consisting of a solid line and a dotted line represents a single bond or a double bond],
by subjecting the compound represented by Formula (XV) to a ring-closing reaction; and
Step 15: a step of subjecting the compound represented by Formula (XVI) to a deprotection reaction, or to a deprotection reaction followed by a further hydroxyl protecting reaction.

7. A compound represented by Formula (II): 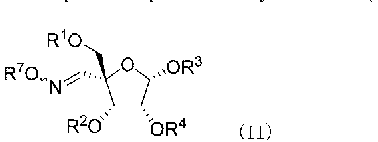 [wherein
R¹ represents a hydroxyl protecting group;
R² represents a hydroxyl protecting group;
R³ and R⁴, which may be the same or different, each independently represent a hydroxyl protecting group, or together form a cyclic protecting group for a diol; and
R⁷ represents a hydrogen atom or an alkyl group].

8. A compound represented by Formula (III): 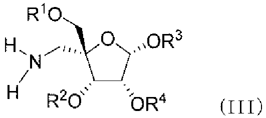 [wherein
R¹ represents a hydroxyl protecting group;
R² represents a hydroxyl protecting group; and
R³ and R⁴, which may be the same or different, each independently represent a hydroxyl protecting group, or together form a cyclic protecting group for a diol].

9. A compound represented by Formula (IV): 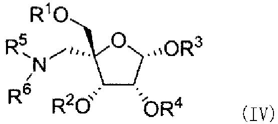 [wherein
R¹ represents a hydroxyl protecting group;
R² represents a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
R³ and R⁴, which may be the same or different, each independently represent a hydroxyl protecting group, or together form a cyclic protecting group for a diol; and
one of R⁵ and R⁶ represents a hydrogen atom or an amino protecting group, and the other represents an amino protecting group].

10. A compound represented by Formula (XI): 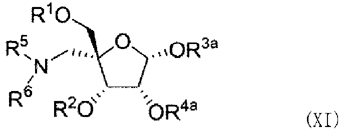 [wherein
R¹ represents a hydroxyl protecting group;
R² represents a hydroxyl protecting group;
R^{3a} and R^{4a} each represent an acyl group; and
one of R⁵ and R⁶ represents a hydrogen atom or an amino protecting group, and the other represents an amino protecting group].

11. The compound according to claim 10, represented by Formula (XI): 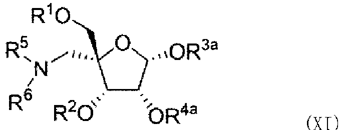 [wherein
R¹ represents a hydroxyl protecting group;
R² represents a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
R^{3a} and R^{4a} each represent an acyl group; and
one of R⁵ and R⁶ represents a hydrogen atom or an amino protecting group, and the other represents an amino protecting group].

12. A compound represented by Formula (XII): 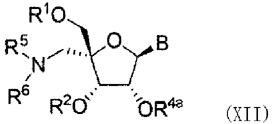 [wherein
R¹ represents a hydroxyl protecting group;
R² represents a hydroxyl protecting group;
R^{4a} represents an acyl group;
one of R⁵ and R⁶ represents a hydrogen atom or an amino protecting group, and the other represents an amino protecting group; and
B represents a nucleic acid base moiety which may be substituted with one or more substituents].

13. The compound according to claim 12, represented by Formula (XII): 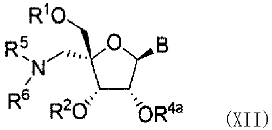 [wherein
R¹ represents a hydroxyl protecting group;
R² represents a benzyl group, a 4,4'-dimethoxytrityl group, a t-butyldimethylsilyl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
R^{4a} represents an acyl group;
one of R⁵ and R⁶ represents a hydrogen atom or an amino protecting group, and the other represents an amino protecting group; and
B represents a nucleic acid base moiety which may be substituted with one or more substituents].

14. The compound according to claim 12 or 13, wherein B is thyminyl.

15. A compound represented by Formula (XIII): 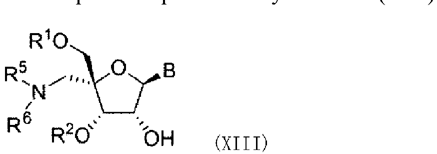 [wherein
R¹ represents a hydroxyl protecting group;
R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
one of R⁵ and R⁶ represents a hydrogen atom or an amino protecting group, and the other represents an amino protecting group; and
B represents a nucleic acid base moiety which may be substituted with one or more substituents].

16. The compound according to claim 15, wherein B is thyminyl.

17. A compound represented by Formula (XIV): 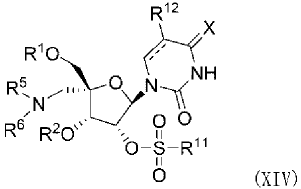 [wherein
R¹ represents a hydroxyl protecting group;
R² represents a hydroxyl protecting group;
one of R⁵ and R⁶ represents a hydrogen atom or an amino protecting group, and the other represents an amino protecting group;
R¹¹ represents an alkyl group or an aryl group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond].

18. The compound according to claim 17, represented by Formula (XIV): 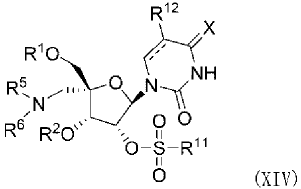 [wherein
R¹ represents a hydroxyl protecting group;
R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
one of R⁵ and R⁶ represents a hydrogen atom or an amino protecting group, and the other represents an amino protecting group;
R¹¹ represents an alkyl group or an aryl group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond].

19. The compound according to claim 17 or 18, wherein R¹² is a methyl group, and X is an oxygen atom.

20. A compound represented by Formula (XV): 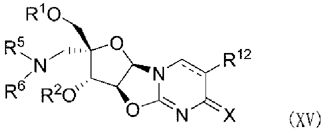 [wherein
R¹ represents a hydroxyl protecting group;
R² represents a hydroxyl protecting group;
one of R⁵ and R⁶ represents a hydrogen atom or an amino protecting group, and the other represents an amino protecting group;
R¹² represents a hydrogen atom, an alkyl group, an alkoxy group or an alkoxyalkyl group;
X represents an oxygen atom or NR¹³ (wherein R¹³ represents a hydrogen atom or an amino protecting group); and
a double line consisting of a solid line and a dotted line represents a single bond or a double bond].

21. The compound according to claim 20, represented by Formula (XV): 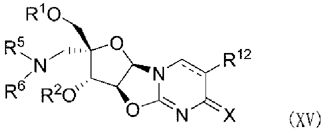 [wherein
R¹ represents a hydroxyl protecting group;
R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
one of R⁵ and R⁶ represents a hydrogen atom or an amino protecting group, and the other represents an amino protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dashed line represents a single bond or a double bond].

22. The compound according to claim 20 or 21, wherein R¹² is a methyl group, and X is an oxygen atom.

23. The compound according to any one of claims 20 to 22, wherein R¹² is a methyl group; X is an oxygen atom; R¹ is a benzyl group; R² is a benzyl group; R⁵ is a benzyloxycarbonyl group; R⁶ is a hydrogen atom; and a double line consisting of a solid line and a dashed line is a single bond or a double bond.

24. A crystal of the compound according to claim 23.

25. The crystal of the compound according to claim 24, having peaks at diffraction angles, expressed in terms of 2θ, of 7.4±0.2°, 19.2±0.2°, 20.1±0.2°, 21.3±0.2°, and 24.7±0.2° in a powder X-ray diffraction spectrum measured using CuKα radiation.

26. A compound represented by Formula (XVI): 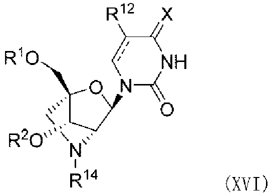 [wherein
R¹ represents a hydroxyl protecting group;
R² represents a hydroxyl protecting group;
R¹² represents a hydrogen atom, an alkyl group, an alkyloxy group, or an alkoxyalkyl group;
R¹⁴ represents a hydrogen atom, a benzyloxycarbonyl group, an isobutyl group, a benzoyl group, a t-butyloxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a 4-methoxybenzyl group, an allyl group, a benzyl group, or a 2-nitrobenzenesulfonyl group;
X represents an oxygen atom or NR¹³ (wherein R¹³ represents a hydrogen atom or an amino protecting group); and
a double line consisting of a solid line and a dashed line represents a single bond or a double bond].

27. The compound according to claim 26, represented by Formula (XVI) 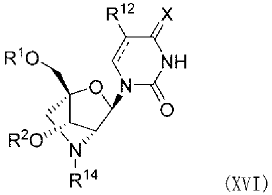 [wherein
R¹ represents a hydroxyl protecting group;
R² represents a benzyl group, a 4,4'-dimethoxytrityl group, a t-butyldiphenylsilyl group, a trimethylsilyl group, a methoxymethyl group, a benzyloxymethyl group, a 2-(trimethylsilyl)ethoxymethyl group, a 2-methoxyethoxymethyl group, a cyanoethyl group, a benzoyl group, or an acetyl group;
R¹² represents a hydrogen atom, an alkyl group, an alkoxy group, or an alkoxyalkyl group;
R¹⁴ represents a hydrogen atom, a benzyloxycarbonyl group, an isobutyl group, a benzoyl group, a t-butyloxycarbonyl group, a trimethylsilylethoxycarbonyl group, an allyloxycarbonyl group, a 9-fluorenylmethoxycarbonyl group, a 2,2,2-trichloroethoxycarbonyl group, an acetyl group, a formyl group, a 4-methoxybenzyl group, an allyl group, a benzyl group, or a 2-nitrobenzenesulfonyl group;
X represents an oxygen atom or NR¹³ (wherein R¹³ represents a hydrogen atom or an amino protecting group); and
a double line consisting of a solid line and a dashed line represents a single bond or a double bond].

28. A method for producing a modified oligonucleotide or a salt thereof represented by the following formula: 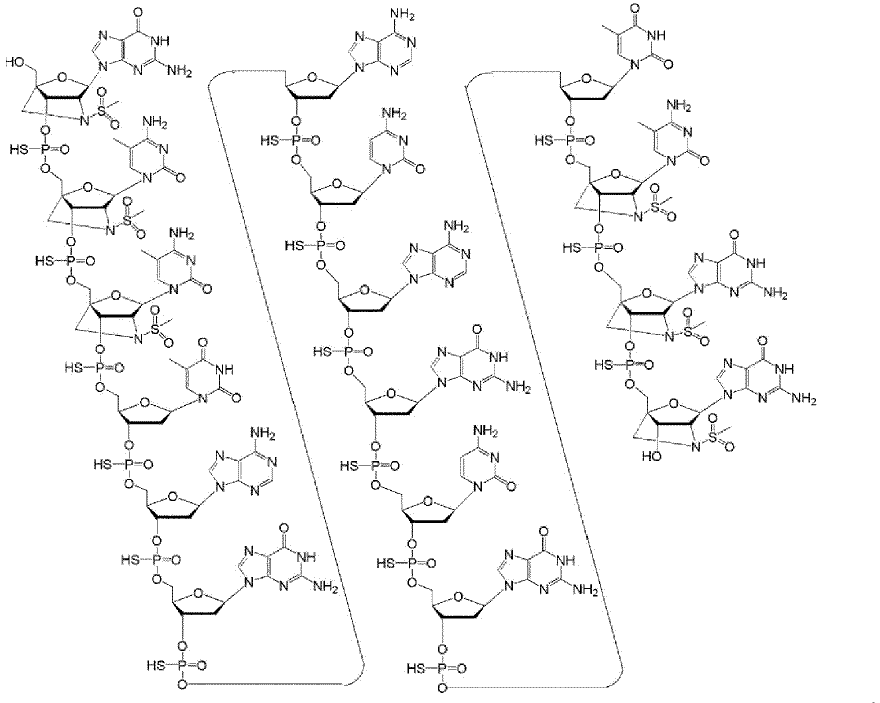 from a compound represented by Formula (XVII"): 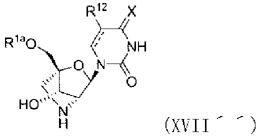 [wherein
R^{1a} represents a hydroxyl protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond],
the production method comprising the following steps:
a step of producing a compound represented by Formula (XVII") using the production method according to claim 6;
a step of producing an ALNA[Ms] amidite from the compound represented by Formula (XVII");
a step of subjecting the ALNA[Ms] amidite and an amidite for DNA synthesis to a phosphoramidite method; and,
as necessary, a step of subjecting a phosphodiester bond to a thiation reaction.

29. A method for producing a modified oligonucleotide or a salt thereof represented by the following formula: 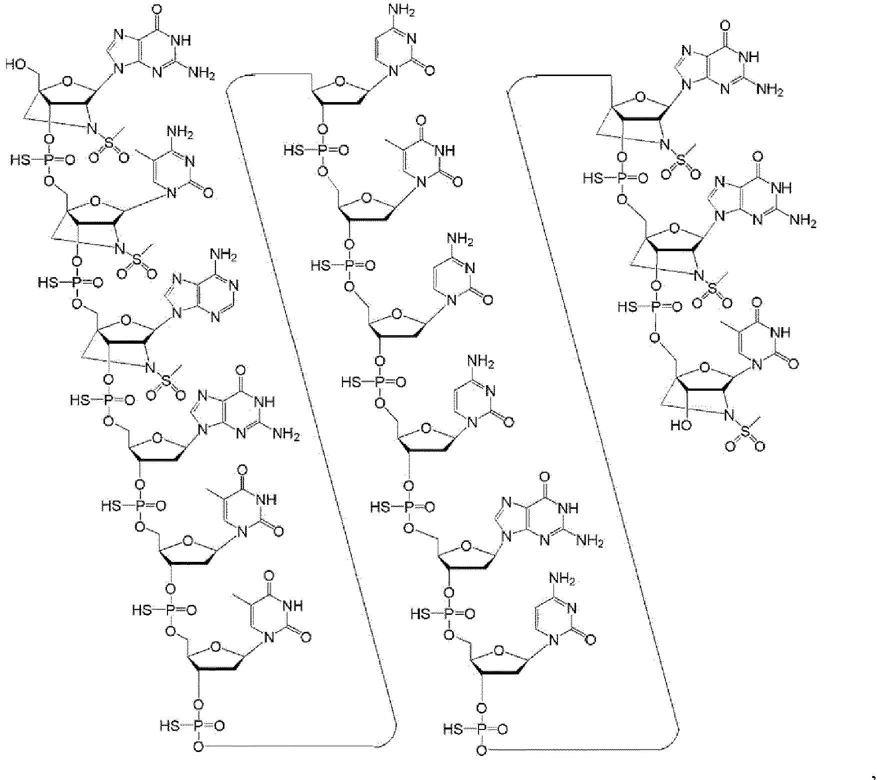 from a compound represented by Formula (XVII"): 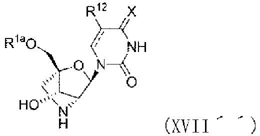 [wherein
R^{1a} represents a hydroxyl protecting group;
R¹² represents a hydrogen atom, an alkyl group, an alkoxy group, or an alkoxyalkyl group;
X represents an oxygen atom or NR¹³ (wherein R¹³ represents a hydrogen atom or an amino protecting group); and
a double line consisting of a solid line and a dotted line represents a single bond or a double bond],
the production method comprising the following steps:
a step of producing a compound represented by Formula (XVII") using the production method according to claim 6;
a step of producing an ALNA[Ms] amidite from the compound represented by Formula (XVII");
a step of subjecting the ALNA[Ms] amidite and an amidite for DNA synthesis to a phosphoramidite method; and,
as necessary, a step of subjecting a phosphodiester bond to a thiation reaction.

30. A modified oligonucleotide or a salt thereof represented by the following formula: 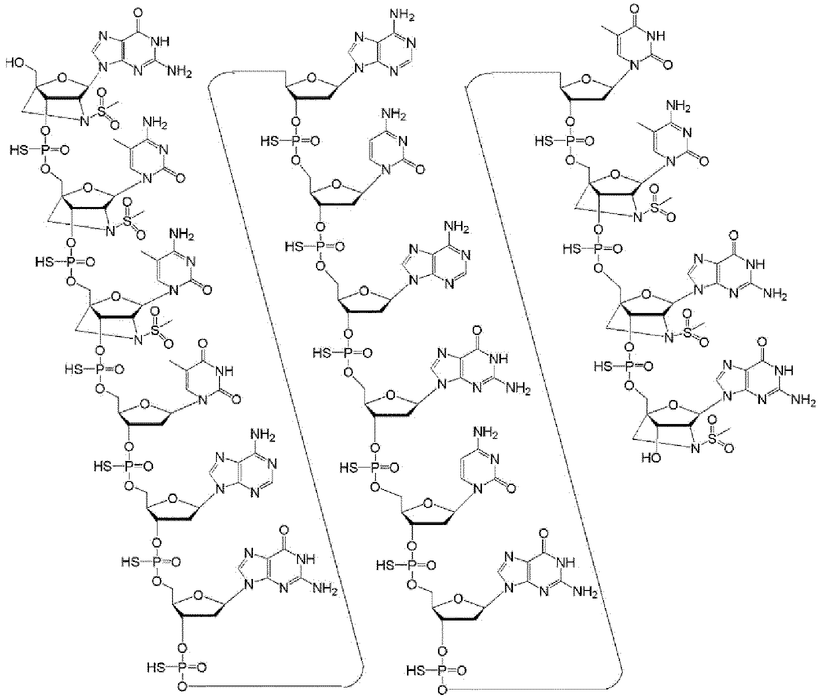 produced using the production method according to claim 28.

31. A modified oligonucleotide or a salt thereof represented by the following formula: 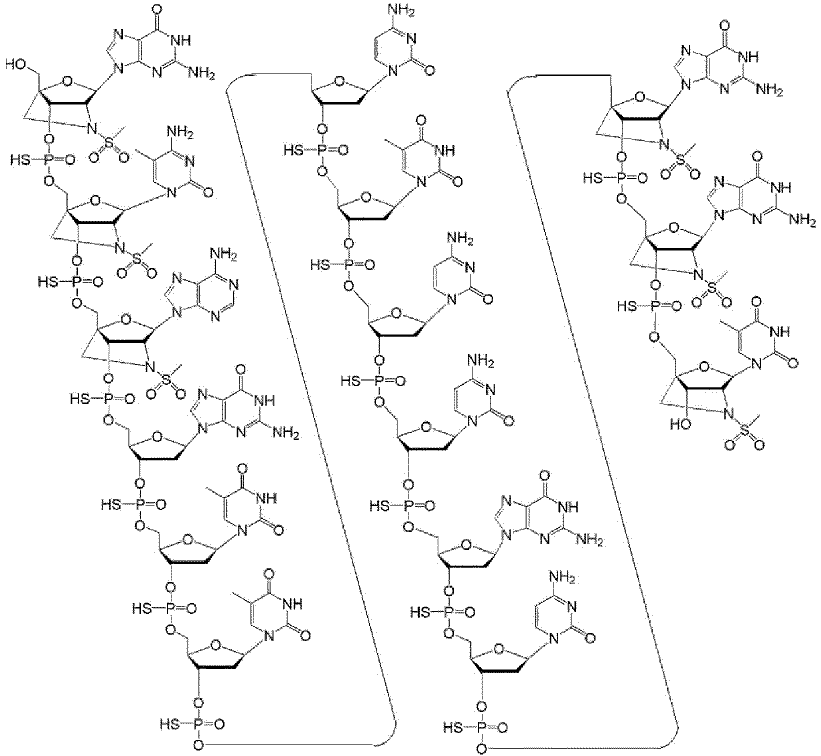 produced using the production method according to claim 29.
